# EUROPÄISCHE PATENTSCHRIFT

(11) **EP 1 594 853 B1**
(45) Veröffentlichungstag und Bekanntmachung des Hinweises auf die Patenterteilung: **09.07.2008**
(21) Anmeldenummer: 04701930.2
(22) Anmeldetag: 14.01.2004
(51) Int. Cl.: C07D 265/28

(54) **VERFAHREN ZUR HERSTELLUNG BICYCLISCHER AROMATISCHER AMINOSÄUREN SOWIE DEREN ZWISCHENPRODUKTE**
METHOD FOR THE PRODUCTION OF BICYCLIC AROMATIC AMINO ACIDS AND INTERMEDIATE PRODUCTS THEREOF
PROCEDE DE PRODUCTION D'ACIDES AMINES AROMATIQUES BICYCLIQUES ET PRODUITS INTERMEDIAIRES DE CES COMPOSES

(30) Priorität: 12.02.2003 DE 10305784
(43) Veröffentlichungstag der Anmeldung: 16.11.2005
(73) Patentinhaber: Merck Patent GmbH, 64293 Darmstadt (DE)
(72) Erfinder: WIESNER, Matthias, 64342 Seeheim-Jugenheim (DE); NEFF, Bernd, 64589 Stockstadt (DE)
(86) Internationale Anmeldenummer: PCT/EP2004/000210
(87) Internationale Veröffentlichungsnummer: WO 2004/072054

(56) Entgegenhaltungen:
- WO-A-94/29273
- WO-A-96/18602
- WO-A-98/35949

## Beschreibung

Bicyclische aromatische Aminosäuren haben sich als geeignete Templates für die Herstellung von Integrin-Antagonisten erwiesen.

Verbindungen dieser Substanzklasse sind z. B. aus WO 94/29273, WO 96/00730 und WO 96/18602 bekannt oder aus WO 98/35949 bekannt-

Typische Vertreter dieser Substanzen enthalten zwei Stereozentren und somit in vier diastereomeren Formen auftreten. Für einen geeignten Herstellungsprozess derartiger Verbindungen mit optimierter Gesamtausbeute ist eine stereoselektive Reaktionsführung von besonderer Bedeutung, da dann eine aufwändige chirale Reinigung vermieden werden kann.

Der Erfindung lag die Aufgabe zugrunde, ein neues Verfahren zur Herstellung von Verbindungen aufzufinden, insbesondere solcher Substanzen, die in der WO 98/35949 beschrieben sind.

Es wurde gefunden, daß aus enantiomerenreiner Glutaminsäure reaktive Derivate der Hydroxyglutarsäure hergestellt werden können, die einen stereoselektiven Aufbau des Benzoxazinon-Gerüsts durch Umsetzung mit geeigneten Tyrosin-Abkömmlingen ermöglichen und zu diastereomerenreinen Produkten führen. Im Unterschied zu dem in WO 98/35949 beschriebenen Syntheseweg verläuft das erfindungsgemäße Verfahren nahezu racemisierungsfrei und eine Trennung der Diastereomeren durch Chromatographie oder Kristallisation kann vermieden werden.

Gegenstand der Erfindung ist ein Verfahren zur Herstellung von Verbindungen der Formel I 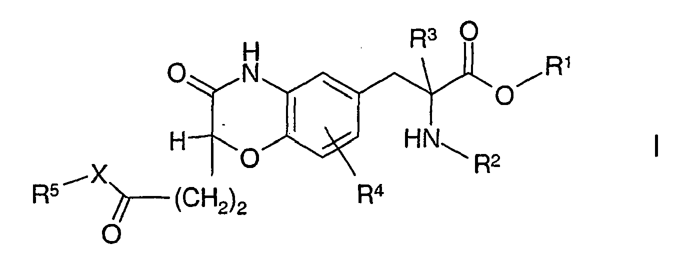 worin
- R¹: H, Alkyl mit 1, 2, 3, 4, 5 oder 6 C-Atomen oder Benzyl,
- R²: R¹⁰, CO-R¹⁰, COOR⁶, COOR¹⁰, CONHR¹⁰, SO₂R⁶ oder SO₂R¹⁰,
- R³: H oder Alkyl mit 1-6 C-Atomen,
- R⁴: H, Hal, OA, NHR¹⁰, N(R¹⁰)₂, -NH-Acyl, -O-Acyl, CN, NO₂, OR¹⁰, SR¹⁰, R² oder CONHR¹⁰,
- R⁵: NH₂, H₂N-C(=NH) oder H₂N-(C=NH)-NH, wobei die primären Aminogruppen auch mit konventionellen Aminoschutzgruppen versehen sein können, oder ein-, zwei- oder dreifach durch R¹⁰, CO-R¹⁰, COOR¹⁰ oder SO₂R¹⁰ substituiert sein können, oder R⁶,
- X: fehlt oder NH,
- R⁶: einen ein- oder zweikernigen gesättigten, ungesättigten oder aromatischen Heterocyclus mit 1 bis 4 N-, O- und / oder S-Atomen, der unsubstituiert oder ein-, zwei- oder dreifach durch Hal, A, -CO-A, OA, CN, COOH, COOA, CONHA, NO₂, =NH oder =O substituiert sein kann,
- R⁹: H, Hal, OA, NHA, NAA', NHAcyl, OAcyl, CN, NO₂, SA, SOA, SO₂A, SO₂Ph oder SO₃H,
- R¹⁰: H, A, Ar oder Aralkylen mit 7-14 C-Atomen,
- A, A': jeweils unabhängig voneinander H oder unsubstituiertes oder ein-, zwei- oder dreifach durch R⁹ substituiertes Alkyl oder Cycloalkyl mit 1-15 C-Atomen und worin eine, zwei- oder drei Methylengruppen durch N, O und/oder S ersetzt sein können,
- Ar: unsubstituiertes oder ein-, zwei- oder dreifach durch A und/oder R⁹ substituiertes ein- oder zweikerniges aromatisches Ringsystem mit 0, 1, 2, 3 oder 4 N-, O- und/oder S-Atomen,
- Ph: unsubstituiertes oder ein-, zwei- oder dreifach durch A substituiertes Phenyl,
- Hal: F, Cl, Br oder I und
bedeuten,
sowie deren Salze, Solvate und Stereoisomere,
wobei
a) 5-Oxo-tetrahydrofuran-2-carbonsäure mit einer Verbindung der Formel II

   R⁷-L II

   worin
   - R⁷: Benzyl oder Alkyl mit 1, 2, 3, 4, 5 oder 6 C-Atomen und
   - L: Cl, Br, I oder eine freie oder reaktionsfähig funktionell abgewandelte OH-Gruppe
   bedeuten,
   zu einer Verbindung der Formel III 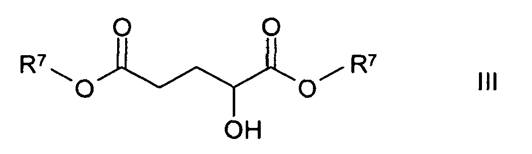 worin
   - R⁷: Benzyl oder Alkyl mit 1, 2, 3, 4, 5 oder 6 C-Atomen bedeutet,
   umgesetzt wird,
b) dann eine Verbindung der Formel III mit einer Verbindung der Formel IV

   R⁸-L IV

   worin
   - R⁸: Alkylsulfonyl mit 1-6 C-Atomen oder Arylsulfonyl mit 6-10 C-Atomen und
   - L: Cl, Br, I oder eine freie oder reaktionsfähig funktionell abgewandelte OH-Gruppe
   bedeuten,
   zu einer Verbindung der Formel V 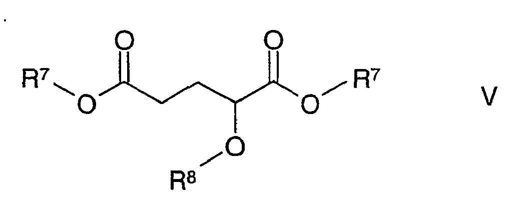 worin
   - R⁷: Benzyl oder Alkyl mit 1, 2, 3, 4, 5 oder 6 C-Atomen und
   - R⁸: Alkylsulfonyl mit 1-6 C-Atomen oder Arylsulfonyl mit 6-10 C-Atomen
   bedeuten,
   umgesetzt wird,
c) dann eine Verbindung der Formel III oder V mit einer Verbindung der Formel VI 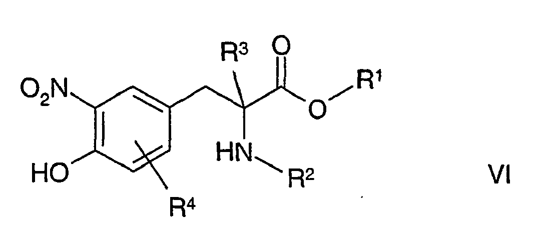 worin
   - R¹: H, Alkyl mit 1, 2, 3, 4, 5 oder 6 C-Atomen oder Benzyl,
   - R²: R¹⁰, CO-R¹⁰, COOR⁶, COOR¹⁰, CONHR¹⁰, SO₂R⁶ oder SO₂R¹⁰,
   - R³: H oder Alkyl mit 1-6 C-Atomen,
   - R⁴: H, Hal, OA, NHR¹⁰, N(R¹⁰)₂, -NH-Acyl, -O-Acyl, CN, NO₂, OR¹⁰, SR¹⁰, R² oder CONHR¹⁰,
   - R⁶: einen ein- oder zweikernigen gesättigten, ungesättigten oder aromatischen Heterocyclus mit 1 bis 4 N-, O- und / oder S-Atomen, der unsubstituiert oder ein-, zwei- oder dreifach durch Hal, A, -CO-A, OA, CN, COOH, COOA, CONHA, NO₂, =NH oder =O substituiert sein kann,
   - R⁹: H, Hal, OA, NHA, NAA', NHAcyl, OAcyl, CN, NO₂, SA, SOA, SO₂A, SO₂Ph oder SO₃H,
   - R¹⁰: H, A, Ar oder Aralkylen mit 7-14 C-Atomen,
   - A, A': jeweils unabhängig voneinander H oder unsubstituiertes oder ein-, zwei- oder dreifach durch R⁹ substituiertes Alkyl oder Cycloalkyl mit 1-15 C-Atomen und worin eine, zwei- oder drei Methylengruppen durch N, O und/oder S ersetzt sein können,
   - Ar: unsubstituiertes oder ein-, zwei- oder dreifach durch A und/oder R⁹ substituiertes ein- oder zweikerniges aromatisches Ringsystem mit 0, 1, 2, 3 oder 4 N-, O- und/oder S-Atomen,
   - Ph: unsubstituiertes oder ein-, zwei- oder dreifach durch A substituiertes Phenyl,
   - Hal: F, Cl, Br oder I
   bedeuten,
   zu einer Verbindung der Formel VII 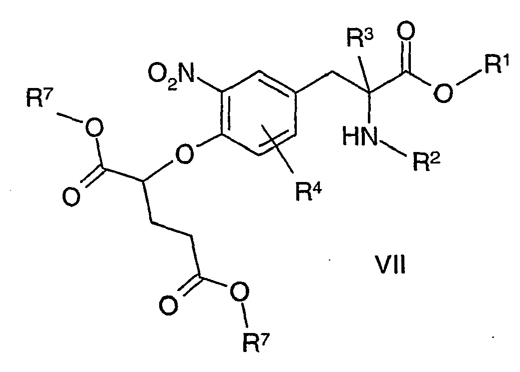 worin
   - R¹: H, Alkyl mit 1, 2, 3, 4, 5 oder 6 C-Atomen oder Benzyl,
   - R²: R¹⁰, CO-R¹⁰; COOR⁶, COOR¹⁰, CONHR¹⁰, SO₂R⁶ oder SO₂R¹⁰,
   - R³: H oder Alkyl mit 1-6 C-Atomen,
   - R⁴: H, Hal, OA, NHR¹⁰, N(R¹⁰)₂, -NH-Acyl, -O-Acyl, CN, NO₂, OR¹⁰, SR¹⁰, R² oder CONHR¹⁰,
   - R⁶: einen ein- oder zweikernigen gesättigten, ungesättigten oder aromatischen Heterocyclus mit 1 bis 4 N-, O- und / oder S-Atomen, der unsubstituiert oder ein-, zwei- oder dreifach durch Hal, A, -CO-A, OA, CN, COOH, COOA, CONHA, NO₂, =NH oder =O substituiert sein kann,
   - R⁷: Benzyl oder Alkyl mit 1, 2, 3, 4, 5 oder 6 C-Atomen,
   - R⁹: H, Hal, OA, NHA, NAA', NHAcyl, OAcyl, CN, NO₂, SA, SOA, SO₂A, SO₂Ph oder SO₃H,
   - R¹⁰: H, A, Ar oder Aralkylen mit 7-14 C-Atomen,
   - A, A': jeweils unabhängig voneinander H oder unsubstituiertes oder ein-, zwei- oder dreifach durch R⁹ substituiertes Alkyl oder Cycloalkyl mit 1-15 C-Atomen und worin eine, zwei- oder drei Methylengruppen durch N, O und/oder S ersetzt sein können,
   - Ar: unsubstituiertes oder ein-, zwei- oder dreifach durch A und/oder R⁹ substituiertes ein- oder zweikerniges aromatisches Ringsystem mit 0, 1, 2, 3 oder 4 N-, O- und/oder S-Atomen,
   - Ph: unsubstituiertes oder ein-, zwei- oder dreifach durch A substituiertes Phenyl,
   - Hal: F, Cl, Br oder I
   bedeuten,
   umgesetzt wird,
d) dann eine Verbindung der Formel VII zu einer Verbindung der Formel VIII 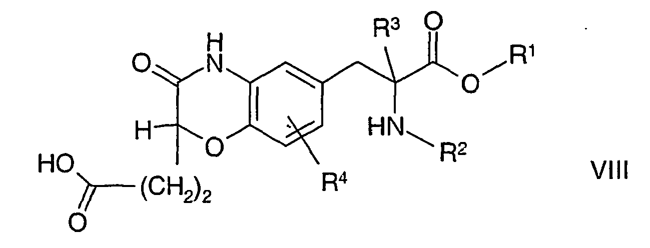 worin
   - R¹: H, Alkyl mit 1, 2, 3, 4, 5 oder 6 C-Atomen oder Benzyl,
   - R²: R¹⁰, CO-R¹⁰, COOR , COOR¹⁰, CONHR¹⁰, SO₂R⁶ oder SO₂R¹⁰ ,
   - R³: H oder Alkyl mit 1-6 C-Atomen,
   - R⁴: H, Hal, OA, NHR¹⁰, N(R¹⁰)₂, -NH-Acyl, -O-Acyl, CN, NO₂, OR¹⁰, SR¹⁰, R² oder CONHR¹⁰,
   - R⁶: einen ein- oder zweikernigen gesättigten, ungesättigten oder aromatischen Heterocyclus mit 1 bis 4 N-, O- und / oder S-Atomen, der unsubstituiert oder ein-, zwei- oder dreifach durch Hal, A, -CO-A, OA, CN, COOH, COOA, CONHA, NO₂, =NH oder =O substituiert sein kann,
   - R⁹: H, Hal, OA, NHA, NAA', NHAcyl, OAcyl, CN, NO₂, SA, SOA, SO₂A, SO₂Ph oder SO₃H,
   - R¹⁰: H, A, Ar oder Aralkylen mit 7-14 C-Atomen,
   - A, A': jeweils unabhängig voneinander H oder unsubstituiertes oder ein-, zwei- oder dreifach durch R⁹ substituiertes Alkyl oder Cycloalkyl mit 1-15 C-Atomen und worin eine, zwei- oder drei Methylengruppen durch N, O und/oder S ersetzt sein können,
   - Ar: unsubstituiertes oder ein-, zwei- oder dreifach durch A und/oder R⁹ substituiertes ein- oder zweikerniges aromatisches Ringsystem mit 0,1 , 2, 3 oder 4 N-, O- und/oder S-Atomen,
   - Ph: unsubstituiertes oder ein-, zwei- oder dreifach durch A substituiertes Phenyl,
   - Hal: F, Cl, Br oder I bedeuten,
   umgesetzt wird,
e) anschließend gegebenenfalls eine Verbindung der Formel VIII in eine Verbindung der Formel VIIIa 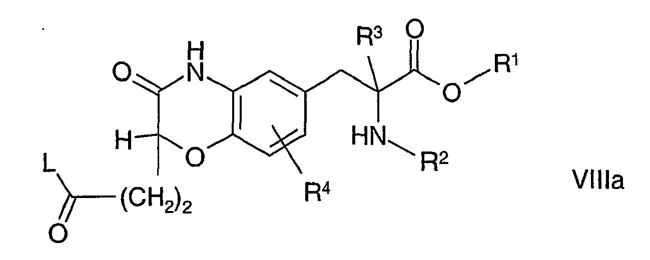 worin
   - R¹, R², R³ und R⁴: die unter d) angegebenen Bedeutungen haben und
   - L: Cl, Br, I oder eine reaktionsfähig funktionell abgewandelte OH-Gruppe bedeutet,
   umwandelt,
e) dann eine Verbindung der Formel VIII oder eine Verbindung der Formel VIIIa
   e) i) mit einer Verbindung der Formel IX

   R⁵-H IX

   worin
   - R⁵: NH₂, H₂N-C(=NH) oder H₂N-(C=NH)-NH, wobei die primären Aminogruppen auch mit konventionellen Aminoschutzgruppen versehen sein können, oder ein-, zwei- oder dreifach durch R¹⁰, CO-R¹⁰, COOR¹⁰ oder SO₂R¹⁰ substituiert sein können, worin R¹⁰ die bei Formel I angegebenen Bedeutungen hat,
   bedeutet,
   oder
e) ii) mit einer Verbindung der Formel X

   R⁶-NH₂ X

   worin
   - R⁶: einen ein- oder zweikernigen gesättigten, ungesättigten oder aromatischen Heterocyclus mit 1 bis 4 N-, O- und / oder S-Atomen, der unsubstituiert oder ein-, zwei- oder dreifach durch Hal, A, -CO-A, OA, CN, COOH, COOA, CONHA, NO₂, =NH oder =O substituiert sein kann,
   bedeutet,
   zu einer Verbindung der Formel umsetzt,
   gegebenenfalls anschließend einen Rest R² in einen anderen Rest R² umwandelt,
   indem man eine Verbindung der Formel I, worin R² H bedeutet mit einer Verbindung ausgewählt aus der Gruppe

L-R¹⁰, L-CO-R¹⁰, L-COOR⁶, L-COOR¹⁰, R¹⁰-N=C=O, R⁶-SO₂L, R¹⁰-SO₂L,
worin
- R¹⁰: A, Ar oder Aralkylen mit 7-14 C-Atomen,
- A und Ar: die angegebenen Bedeutungen haben und
- L Cl, Br, I: oder eine freie oder reaktionsfähig funktionell abgewandelte OH-Gruppe bedeutet,
umsetzt,
und/ oder eine Base oder Säure der Formel I in eines ihrer Salze umwandelt.

Die WO 98/35949 ist als nächstliegender Stand der Technik anzusehen. Analog nachstehendem Schema erfolgt dort die Umsetzung von einer Dibrom-Verbindung ausgehend (Beispiel 8 in WO 98/35949):

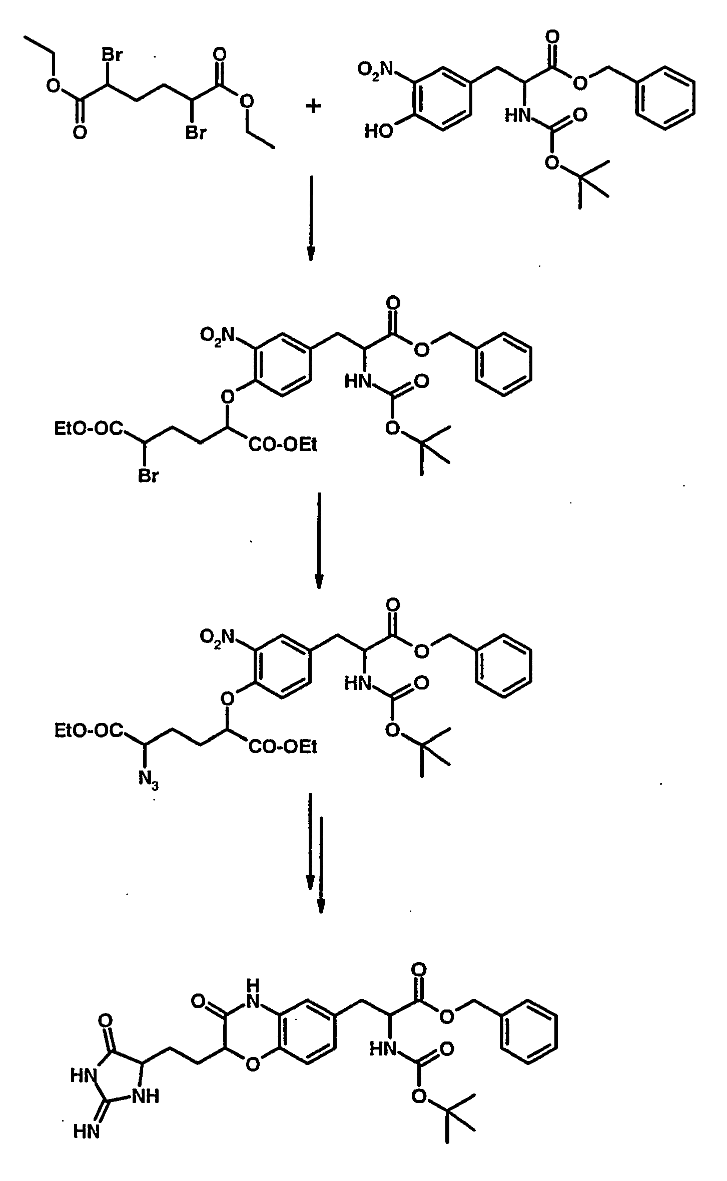

In Beispiel 11 der WO 98/35949 erfolgt die Umsetzung analog nachstehendem Schema:

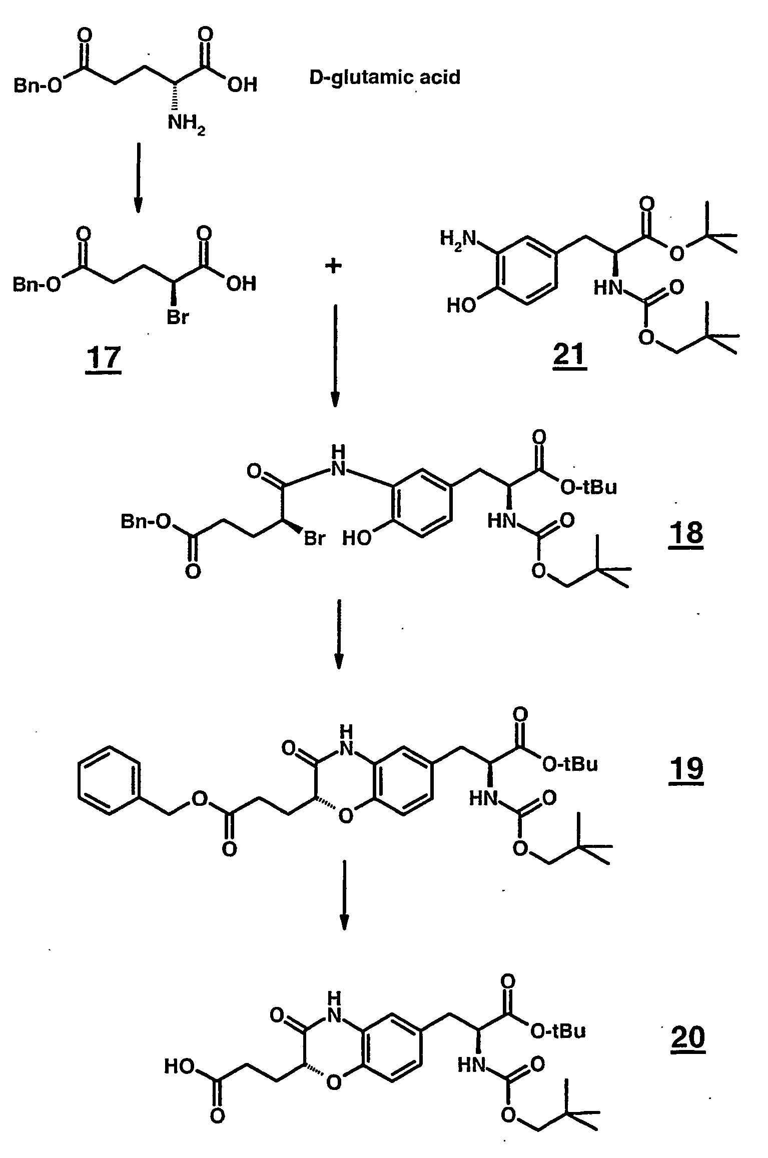

Im Gegensatz zu den Verfahren, die in WO 98/35949 beschrieben sind, verlaufen die erfindungsgemäßen Verfahren überraschenderweise hoch stereospezifisch, d.h. es ist keine Racemisierung nachweisbar oder die Selektivität liegt bei > 96%.

Die Verbindungen der Formel I und ihre Salze besitzen bei guter Verträglichkeit sehr wertvolle pharmakologische Eigenschaften. Vor allem wirken sie als Integrin-Inhibitoren, wobei sie insbesondere die Wechselwirkungen der αᵥ-Integrin-Rezeptoren mit Liganden hemmen. Besondere Wirksamkeit zeigen die Verbindungen im Fall der Integrine αᵥβ₃ und αᵥβ₅. Ganz besonders wirksam sind die Verbindungen als Adhäsionsrezeptor-Antagonisten für den Vitronectin-Rezeptor αᵥβ₃ . Diese Wirkung kann z.B. nach der Methode nachgewiesen werden, die von J.W. Smith et al. in J. Biol. Chem. 265, 11008-11013 und 12267-12271 (1990) beschrieben wird.
B. Felding-Habermann und D.A. Cheresh beschreiben in Curr. Opin. Cell. Biol. 5, 864 (1993) die Bedeutungen der Integrine als Adhäsionsrezeptoren für die unterschiedlichsten Phänomene und Krankheitsbilder, speziell in Bezug auf den Vitronectinrezeptor αᵥβ₃.

Die Abhängigkeit der Entstehung von Angiogenese von der Wechselwirkung zwischen vaskulären Integrinen und extrazellulären Matrixproteinen ist von P.C. Brooks, R.A. Clark und D.A. Cheresh in Science 264, 569-71 (1994) beschrieben.

Die Möglichkeit der Inhibierung dieser Wechselwirkung und damit zum Einleiten von Apoptose (programmierter Zelltod) angiogener vaskulärer Zellen durch ein cyclisches Peptid ist von P.C. Brooks, A.M. Montgomery, M. Rosenfeld, R.A. Reisfeld, T.-Hu, G. Klier und D.A. Cheresh in Cell 79, 1157-64 (1994) beschrieben.

Der experimentelle Nachweis, daß auch die erfindungsgemäßen Verbindungen die Anheftung von lebenden Zellen auf den entsprechenden Matrixproteinen verhindern und dementsprechend auch die Anheftung von Tumorzellen an Matrixproteine verhindern, kann in einem Zelladhäsionstest erbracht werden, der analog der Methode von F. Mitjans et al., J. Cell Science 108, 2825-2838 (1995) durchgeführt wird.

P.C. Brooks et al. beschreiben in J. Clin. Invest. 96, 1815-1822 (1995) αᵥβ₃-Antagonisten zur Krebsbekämpfung und zur Behandlung tumorinduzierter angiogener Krankheiten.
Die erfindungsgemäßen Verbindungen der Formel I können daher als Arzneimittelwirkstoffe insbesondere zur Behandlung von Tumorerkrankungen, Osteoporosen, osteolytischen Erkrankungen sowie zur Unterdrückung der Angiogenese eingesetzt werden.

Verbindungen der Formel I, die die Wechselwirkung von Integrinrezeptoren und Liganden blockieren, verhindern die Ausbreitung von Tumorzellen durch Metastase. Dies wird durch folgende Beobachtungen belegt:
Die Verbreitung von Tumorzellen von einem lokalen Tumor in das vaskuläre System erfolgt durch die Bildung von Mikroaggregaten (Mikrothromben) durch Wechselwirkung der Tumorzellen mit Blutplättchen. Die Tumorzellen sind durch den Schutz im Mikroaggregat abgeschirmt und werden von den Zellen des Immunsystems nicht erkannt.
Die Mikroaggregate können sich an Gefäßwandungen festsetzen, wodurch ein weiteres Eindringen von Tumorzellen in das Gewebe erleichtert wird. Da die Bildung der Mikrothromben durch Fibrinogenbindung an die Fibrinogenrezeptoren auf aktivierten Blutplättchen vermittelt wird, können die GPIIa/IIIb-Antagonisten als wirksame Metastase-Hemmer angesehen werden.

Verbindungen der Formel I hemmen neben der Bindung von Fibrinogen, Fibronectin und des Willebrand-Faktors an den Fibrinogenrezeptor der Blutplättchen auch die Bindung weiterer adhäsiver Proteine, wie Vitronectin, Kollagen und Laminin, an die entsprechenden Rezeptoren auf der Oberflache verschiedener Zelltypen. Sie verhindern insbesondere die Entstehung von Blutplättchenthromben und können daher zur Behandlung von Thrombosen, Apoplexie, Herzinfarkt, Entzündungen und Arteriosklerose eingesetzt werden.

Insbesondere können die Verbindungen der Formel I auch in der Ophthalmologie verwendet werden.

Die Eigenschaften der Verbindungen können auch nach Methoden nachgewiesen werden, die in der EP-A1-0 462 960 beschrieben sind. Die Hemmung der Fibrinogenbindung an den Fibrinogenrezeptor kann nach der Methode nachgewiesen werden, die in der EP-A1-0 381 033 angegeben ist.

Die thrombozytenaggregationshemmende Wirkung läßt sich in vitro nach der Methode von Born (Nature 4832, 927-929, 1962) nachweisen.

Die Verbindungen der Formel I können als Arzneimittelwirkstoffe in der Human- und Veterinärmedizin eingesetzt werden, zur Prophylaxe und/oder Therapie von Thrombose, myocardialem Infarkt, Arteriosklerose, Entzündungen, Apoplexie, Angina pectoris, Tumorerkrankungen, osteolytischen Krankheiten wie Osteoporose, pathologisch angiogenen Krankheiten wie z. B. Entzündungen, ophthalmologischen Krankheiten, diabetischer Retinopathie, makularer Degeneration, Myopia, okularer Histoplasmose, rheumatischer Arthritis, Osteoarthritis, rubeotischem Glaukom, ulcerativer Colitis, Morbus Crohn, Atherosklerose, Psoriasis, Restenose nach Angioplastie, viraler Infektion, bakterieller Infektion, Pilzinfektion, bei akutem Nierenversagen und bei der Wundheilung zur Unterstützung der Heilungsprozesse.

Die Verbindungen der Formel I können als antimikrobiell wirkende Substanzen bei Operationen eingesetzt werden, wo Biomaterialien, Implantate, Katheter oder Herzschrittmacher verwendet werden. Dabei wirken sie antiseptisch. Die Wirksamkeit der antimikrobiellen Aktivität kann durch das von P.Valentin-Weigund et al., in Infection and Immunity, 2851-2855 (1988) beschriebene Verfahren nachgewiesen werden.

Die vor- und nachstehend aufgeführten Abkürzungen stehen für:
- Ac: Acetyl
- Bn: Benzyl
- BOC: tert.-Butoxycarbonyl
- CBZ oder Z: Benzyloxycarbonyl
- DBU: 1,8-Diazabicyclo[5.4.0]undec-7-en
- DCCI: Dicyclohexylcarbodiimid
- DMF: Dimethylformamid
- DOPA: (3,4-Dihydroxyphenyl)-alanin
- DPFN: 3,5-Dimethylpyrazol-1-formamidinium-nitrat
- EDCI: N-Ethyl-N,N'-(dimethylaminopropyl)-carbodiimid
- Et: Ethyl
- Fmoc: 9-Fluorenylmethoxycarbonyl
- HOBt: 1-Hydroxybenzotriazol
- Me: Methyl
- Mtr: 4-Methoxy-2,3,6-trimethylphenyl-sulfonyl
- HONSu: N-Hydroxysuccinimid
- POA: Phenoxyacetyl
- TBTU: O-(Benzotriazol-1-yl)-N,N,N',N'-tetramethyluroniumtetrafluoroborat
- TFA: Trifluoressigsäure
- Trt: Trityl (Triphenylmethyl)
- Z oder CBZ: Benzyloxycarbonyl.

Unter den Verbindungen der Formel I versteht man auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B.
Mono- oder Dihydrate oder Alkoholate.

Unter pharmazeutisch verwendbaren Derivaten versteht man z.B. die Salze der erfindungsgemäßen Verbindungen als auch sogenannte Prodrug-Verbindungen.
Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.
Hierzu gehören auch bioabbaubare Polymerderivate der erfindungsgemäßen Verbindungen, wie dies z. B. in Int. J. Pharm. 115, 61-67 (1995) beschrieben ist.

Die Verbindungen der Formel I umfassen auch Mischungen der Verbindungen der Formel I, z.B. Gemische zweier Diastereomerer z.B. im Verhältnis 1:1, 1:2, 1:3, 1:4,1:5, 1:10,1:100 oder 1:1000.
Besonders bevorzugt handelt es sich dabei um Mischungen stereoisomerer Verbindungen.

Für die gesamte Erfindung gilt, daß sämtliche Reste, die mehrfach auftreten, wie z.B. A und A', gleich oder verschieden sein können, d.h. unabhängig voneinander sind.

In den vorstehenden Formeln hat Alkyl 1, 2, 3, 4, 5, 6, 7, 8, 9, 10, 11, 12, 13, 14 oder 15 C-Atome und steht vorzugsweise für Methyl, Ethyl, Propyl, Isopropyl, Butyl, Isobutyl, sek.-Butyl oder tert.-Butyl, ferner auch für Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2-, 1,2,2-Trimethylpropyl, Heptyl, Octyl, Nonyl oder Decyl.

In einer bevorzugten Ausführungsform bedeutet A unsubstituiertes Alkyl oder Cycloalkyl mit 1, 2, 3, 4, 5, 6, 7, 8, 9, 10, 11, 12, 13, 14 oder 15 C-Atomen.
Cycloalkyl bedeutet vorzugsweise Cyclopropyl, Cyclobutyl, Cylopentyl, Cyclohexyl, Cycloheptyl oder 3-Menthyl. Cycloalkyl bedeutet insbesondere den Rest eines bicyclischen Terpens, ganz besonders bevorzugt ist der Campher-10-yl-Rest.

Alkylen bedeutet bevorzugt Methylen, Ethylen, Propylen, Butylen, Pentylen, ferner auch Hexylen, Heptylen, Ocytylen, Nonylen oder Decylen. Aralkylen bedeutet vorzugsweise Alkylenphenyl und ist z.B. vorzugsweise Benzyl oder Phenethyl.

Cycloalkylen bedeutet bevorzugt Cyclopropylen, 1,2- oder 1,3-Cyclobutylen, 1,2- oder 1,3-Cyclopentylen, 1,2- , 1,3- oder 1,4-Cyclohexylen, ferner 1,2- , 1,3- oder 1,4-Cycloheptylen.

CO-A ist Alkanoyl oder Cycloalkanoyl und bedeutet vorzugsweise Formyl, Acetyl, Propionyl, Butyryl, Pentanoyl, Hexanoyl, Heptanoyl, Octanoyl, Nonanoyl, Decanoyl, Undecanoyl, Dodecanoyl, Tridecanoyl, Tetradecanoyl, Pentadecanoyl, Hexadecanoyl, Heptadecanoyl oder Octadecanoyl.

Acyl ist C₁-C₇-Acyl und hat 1, 2, 3, 4, 5, 6 oder 7 C-Atome und bedeutet bevorzugt z.B. Formyl, Acetyl, Propionyl, Butyryl, Trifluoracetyl oder Benzoyl.

Bevorzugte Substituenten für Alkyl, Alkylen, Cycloalkyl, Cycloalkylen, Alkanoyl und Cycloalkanoyl sind z.B. Hal, OA, NHA, NAA', CN, NO₂, SA, SOA, SO₂A, SO₂Ar und/oder SO₃H, insbesondere z.B. F, Cl, Hydroxy, Methoxy, Ethoxy, Amino, Dimethylamino, Methylthio, Methylsulfinyl, Methylsulfonyl oder Phenylsulfonyl.

Bevorzugte Substituenten für Ar und Arylen sind z.B. A und/oder Hal, OA, NHA, NAA', CN, NO₂, SA, SOA, SO₂A, SO₂Ar und/oder SO₃H, insbesondere z.B. F, Cl, Hydroxy, Methoxy, Ethoxy, Amino, Dimethylamino, Methylthio, Methylsulfinyl, Methylsulfonyl oder Phenylsulfonyl.

In den Resten Alkyl, Alkylen, Cycloalkyl, Cycloalkylen, Alkanoyl und Cycloalkanoyl können jeweils eine, zwei- oder drei Methylengruppen durch N, O und/oder S ersetzt sein.

Ar-CO ist Aroyl und bedeutet vorzugsweise Benzoyl oder Naphthoyl.

Ar ist unsubstituiertes, vorzugsweise - wie angegeben - monosubstituiertes Phenyl, im einzelnen bevorzugt Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Cyanphenyl, o-, m- oder p-Methoxyphenyl, o-, m- oder p-Ethoxyphenyl, o-, m- oder p-Fluorphenyl, o-, m- oder p-Bromphenyl, o-, m- oder p- Chlorphenyl, o-, m- oder p-Methylthiophenyl, o-, m- oder p-Methylsulfinylphenyl, o-, m- oder p-Methylsulfonylphenyl, o-, m- oder p-Aminophenyl, o-, m- oder p-Methylaminophenyl, o-, m- oder p-Dimethylaminophenyl, o-, m- oder p-Nitrophenyl,
weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2-Chlor-3-methyl-, 2-Chlor-4-methyl-, 2-Chlor-5-methyl-, 2-Chlor-6-methyl-, 2-Methyl-3-chlor-, 2-Methyl-4-chlor-, 2-Methyl-5-chlor-, 2-Methyl-6-chlor-, 3-Chlor-4-methyl-, 3-Chlor-5-methyl- oder 3-Methyl-4-chlorphenyl, 2-Brom-3-methyl-, 2-Brom-4-methyl-, 2-Brom-5-methyl-, 2-Brom-6-methyl-, 2-Methyl-3-brom-, 2-Methyl-4-brom-, 2-Methyl-5-brom-, 2-Methyl-6-brom-, 3-Brom-4-methyl-, 3-Brom-5-methyl- oder 3-Methyl-4-bromphenyl, 2,4- oder 2,5-Dinitrophenyl, 2,5- oder 3,4-Dimethoxyphenyl, 2,3,4-, 2,3,5-, 2,3,6-, 2,4,6- oder 3,4,5-Trichlorphenyl, 2,4,6-Tritert.-Butylphenyl, 2,5-Dimethylphenyl, p-lodphenyl, 4-Fluor-3-chlorphenyl, 4-Fluor-3,5-dimethylphenyl, 2-Fluor-4-bromphenyl, 2,5-Difluor-4-bromphenyl, 2,4-Dichlor-5-methylphenyl, 3-Brom-6-methoxyphenyl, 3-Chlor-6-methoxyphenyl, 2-Methoxy-5-methylphenyl, 2,4,6-Triisopropylphenyl, Naphthyl, 1,3-Benzodioxol-5-yl, 1,4-Benzodioxan-6-yl, Benzothiadiazol-5-yl oder Benzoxadiazol-5-yl.

Weiter bedeutet Ar vorzugsweise 2- oder 3-Furyl, 2- oder 3-Thienyl, 1-, 2-oder 3-Pyrrolyl, 1-, 2, 4- oder 5-Imidazolyl, 1-, 3-, 4- oder 5-Pyrazolyl, 2-, 4-oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1-oder 5-Tetrazolyl, 1,2,3-Oxadiazol-4- oder -5-yl, 1,2,4-Oxadiazol-3- oder - 5-yl, 1,3,4-l'.hiadiazol-2- oder -5-yl, 1,2,4-Thiadiazol-3- oder -5-yl, 1,2,3-Thiadiazol-4- oder -5-yl, 2-, 3-,4-, 5- oder 6-2H-Thiopyranyl, 2-, 3- oder 4-4-H-Thiopyranyl, 3- oder 4-Pyridazinyl, Pyrazinyl, 2-, 3-, 4-, 5- 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 4- oder 5-Benzimidazolyl, 1-, 3-, 4-, 5-, 6- oder 7-Benzopyrazolyl, 2-,4-,5-,6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7-oder 8-Cinnolinyl, 2-, 4-, 5-, 6-, 7- oder 8-Chinazolinyl.

Arylen hat die gleichen Bedeutungen wie für Ar angegeben, mit der Maßgabe, daß eine weitere Bindung vom aromatischen System zum nächsten Bindungsnachbar geknüpft ist.

Heterocycloalkylen bedeutet vorzugsweise 1,2-, 2,3- oder 1,3-Pyrrolidinyl, 1,2-, 2,4-, 4,5- oder 1,5-Imidazolidinyl, 1,2-, 2,3-, oder 1,3-Pyrazolidinyl, 2,3-, 3,4-, 4,5- oder 2,5-Oxazolidinyl, 1,2-, 2,3-, 3,4- oder 1,4- Isoxazolidinyl, 2,3-, 3,4-, 4,5- oder 2,5-Thiazolidinyl, 2,3-, 3,4-, 4,5- oder 2,5-Isothiazolidinyl, 1,2-, 2,3-, 3,4- oder 1,4-Piperidinyl, 1,4- oder 1,2-Piperazinyl, weiterhin bevorzugt 1,2,3-Tetrahydro-triazol-1,2- oder -1,4-yl, 1,2,4-Tetrahydro-triazol-1,2- oder 3,5-yl, 1,2- oder 2,5-Tetrahydrotetrazolyl, 1,2,3-Tetrahydro-oxadiazol-2,3-, -3,4-, -4,5- oder -1,5-yl, 1,2,4-Tetrahydro-oxadiazol-2,3-, -3,4- oder -4,5-yl, 1,3,4-Tetrahydro-thiadiazol-2,3-, -3,4-, -4,5- oder -1,5-yl, 1,2,4-Tetrahydro-thiadiazol-2,3-, -3,4-, -4,5-oder -1,5-yl, 1,2,3-Thiadiazol-2,3-, -3,4-, -4,5- oder -1,5-yl, 2,3- oder 3,4-Morpholinyl, 2,3-, 3,4- oder 2,4-Thiomorpholinyl.

R⁶ ist ein ein- oder zweikerniger gesättigten, ungesättigten oder aromatischen Heterocyclus, vorzugsweise 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 2-, 3-, 4-, 5- oder 6-2H-Thiopyranyl, 2-, 3- oder 4-4-H-Thiopyranyl, 3- oder 4-Pyridazinyl, Pyrazinyl, 2-, 3-, 4-, 5- 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7-oder 8-Isochinolyl, 3-, 4-, 5-, 6-, 7- oder 8-Cinnolinyl, 2-, 4-, 5-, 6-, 7- oder 8-Chinazolinyl.
Die heterocyclischen Reste können auch teilweise oder vollständig hydriert sein.
R⁶ kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder-4-pyrazolyl, 1,4-Dihydro-1-, -2-, -3-oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1 -,-2-,-3-, -4-, -5-, -6-, -7- oder -8-isochinolyl.

Die genannten heterocyclischen Ringe können auch ein-, zwei- oder dreifach durch Hal, A, -CO-A, OA, CN, COOH, COOA, CONHA, NO₂, =NH oder =O substituiert sein.

R⁶ bedeutet besonders bevorzugt 1*H*-Imidazol-2-yl, Thiazol-2-yl, 1*H-*Benzimidazol-2-yl, 2*H*-Pyrazol-2-yl, 1*H*-Tetrazol-5-yl, 2-Imino-imidazolidin-4-on-5-yl, 1-A-1,5-dihydro-imidazol-4-on-2-yl, Pyrimidin-2-yl oder 1,4,5,6-Tetrahydro-pyrimidin-2-yl, ganz besonders bevorzugt ist 1*H*-Imidazol-2-yl.
R³ bedeutet vorzugsweise H.
R⁴ bedeutet vorzugsweise H.
R⁹ bedeutet vorzugsweise H.

R¹⁰ bedeutet vorzugsweise H, Alkyl mit 1, 2, 3, 4, 5 oder 6 C-Atomen, Campher-10-yl oder Benzyl.

In dem erfindungsgemäßen Verfahren bedeutet in Schritt b) in den Verbindungen der Formel IV und V
R⁸ vorzugsweise Methylsulfonyl, Trifluormethylsulfonyl, Phenylsulfonyl oder p-Tolylsulfonyl.

Dementsprechend sind Gegenstand der Erfindung insbesondere diejenigen Verfahren zur Herstellung von Verbindungen der Formel I, in denen mindestens einer der genannten Reste eine der vorstehend angegebenen bevorzugten Bedeutungen hat. Einige bevorzugte Gruppen von Verbindungen, die durch das erfindungsgemäße Verfahren hergestellt werden, können durch die folgenden Teilformeln Ia bis If ausgedrückt werden, die der Formel I entsprechen und worin die nicht näher bezeichneten Reste die bei der Formel I angegebene Bedeutung haben, worin jedoch
in Ia)
   - R³: H und
   - R⁴: H bedeuten;
in Ib)
   - R⁶: 1*H*-Imidazol-2-yl, Thiazol-2-yl, 1*H*-Benzimidazol-2-yl, 2*H*-Pyrazol-2-yl, 1*H*-Tetrazol-5-yl, 2-Imino-imidazolidin-4-on-5-yl, 1-A-1,5-dihydro-imidazol-4-on-2-yl, Pyrimidin-2-yl oder 1,4,5,6-Tetrahydro-pyrimidin-2-yl bedeutet;
in Ic)
   - R⁹: H bedeutet;
in Id)
   - R¹⁰: H, Alkyl mit 1, 2, 3, 4, 5 oder 6 C-Atomen, Campher-10-yl oder Benzyl bedeutet;
in Ie)
   - A: unsubstituiertes Alkyl oder Cycloalkyl mit 1-15 C-Atomen bedeutet;
in If)
   - A: Alkyl mit 1,2,3,4,5 oder 6 C-Atomen,
   - R¹⁰: H, Alkyl mit 1, 2, 3, 4, 5 oder 6 C-Atomen, Campher-10-yl oder Benzyl
bedeuten,
sowie deren Salze, Solvate und Stereoisomere.

Bevorzugt ist ein Verfahren zur Herstellung von Verbindungen der Formel I, worin
- R¹: H, Alkyl mit 1, 2, 3, 4, 5 oder 6 C-Atomen oder Benzyl,
- R²: R¹⁰, CO-R¹⁰, COOR¹⁰ oder SO₂R¹⁰,
- R³: H,
- R⁴: H,
- R⁵: R⁶,
- R⁶: einen ein- oder zweikernigen gesättigten, ungesättigten oder aromatischen Heterocyclus mit 1 bis 4 N-, O- und / oder S-Atomen, der unsubstituiert oder ein-, zwei- oder dreifach durch Hal, A, -CO-A, OA, CN, COOH, COOA, CONHA, NO₂, =NH oder =O substituiert sein kann,
- X: NH,
- R¹⁰: H, Alkyl mit 1, 2, 3, 4, 5 oder 6 C-Atomen, Campher-1 0-yl oder Benzyl,
- A: Alkyl mit 1, 2, 3, 4, 5 oder 6 C-Atomen,
- Hal: F, Cl, Br oder I
bedeuten,
sowie deren Salze, Solvate und Stereoisomere.

In der letztgenannten bevorzugten Ausführungsform bedeutet R⁶ vorzugsweise 1*H*-Imidazol-2-yl, Thiazol-2-yl, 1*H*-Benzimidazol-2-yl, 2H-Pyrazol-2-yl, 1*H*-Tetrazol-5-yl, 2-Imino-imidazolidin-4-on-5-yl, 1-A-1,5-dihydro-imidazol-4-on-2-yl, Pyrimidin-2-yl oder 1,4,5,6-Tetrahydropyrimidin-2-yl.

Bevorzugt ist weiterhin ein Verfahren zur Herstellung von Verbindungen der Formel la 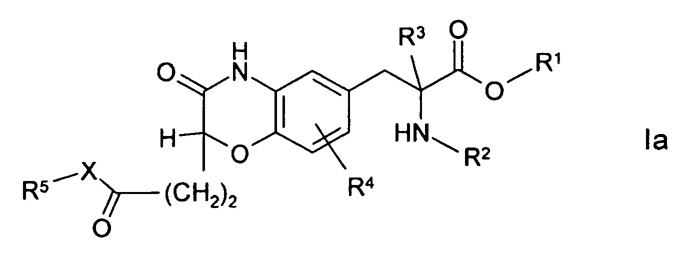 worin
- R¹: H oder Alkyl mit 1, 2, 3 oder 4 C-Atomen,
- R²: R¹⁰, CO-R¹⁰, COOR¹⁰ oder SO₂R¹⁰,
- R³: H,
- R⁴: H,
- R⁵: R⁶,
- X: NH,
- R⁶: 1*H*-Imidazol-2-yl, Thiazol-2-yl, 1*H*-Benzimidazol-2-yl, 2H-Pyrazol-2-yl, 1*H*-Tetrazol-5-yl, 2-Imino-imidazolidin-4-on-5-yl, 1-A-1,5-dihydro-imidazol-4-on-2-yl, Pyrimidin-2-yl oder 1,4,5,6-Tetrahydropyrimidin-2-yl,
- R¹⁰: H oder Alkyl mit 1, 2, 3, 4, 5 oder 6 C-Atomen und
- A: Alkyl mit 1, 2, 3, 4, 5 oder 6 C-Atomen,
bedeuten,
sowie deren Salze, Solvate und Stereoisomere,
wobei
a) 5-Oxo-tetrahydrofuran-2-carbonsäure mit einer Verbindung der Formel II

   R⁷-L II

   worin
   - R⁷: Benzyl oder Alkyl mit 1, 2, 3, 4, 5 oder 6 C-Atomen und
   - L: Cl, Br, 1 oder eine freie oder reaktionsfähig funktionell abgewandelte OH-Gruppe
   bedeuten,
   zu einer Verbindung der Formel III 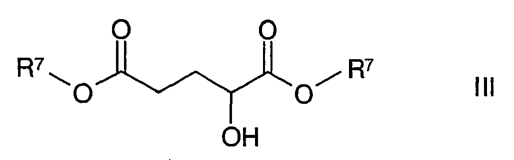 worin
   - R⁷: Benzyl oder Alkyl mit 1, 2, 3, 4, 5 oder 6 C-Atomen bedeutet,
   umgesetzt wird,
b) dann eine Verbindung der Formel III mit einer Verbindung der Formel IV

   R⁸-L IV

   worin
   - R⁸: Alkylsulfonyl mit 1-6 C-Atomen oder Arylsulfonyl mit 6-10 C-Atomen und
   - L: Cl, Br, I oder eine freie oder reaktionsfähig funktionell abgewandelte OH-Gruppe
   bedeuten,
   zu einer Verbindung der Formel V 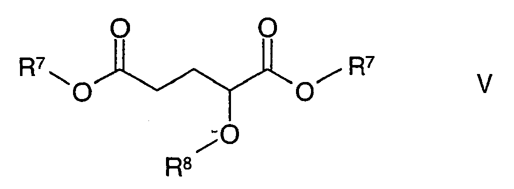 worin
   - R⁷: Benzyl oder Alkyl mit 1, 2, 3, 4, 5 oder 6 C-Atomen und
   - R⁸: Alkylsulfonyl mit 1-6 C-Atomen oder Arylsulfonyl mit 6-10 C-Atomen bedeuten,
   umgesetzt wird,
c) dann eine Verbindung der Formel III oder V mit einer Verbindung der Formel VI 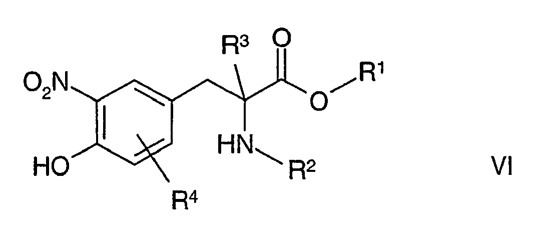 worin
   - R¹: H oder Alkyl mit 1, 2, 3 oder 4 C-Atomen,
   - R²: R¹⁰, CO-R¹⁰, COOR¹⁰ oder SO₂R¹⁰,
   - R³: H,
   - R⁴: H,
   - R¹⁰: H oder Alkyl mit 1, 2, 3, 4, 5 oder 6 C-Atomen und
   bedeuten,
   zu einer Verbindung der Formel VII 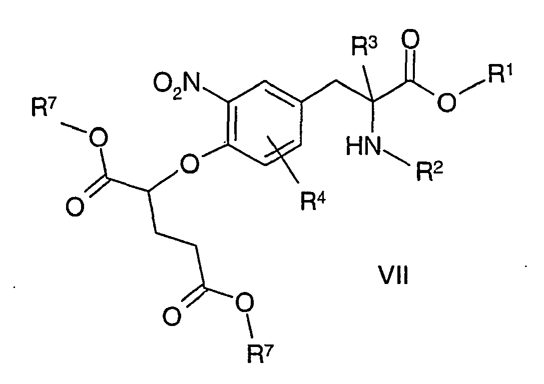 worin
   - R¹: H oder Alkyl mit 1, 2, 3 oder 4 C-Atomen,
   - R²: R¹⁰ CO-R¹⁰ COOR¹⁰ oder SO₂R¹⁰,
   - R³: H,
   - R⁴: H,
   - R⁷: Benzyl oder Alkyl mit 1, 2, 3, 4, 5 oder 6 C-Atomen,
   - R¹⁰: H oder Alkyl mit 1, 2, 3, 4, 5 oder 6 C-Atomen
   bedeuten,
   umgesetzt wird,
d) dann eine Verbindung der Formel VII zu einer Verbindung der Formel VIII 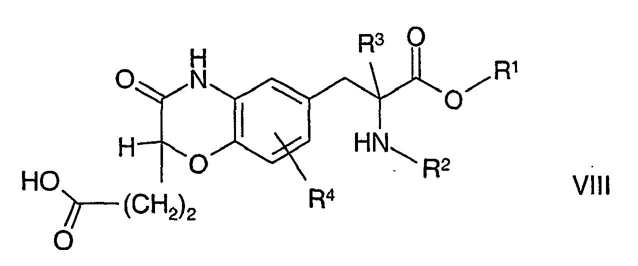 worin
   - R¹: H oder Alkyl mit 1, 2, 3 oder 4 C-Atomen,
   - R²: R¹⁰, CO-R¹⁰, COOR¹⁰ oder SO₂R¹⁰,
   - R³: H,
   - R⁴: H,
   - R¹⁰: H oder Alkyl mit 1, 2, 3, 4, 5 oder 6 C-Atomen bedeuten,
   umgesetzt wird,
e) dann eine Verbindung der Formel VIII
   mit einer Verbindung der Formel X

   R⁶-NH₂ X
worin
- R⁶: 1*H*-Imidazol-2-yl, Thiazol-2-yl, 1*H*-Benzimidazol-2-yl, 2H-Pyrazol-2-yl, 1*H*-Tetrazol-5-yl, 2-Imino-imidazolidin-4-on-5-yl, 1-A-1,5-dihydro-imidazol-4-on-2-yl, Pyrimidin-2-yl oder 1,4,5,6-Tetrahydropyrimidin-2-yl,
bedeutet,
zu einer Verbindung der Formel la umsetzt,
gegebenenfalls anschließend einen Rest R² in einen anderen Rest R² umwandelt,
indem man eine Verbindung der Formel Ia, worin R² H bedeutet mit einer Verbindung ausgewählt aus der Gruppe

L-R^{10'}, L-CO-R^{10'}, L-COOR^{10'}, R^{10'}-SO₂L,

worin
- R^{10'}: Alkyl mit 1, 2, 3, 4, 5 oder 6 C-Atomen und
- L Cl, Br, I: oder eine freie oder reaktionsfähig funktionell abgewandelte OH-Gruppe bedeutet,
umsetzt,
und / oder eine Base oder Säure der Formel la in eines ihrer Salze umwandelt.

Gegenstand der Erfindung ist weiterhin ein Verfahren von Herstellung von (S)-2-(2,2-Dimethyl-propoxycarbonylamino)-3-{(S)-2-[2-(1*H*-imidazol-2-ylcarbamoyl)-ethyl]-3-oxo-3,4-dihydro-2*H*-benzo[1,4]oxazin-6-yl}-propionsäure,
wobei
a) (S)-3-Nitro-tyrosin-ethylester-tosylat mit Chlorameisensäure-2,2-dimethylpropylester zu (S)-2-(2,2-Dimethyl-propoxycarbony(amino)-3-(4-hydroxy-3-nitro-phenyl)-propionsäure-ethylester **12** umgesetzt wird,
b) **12** mit 2-Hydroxy-pentandicarbonsäure-dibenzylester zu (R)-2-{4-[(S)-2-(2,2-Dimethyl-propoxycarbony(amino)-2-ethoxycarbonylethyl]-2-nitro-phenoxy}-pentanedicarbonsäure-dibenzylester **13** umgesetzt wird,
c) **13** zu (2S)-3-[(2S)-2-(2-Carboxy-ethyl)-3-oxo-3,4-dihydro-2H benzo[1,4]oxazin-6-yl]-2-(2,2-dimethyl-propoxycarbonylamino)-propionsäure-ethylester **14** cyclisiert wird,
d) **14** mit 2-Amino-imidazol zu 2-(S)-(2,2-Dimethyl-propoxy-carbonylamino)-3-{3-oxo-2-(S)-[2-(1*H*-imidazol-2-ylcarbamoyl)-ethyl]-3, 4-dihydro-2*H*-benzo[1,4]oxazin-6-yl}-propionsäure-ethylester **15** umgesetzt wird und
e) **15** durch Esterspaltung zu (S)-2-(2,2-Dimethyl-propoxycarbonylamino)-3-{(S)-2-[2-(1*H*-imidazol-2-ylcarbamoyl)-ethyl]-3-oxo-3,4-dihydro-2*H* benzo[1,4]oxazin-6-yl}-propionsäure **16** umgesetzt wird.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC, 2-lodethoxycarbonyl; Aralkyloxycarbonyl wie CBZ ("Carbobenzoxy"), 4-Methoxybenzyloxycarbonyl, FMOC; Arylsulfonyl wie Mtr. Bevorzugte Aminoschutzgruppen sind BOC und Mtr, ferner CBZ, Fmoc, Benzyl und Acetyl.

Die Abspaltung der Aminoschutzgruppe gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Hydrogenolytisch entfernbare Schutzgruppen (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammoniumformiat (anstelle von Wasserstoff) an Pd/C in Methanol/DMF bei 20-30°.

In den Verbindungen der Formel II, IV, sowie in den Verbindungen ausgewählt aus den Gruppen
L-R¹⁰, L-CO-R¹⁰, L-COOR⁶, L-COOR¹⁰, R¹⁰-N=C=O, R⁶-SO₂L, R¹⁰-SO₂L (Stufe e) ii)) bzw. L-R¹⁰', L-CO-R^{10'}, L-COOR⁶, L-COOR^{10'}, R^{10'}-N=C=O, R⁶-SO₂L, R^{10'}-SO₂L
bedeutet L vorzugsweise Cl, Br, I oder eine freie oder reaktionsfähig abgewandelte OH-Gruppe wie Alkylsulfonyloxy mit 1-6 C-Atomen (bevorzugt Methylsulfonyloxy) oder Arylsulfonyloxy mit 6-10 C-Atomen (bevorzugt Phenyl- oder p-Tolylsulfonyloxy).

Die Umsetzung von 5-Oxo-tetrahydrofuran-2-carbonsäure mit einer Verbindung der Formel II zu einer Verbindung der Formel III erfolgt in der Regel in einem inerten Lösungsmittel, in Gegenwart eines säurebindenden Mittels vorzugsweise einer organischen Base wie Triethylamin, Dimethylanilin, Pyridin oder Chinolin.
Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.

Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat, Wasser oder Gemische der genannten Lösungsmittel.

Die Umsetzung einer Verbindung der Formel III mit einer Verbindung der Formel IV zu einer Verbindung der Formel V erfolgt in der Regel in einem inerten Lösungsmittel, in Gegenwart eines säurebindenden Mittels, in einem inerten Lösungsmittel und bei Temperaturen, wie bei der Umsetzung von 5-Oxo-tetrahydrofuran-2-carbonsäure mit einer Verbindung der Formel II beschrieben.

Erfolgt die weitere Umsetzung durch Reaktion einer Verbindung der Formel V mit einer Verbindung der Formel VI zu einer Verbindung der Formel VII, so erfolgt diese Umsetzung in der Regel in einem inerten Lösungsmittel, in Gegenwart eines säurebindenden Mittels, in einem inerten Lösungsmittel und bei Temperaturen, wie bei der Umsetzung von 5-Oxo-tetrahydrofuran-2-carbonsäure mit einer Verbindung der Formel II beschrieben.

Erfolgt alternativ die weitere Umsetzung durch Reaktion einer Verbindung der Formel III mit einer Verbindung der Formel VI zu einer Verbindung der Formel VII, so geschieht dies unter Bedingungen der Mitsunobu-Reaktion, die dem Fachmann für diese Reaktion bekannt sind.

Die Umsetzung einer Verbindung der Formel VII zu einer Verbindung der Formel VIII erfolgt, falls R⁷ Benzyl bedeutet, vorzugsweise hydrogenolytisch unter Bedingungen wie oben beschrieben. Dabei erfolgt die Reduktion der Nitrogruppe sowie die Abspaltung der Benzylgruppen unter Cyclisierung.

Alternativ erfolgt die Umsetzung einer Verbindung der Formel VII zu einer Verbindung der Formel VIII
a) durch Reduktion der Nitrogruppe, anschließender Esterspaltung und Cyclisierung oder
b) durch Esterspaltung, anschließender Reduktion der Nitrogruppe und Cyclisierung.

Eine Esterspaltung erfolgt unter Standardbedingungen. Zweckmäßig erfolgt dies durch Solvolyse oder Hydrogenolyse, wie oben angegeben, z.B. mit NaOH oder KOH in Dioxan-Wasser bei Temperaturen zwischen 0 und 60° C, vorzugsweise zwischen 10 und 40° C.

Die Umsetzung einer Verbindung der Formel VIII oder VIIIa mit einer Verbindung der Formel IX oder X zu einer Verbindung der Formel I erfolgt in der Regel in einem inerten Lösungsmittel, in Gegenwart eines säurebindenden Mittels, in einem inerten Lösungsmittel und bei Temperaturen, wie bei der Umsetzung von 5-Oxo-tetrahydrofuran-2-carbonsäure mit einer Verbindung der Formel II beschrieben.

In den Verbindungen der Formel VIIIa bedeutet L vorzugsweise Cl, Br,I oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy). Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart;) beschrieben.
Aktivierte Ester werden zweckmäßig in situ gebildet, z. B. durch Zusatz von HOBt oder N-Hydroxysuccinimid.

Gegebenenfalls wird in einer Verbindung der Formel I ein Rest R² in einen anderen Rest R² umgewandelt. Vorzugsweise geschieht dies mit Verbindungen der Formel I, worin R² H bedeutet. Die Umsetzung erfolgt mit einer Verbindung ausgewählt aus der Gruppe L-R¹⁰, L-CO-R¹⁰, L-COOR⁶, L-COOR¹⁰, R¹⁰-N=C=O, R⁶-SO_{2L}, R¹⁰-SO₂L,
worin
R¹⁰ A, Ar oder Aralkylen mit 7-14 C-Atomen,
A und Ar die bei Anspruch 1 angegebenen Bedeutungen haben und L Cl, Br, I oder eine freie oder reaktionsfähig funktionell abgewandelte OH-Gruppe bedeutet.
Die Umsetzung erfolgt in der Regel in einem inerten Lösungsmittel, in Gegenwart eines säurebindenden Mittels, in einem inerten Lösungsmittel und bei Temperaturen, wie bei der Umsetzung von 5-Oxo-tetrahydrofuran-2-carbonsäure mit einer Verbindung der Formel II beschrieben.

Die Umwandlung einer Cyangruppe in eine Amidinogruppe erfolgt durch Umsetzung mit z.B. Hydroxylamin und anschließender Reduktion des N-Hydroxyamidins mit Wasserstoff in Anwesenheit eines Katalysators wie z.B. Pd/C.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und Disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits kann eine Säure der Formel I durch Umsetzung mit einer Base in eines ihrer physiologisch unbedenklichen Metall- oder Ammoniumsalze übergeführt werden. Als Salze kommen dabei insbesondere die Natrium-, Kalium-, Magnesium-, Calcium- und Ammoniumsalze in Betracht, ferner substituierte Ammoniumsalze, z. B. die Dimethyl-, Diethyl- oder Diisopropyl-ammoniumsalze, Monoethanol-, Diethanol- oder Diisopropylammoniumsalze, Cyclohexyl-, Dicyclohexylammoniumsalze, Dibenzylethylendiammoniumsalze, weiterhin z. B. Salze mit Arginin oder Lysin.

Die Verbindungen der Formel I enthalten ein oder mehrere chirale Zentren und können daher in racemischer oder in optisch-aktiver Form vorliegen. Erhaltene Racemate können nach an sich bekannten Methoden mechanisch oder chemisch in die Enantiomeren getrennt werden. Vorzugsweise werden aus dem racemischen Gemisch durch Umsetzung mit einem optisch aktiven Trennmittel Diastereomere gebildet. Als Trennmittel eignen sich z.B. optisch aktive Säuren, wie die D- und L-Formen von Weinsäure, Diacetylweinsäure, Dibenzoylweinsäure, Mandelsäure, Äpfelsäure, Milchsäure oder die verschiedenen optisch aktiven Camphersulfonsäuren wie β-Camphersulfonsäure. Vorteilhaft ist auch eine Enantiomerentrennung mit Hilfe einer mit einem optisch aktiven Trennmittel (z.B. Dinitrobenzoylphenylglycin) gefüllten Säule; als Laufmittel eignet sich z.B. ein Gemisch Hexan/Isopropanol/Acetonitril, z.B. im Volumenverhältnis 82:15:3.

Natürlich ist es auch möglich, optisch aktive Verbindungen der Formel I nach den oben beschriebenen Methoden zu erhalten, indem man Ausgangsstoffe verwendet, die bereits optisch aktiv sind.

Gegenstand der Erfindung sind weiterhin die Zwischenverbindungen der Formel VII 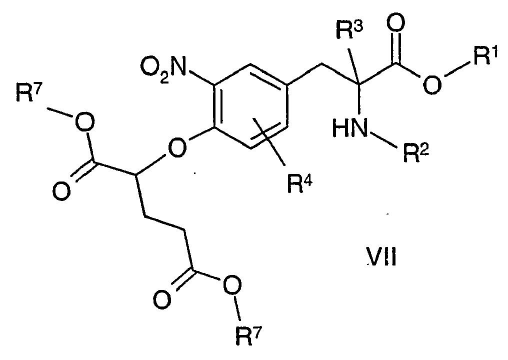 worin
- R¹: H oder Alkyl mit 1, 2, 3 oder 4 C-Atomen,
- R²: R¹⁰ CO-R¹⁰ COOR¹⁰ oder SO₂R¹⁰,
- R³: H,
- R⁴: H,
- R⁷: Benzyl oder Alkyl mit 1, 2, 3, 4, 5 oder 6 C-Atomen,
- R⁹: H, Hal, OA, NHA, NAA', NHAcyl, OAcyl, CN, NO₂, SA, SOA, SO₂A, SO₂Ph oder SO₃H,
- R¹⁰: H, A, Ar oder Aralkylen mit 7-14 C-Atomen,
- A, A': jeweils unabhängig voneinander H oder unsubstituiertes oder ein-, zwei- oder dreifach durch R⁹ substituiertes Alkyl oder Cycloalkyl mit 1-15 C-Atomen und worin eine, zwei- oder drei Methylengruppen durch N, 0 und/oder S ersetzt sein können,
- Ar: unsubstituiertes oder ein-, zwei- oder dreifach durch A und/oder R⁹ substituiertes ein- oder zweikerniges aromatisches Ringsystem mit 0, 1, 2, 3 oder 4 N-, O- und/oder S-Atomen,
bedeuten,
sowie deren Salze, Solvate und Stereoisomere.

Bevorzugt sind weiterhin die Zwischenverbindungen der Formel VII 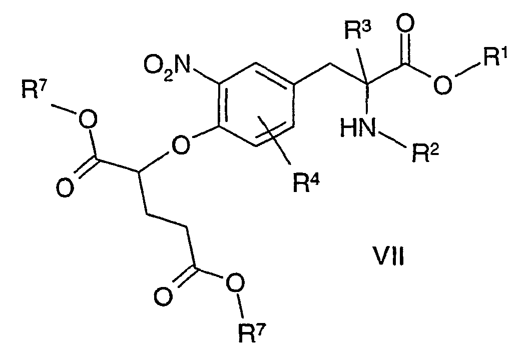 worin
- R¹: H oder Alkyl mit 1, 2, 3 oder 4 C-Atomen,
- R²: R¹⁰, CO-R¹⁰, COOR¹⁰ oder SO₂R¹⁰,
- R³: H,
- R⁴: H,
- R⁷: Benzyl oder Alkyl mit 1, 2, 3, 4, 5 oder 6 C-Atomen,
- R¹⁰: H oder Alkyl mit 1, 2, 3, 4, 5 oder 6 C-Atomen
bedeuten,
sowie deren Salze, Solvate und Stereoisomere.

Darüberhinaus ist Gegenstand der Erfindung ein Verfahren zur Herstellung von Verbindungen der Formel VII,
sowie deren Salze, Solvate und Stereoisomere,
wobei
a) 5-Oxo-tetrahydrofuran-2-carbonsäure mit einer Verbindung der Formel II

   R⁷-L II

   worin
   - R⁷: Benzyl oder Alkyl mit 1, 2, 3, 4, 5 oder 6 C-Atomen und
   - L: Cl, Br, I oder eine freie oder reaktionsfähig funktionell abgewandelte OH-Gruppe
   bedeuten,
   zu einer Verbindung der Formel III 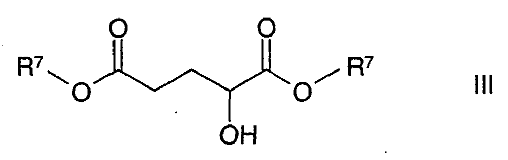 worin
   - R⁷: Benzyl oder Alkyl mit 1, 2, 3, 4, 5 oder 6 C-Atomen bedeutet,
   umgesetzt wird,
b) dann eine Verbindung der Formel III mit einer Verbindung der Formel IV

   R⁸-L IV

   worin
   - R⁸: Alkylsulfonyl mit 1-6 C-Atomen oder Arylsulfonyl mit 6-10 C-Atomen und
   - L: Cl, Br, I oder eine freie oder reaktionsfähig funktionell abgewandelte OH-Gruppe
   bedeuten,
   zu einer Verbindung der Formel V 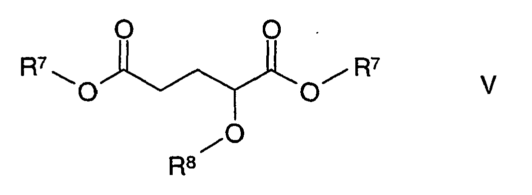 worin
   - R⁷: Benzyl oder Alkyl mit 1,2,3,4,5 oder 6 C-Atomen und
   - R⁸: Alkylsulfonyl mit 1-6 C-Atomen oder Arylsulfonyl mit 6-10 C-Atomen bedeuten,
   umgesetzt wird,
c) dann eine Verbindung der Formel III oder V mit einer Verbindung der Formel VI 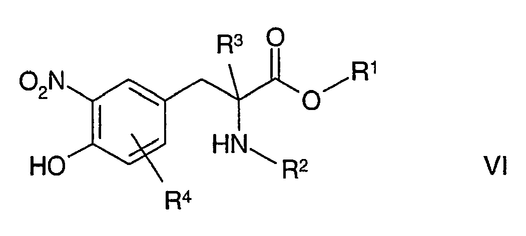
worin
- R¹: H oder Alkyl mit 1, 2, 3 oder 4 C-Atomen,
- R²: R¹⁰, CO-R¹⁰, COOR¹⁰ oder SO₂R¹⁰,
- R³: H,
- R⁴: H,
- R⁹: H, Hal, OA, NHA, NAA', NHAcyl, OAcyl, CN, NO₂, SA, SOA, SO₂A, SO₂Ph oder SO₃H,
- R¹⁰: H, A, Ar oder Aralkylen mit 7-14 C-Atomen,
- A, A': jeweils unabhängig voneinander H oder unsubstituiertes oder ein-, zwei- oder dreifach durch R⁹ substituiertes Alkyl oder Cycloalkyl mit 1-15 C-Atomen und worin eine, zwei- oder drei Methylengruppen durch N, O und/oder S ersetzt sein können,
- Ar: unsubstituiertes oder ein-, zwei- oder dreifach durch A und/oder R⁹ substituiertes ein- oder zweikerniges aromatisches Ringsystem mit 0, 1, 2, 3 oder 4 N-, O- und/oder S-Atomen,
bedeuten,
zu einer Verbindung der Formel VII umgesetzt wird,
und / oder eine Säure der Formel VII in eines ihrer Salze umgewandelt wird.

Die Umsetzungen erfolgen analog den Bedingungen wie für die Herstellung der Verbindungen der Formel I beschrieben.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation.

| | |
|---|---|
| Massenspektrometrie (MS): | EI (Elektronenstoß-Ionisation) M⁺ |
| | FAB (Fast Atom Bombardment) (M+H)⁺ |

### Beispiel 1

Die Herstellung von 2-(S)-Amino-3-{3-oxo-2-(R)-[2-(1*H*-imidazol-2-ylcarbamoyl)-ethyl]-3,4-dihydro-2*H*-benzo[1,4]oxazin-6-yl}-propionsäure-ethylester Hydrochlorid (**6**) erfolgt analog nachstehendem Schema:

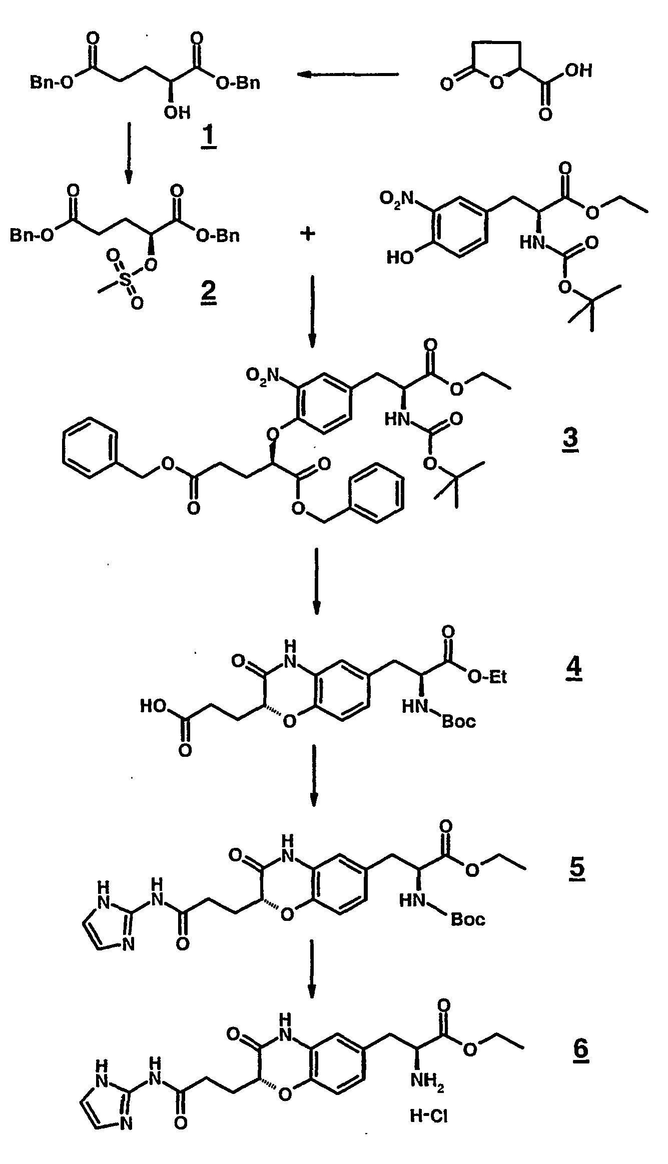

### 1.1 (S)-2-Hydroxy-pentandicarbonsäure-dibenzylester 1

Zu einer Lösung von 100 g (S)-5-Oxo-furan-2-carbonsäure in 1 L Wasser gibt man 300 g Cäsiumcarbonat. Die Mischung wird unter Reflux erhitzt und 18 Stunden gerührt. Das Wasser wird entfernt und der Rückstand wird in 750 ml Methanol gelöst. Man gibt 200 g Silicagel zu und rührt 15 Minuten. Das Lösungsmittel wird entfernt und man erhält das bis-Cäsiumsalz auf Silicagel als weißes Pulver, das im Vakuum 18 Stunden bei 60° getrocknet wird.
Das Rohprodukt wird in 1 L absolutem DMF suspendiert, 183 ml Benzylbromid zugegeben und bei Raumtemperatur 16 Stunden gerührt. Nach Beendigung der Reaktion (HPLC Kontrolle) wird das Silicagel durch Filtration abgetrennt, und die Lösung in 2 L Wasser gegossen. Nach üblicher Aufarbeitung erhält man **1** als Öl.
Ausbeute: 237 g (94 %)
HPLC (Chromolith™ Performance RP-18e, Eluent: Gradientensystem, Wasser + 0.1% TFA / Acetonitril + 0. 1 % TFA 99:1 → 1:99 in 10 min, 3 ml/min) Rₜ = 5.25 min
Chirale HPLC (Chiralcel OD-H, 250 mm, Heptan / Ethanol 85:15, 0.8 ml/min): Rₜ = 12.95 min
ESI-MS: M⁺¹ = 329
¹H-NMR (400 MHz, DMSO): entspricht
[α]_{D} = -9.6° (c = 1.1 in Ethanol).

### 1.2 (S)-2-Methansulfonyloxy-pentandicarbonsäure-dibenzylester 2

Zu einer Lösung von 236,9 g **1** in 1,2 L Dichlormethan gibt man 500 ml Triethylamin. Unter Eiskühlung wird eine Lösung von 83,6 ml Methansulfonylchlorid in 200 ml Dichlormethan zugegeben. Man rührt 30 Minuten bei 5 - 10°, entfernt das ausgefallene Triethylamin Hydrochlorid, arbeitet wie üblich auf und erhält **2** als Öl.
Ausbeute: 277 g (94 %)
HPLC (Chromolith™ Performance RP-18e, Eluent Gradientensystem: Wasser + 0.1 % TFA / Acetonitril + 0.1%TFA 99:1 → 1:99 in 10 min, 3 ml/min) Rₜ = 5.92 min
DC: Si-60, Toluol / Aceton 9:1, R_{f} = 0.5
ESI-MS: M⁺¹ = 407
[α]_{D} = -31.1° (c = 1.01 in Chloroform).

### 1.3 2-(R)-[4-(2-(S)-tert.-Butoxycarbonylamino-2-ethoxycarbonyl-ethyl)-2-nitro-phenoxy]-pentandicarbonsäure-dibenzylester 3

Zu einer Lösung von 178,6 g N-Boc-L-Tyrosin-ethylester und 257 g **2** in 1,25 L Acetonitril gibt man 174,1 g Kaliumcarbonat und erhitzt 16 Stunden unter Rückfluß. Nach Abtrennung der unlöslichen Salze arbeitet man wie üblich auf und erhält **3**.
Ausbeute: 369 g (99%)
HPLC (Chromolith™ Performance RP-18e, Eluent Gradientensystem: Wasser + 0.1 % TFA / Acetonitril + 0.1 % TFA 99:1 → 1:99 in 10 min, 3 ml/min) Rₜ = 7.04 min, Reinheit 90%.
Chiral HPLC: (Chiralpak™ AD, 250 mm, Heptan / Isopropanol 60:40, 0.8 ml/min) Rₜ = 27.23 min, Reinheit = 96.6 % (de [Diastereomerenüberschuß] = 93.2%)
DC: Si-60, Toluol / Aceton 9:1, Rf = 0.38
ESI-MS: M⁺¹ = 665
[α]_{D} = -35.8° (c = 1.02 in Ethanol).

### 1.4 2-(S)-tert.-Butoxycarbonylamino-3-[2-(R)-(2-carboxy-ethyl)-3-oxo-3,4-dihydro-2H benzo[1,4]oxazin-6-yl]-propionsäure-ethylester 4

Durch eine Lösung von 369 g **3** in 2,6 L Essigsäure leitet man bei 60° in Gegenwart von 95 g Palladium (5% auf Aktivkohle) 6 Stunden lang Wasserstoff. Nach Entfernen des Katalysators und üblicher Aufarbeitung erhält man **4**.
Ausbeute: 164 g (75%)
HPLC (Chromolith™ Performance RP-18e, Eluent Gradientensystem: Wasser + 0.1% TFA / Acetonitril + 0.1% TFA 99:1 → 1:99 in 10 min, 3 ml/min) Rₜ = 4.51 min.
DC: Si-60, Dichloromethan / Methanol 9:1, Rf = 0.35
ESI-MS: M⁺¹ = 437
[α]_{D} = +14.0° (c = 1.0 in Methanol)
F. 154-158°.

### 1.5 2-(S)-tert.-Butoxycarbonylamino-3-13-oxo-2-(R)-[2-(1H-imidazol-2-ylcarbamoyl)-ethyl]-3,4-dihydro-2H-benzo[1,4]oxazin-6-yl}-propionsäure-ethylester 5

Zu einer Lösung von 163 g **4**, 74 g 2-Amino-imidazol Sulfat, 155,7 g TBTU und 15,13 g HOBt in 1,2 L absolutem DMF gibt man 128,54 g Kaliumcarbonat und rührt unter Stickstoff 16 Stunden bei 70°. Man gibt weitere 25 g 2-Amino-imidazol Sulfat, 51 g TBTU, 5 g HOBt und 26 g Kaliumcarbonat zu und rührt weitere 5 Stunden. Die unlöslichen Salze werden abgetrennt, das DMF entfernt und der Rückstand in 3 L Eiswasser gegossen. Der Niederschlag wird abgetrennt, mit Wasser gewaschen und an der Luft getrocknet. Umkristallisation aus Ethanol ergibt **5**.
Ausbeute: 125 g (67%)
HPLC (Chromolith™ Performance RP-18e, Eluent Gradientensystem: Wasser + 0.1 1% TFA / Acetonitril + 0.1% TFA 99:1 →1 :99 in 10 min, 3 ml/min) Rₜ = 4.13 min.
Chiral HPLC: (Chiralpak™ AD, 250 mm, Heptan / Ethanol 40:60, 0.8 ml/min) Rₜ = 16.21 min, Reinheit = 96.6 % (de = 93.2%)
DC: Si-60, Dichloromethan / Methanol 9:1, Rf = 0.28
ESI-MS: M⁺¹ = 502
[α]_{D} = +17.3° (c = 1.04 in DMSO)
F. 215°C (Zersetzung).

### 1.6 2-(S)-Amino-3-{3-oxo-2-(R)-[2-(1H-imidazol-2-ylcarbamoyl)-ethyl]-3,4-dihydro-2H-benzo[1,4]oxazin-6-yl}-propionsäure-ethylester, Hydrochlorid 6

Zu einer Suspension von 112 g **5** in 1 L Dioxan gibt man 0,9 L einer 4,5 M Lösung von HCl in Dioxan und rührt 16 Stunden nach.
Der Niederschlag wird abgetrennt, mit wenig Dioxan und 3x mit Petrolether gewaschen. Nach trocknen im Vakuum erhält man **6** als weißes Produkt.
Ausbeute: 112,6 g (quant.)
HPLC (Chromolith™ Performance RP-18e, Eluent Gradientensystem: Wasser + 0.1% TFA / Acetonitril + 0.1% TFA 99:1 → 1:99 in 10 min, 3 ml/min) Rₜ = 2.67 min.
ESI-MS: M⁺¹ = 402
F. 220° (Zersetzung)
[α]_{D} = +34.1 ° (c = 1.0 in Wasser).

Analog erhält man durch Umsetzung von
2-(S)-tert.-Butoxycarbonylamino-3-[2-(R)-(2-carboxy-ethyl)-3-oxo-3,4-dihydro-2*H*-benzo[1,4]oxazin-6-yl]-propionsäure-ethylester **4**
   mit
2-Amino-benzimidazol
   die Verbindung
2-(S)-tert.-Butoxycarbonylamino-3-{3-oxo-2-(R)-[2-(1H-benzimidazol-2-ylcarbamoyl)-ethyl]-3,4-dihydro-2*H*-benzo[1,4]oxazin-6-yl}-propionsäure-ethylester **5-a.**

### Beispiel 2

Die Herstellung von (S)-2-(2,2-Dimethyl-propoxycarbonylamino)-3-{(R)-2-[2-(1*H*-imidazol-2-ylcarbamoyl)-ethyl]-3-oxo-3,4-dihydro-2*H* benzo[1,4]oxazin-6-yl}-propionsäure, Natriumsalz (**11**) erfolgt analog nachstehendem Schema:

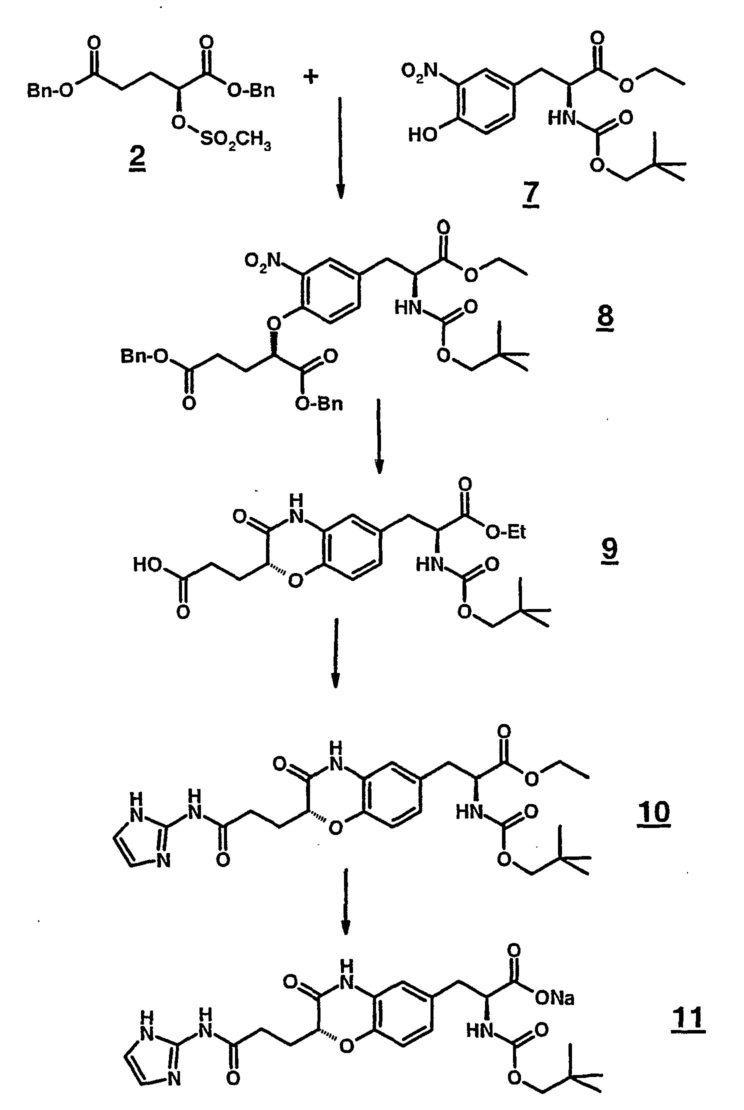

### 2.1 (S)-2-(2,2-Dimethyl-propoxycarbonylamino)-3-(4-hydroxy-3-nitrophenyl)-propionsäure-ethylester 7

Zu einer Suspension von 206,4 g L-3-Nitro-tyrosin-ethylester-tosylat in 750 ml Wasser und 250 ml THF gibt man in kleinen Portionen 81,32 g NaHCO₃. Anschließend wird eine Lösung von 60 ml 2,2-Dimethylpropylchlorformiat in 250 ml THF zugetropft und bei Raumtemperatur 2 Stunden nachgerührt. Nach Entfernen des THF wird wie üblich aufgearbeitet. Das kristallisierte Produkt **7** wird mit Petrolether gewaschen und im Vakuum getrocknet.
Ausbeute: 107 g (72%)
HPLC (Chromolith™ Performance RP-18e, Eluent Gradientensystem: Wasser + 0.1 % TFA / Acetonitril + 0.1% TFA 99:1 → 1:99 in 10 min, 3 ml/min) Rₜ = 5.68 min.
DC: Si-60, Toluol / Aceton 9:1, Rf = 0.42
ESI-MS: M⁺¹ = 369
[a]_{D} = +0.9° (c = 1.03 in Methanol)
F. 64-67°.

### 2.2 (S) 2-{4-[(S)-2-(2,2-Dimethyl-propoxycarbonylamino)-2-ethoxycarbonyl-ethyl]-2-nitro-phenoxy}-pentandicarbonsäure-dibenzyl ester 8

Zu einer Lösung von 9,85 g **7** in 100ml Acetonitril gibt man 9,24 g K₂CO₃. Dann wird eine Lösung von 16,3 g des Mesylats **2** in 50 ml Acetonitril zugetropft und 16 Stunden unter Rückfluß (Badtemperatur 80°) gerührt. Man kühlt auf Raumtemperatur ab, trennt die unlöslichen Salze ab, entfernt das Lösungsmittel, löst den Rückstand in 200 ml Ethylacetat, arbeitet wie üblich auf und erhält **8** als gelben Sirup.
Ausbeute: 23 g (75% Reinheit, enthält noch Mesylat)
HPLC (Chromolith™ Performance RP-18e, Eluent Gradientensystem: Wasser + 0.1% TFA / Acetonitril + 0.1 % TFA 99:1 → 1:99 in 10 min, 3 ml/min) Rₜ = 7.17 min.
Chiral HPLC: (Chiralpak™ AD, 250 mm, Heptan /Isopropanol 60:40, 0.8 ml/min) Rₜ = 22.35 min, Diastereomerenreinheit = 98% (de = 96%)
DC: Si-60, Toluol / Aceton 9:1, Rf = 0.38
ESI-MS: M⁺¹ = 679
[α]_{D} = -36.9° (c = 1 in Ethanol)

### 2.3 (S)-3-[(R)-2-(2-Carboxy-ethyl)-3-oxo-3,4-dihydro-2H-benzo[1,4]oxazin-6-yl]-2-(2,2-dimethyl-propoxycarbonylamino)-propionsäure-ethylester 9

Durch eine Lösung von 23 g **8** (Rohprodukt aus 2.2) in 230 ml Essigsäure leitet man bei 60° in Gegenwart von 7,5 g Palladium (10% auf Aktivkohle) 6 Stunden lang Wasserstoff. Nach Entfernen des Katalysators und üblicher Aufarbeitung und Umkristallisation aus Ethanol erhält man **9**.
Ausbeute: 10 g (87%)
HPLC (Chromolith™ Performance RP-18e, Eluent Gradientensystem: Wasser + 0.1 % TFA / Acetonitril + 0.1 % TFA 99:1 → 1:99 in 10 min, 3 ml/min) Rₜ = 4.88 min.
DC: Si-60, Dichlormethan / Methanol 9:1, Rf = 0.42
ESI-MS: M⁺¹ = 451
[α]_{D} = +9.6° (c = 1 in Methanol)
F. 99-102°.

### 2.4 2-(S)-(2,2-Dimethyl-propoxycarbonylamino)-3-{3-oxo-2-(R)-[2-(1H-imidazol-2-ylcarbamoyl)-ethyl]-3,4-dihydro-2H-benzo[1,4]oxazin-6-yl}-propionsäure-ethylester 10

Zu einer Lösung von 10 g 9, 4,4 g 2-Amino-imidazol Sulfat, 9,26 g TBTU und 0,9 g HOBt in 100 ml absolutem DMF gibt man 4,6 g Kaliumcarbonat und rührt unter Stickstoff 16 Stunden bei 70°. Man kühlt auf Raumtemperatur gießt in 750 ml kaltes Wasser, trennt den Niederschlag ab, wäscht mit kaltem Wasser und trocknet. Das Rohprodukt wird mit heißem Acetonitril behandelt und nach Abkühlen abfiltriert. Der Rückstand wird aus Ethanol umkristallisiert und man erhält **10**.
Ausbeute: 8.5 g (74%)
HPLC (Chromolith™ Performance RP-18e, Eluent Gradientensystem: Wasser + 0.1% TFA / Acetonitril + 0.1% TFA 99:1 → 1:99 in 10 min, 3 ml/min) Rₜ = 4.48 min.
Chiral HPLC: (Chiralpak™ AD, 250 mm, Heptan /Ethanol 40:60, 0.8 ml/min) Rₜ = 21.95 min, Diastereomerenreinheit = 99% (de = 98%)
DC: Si-60, Dichlormethan / Methanol 9:1, Rf = 0.35
ESI-MS: M⁺¹ = 516
F. 210°C (Zersetzung)

### 2.5 (S)-2-(2,2-Dimethyl-propoxycarbonylamino)-3-{(R)-2-[2-(1H-imidazol-2-ylcarbamoyl)-ethyl]-3-oxo-3,4-dihydro-2H benzo[1,4]oxazin-6-yl}-propionsäure, Natriumsatz 11

Zu einer Lösung von 7 g **10** in 70 ml Dioxan gibt man 13,6 ml 2N NaOH und rührt 16 Stunden bei Raumtemperatur nach. Man neutralisiert mit verdünnter HCl, entfernt das Dioxan im Vakuum, verdünnt mit Wasser, säuert an und trennt den Niederschlag ab, der mit Wasser gewaschen wird. Nach Trocknen erhält man 11 als freie Säure.
Ausbeute: 5.1 g (77%).

Zur Herstellung des Natriumsalzes wird das Produkt in einem Äquivalent (10,46 ml) 1N NaOH gelöst und anschließend lyophilisiert.
Ausbeute: 5.3 g
HPLC (Chromolith™ Performance RP-18e, Eluent Gradientensystem: Wasser + 0.1% TFA /Acetonitril+0.1% TFA 99:1 → 1:99 in 10 min, 3 ml/min) Rₜ = 3.79 min.
Trennung der Diastereomeren: LiChroSorb™ RP-18, 250/4 mm, Eluent: 0.05 M (NH₄)H₂PO₄ buffer pH 4.0 / Acetonitril 82/18, 0.8 ml/min, Rₜ = 36.7 min
ESI-MS: M⁺¹ = 488
[α]_{D} = +18° (c = 1 in DMSO)
F. 185° (Zersetzung).

### Beispiel 3

Die Herstellung von (S)-2-(2,2-Dimethyl-propoxycarbonylamino)-3-{(S)-2-[2-(1*H*-imidazol-2-ylcarbamoyl)-ethyl]-3-oxo-3,4-dihydro-2*H* benzo[1,4]oxazin-6-yl}-propionsäure (**16**) erfolgt analog nachstehendem Schema über Mitunobu-Reaktion:

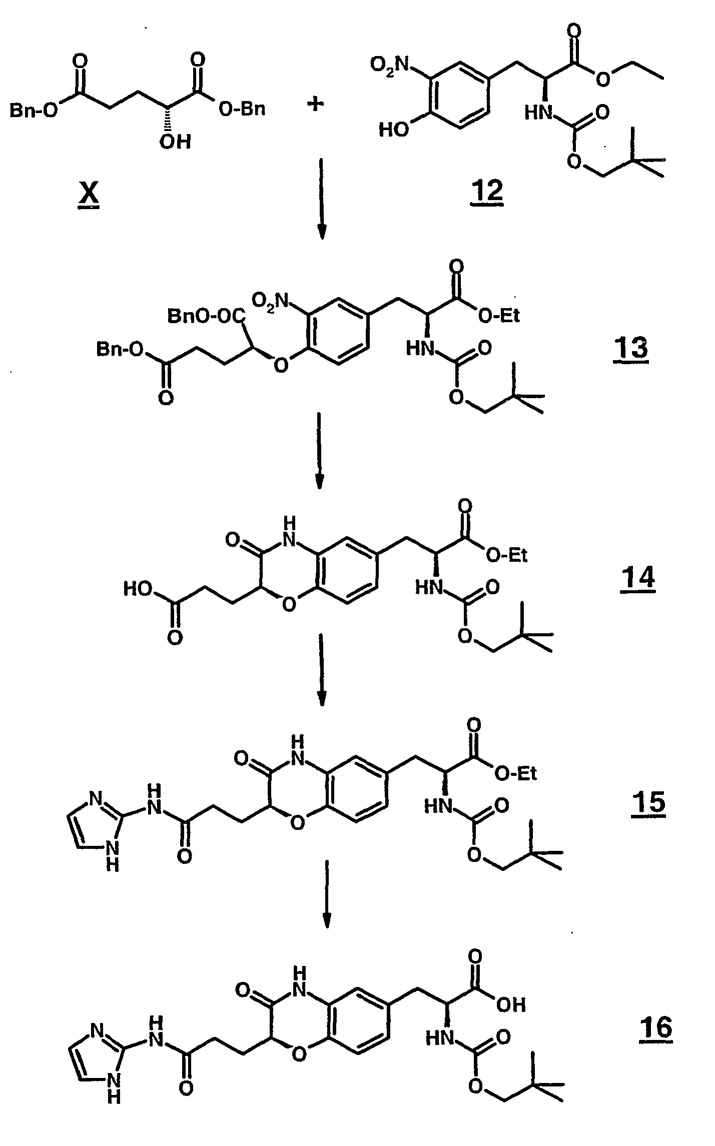

### 3.1 (S)-2-(2,2-Dimethyl-propoxycarbonylamino)-3-(4-hydroxy-3-nitrophenyl)-propionsäure-ethylester 12

Zu einer Lösung von 206,4 g (S)-3-Nitro-tyrosin-ethylester-tosylat in 750 Wasser und 250 ml THF gibt man in kleinen Portionen 81,3 g NaHCO₂. Dann wird langsam eine Lösung von 60 ml Chlorameisensäure-2,2-dimethylpropylester (Neopentylchlorformiat) in 250 ml THF zugetropft und 2 Stunden bei Raumtemperatur nachgerührt. Nach Entfernen des Lösungsmittels wird wie üblich aufgearbeitet und man erhält **12**.
Ausbeute: 107 g (72%)
HPLC (Chromolith™ Performance RP-18e, Eluent Gradientensystem: Wasser + 0.1 % TFA / Acetonitril + 0.1 % TFA 99:1 → 1:99 in 10 min, 3 ml/min) Rₜ = 5.68 min.
DC: Si-60, Toluol / Aceton 9:1, Rf = 0.42
ESI-MS: M⁺¹ = 369
F. 64-67°
[α]_{D} = +0.9° (c = 1 in Methanol).

### 3.2 (R)-2-{4-[(S)-2-(2,2-Dimethyl-propoxycarbonylamino)-2-ethoxycarbonyl-ethyl]-2-nitro-phenoxy}-pentanedicarbonsäure-dibenzyl ester 13

Zu einer Lösung von 4 g **12** und 5,35 g 2-Hydroxy-pentandicarbonsäure-dibenzylester (**X**) in wasserfreiem THF gibt man 5,43 g polymergebundenes DEAD (Diethylazodicarboxylat) in 30 ml THF. Die Mischung wird 16 Stunden geschüttelt. Das Polymer wird abgetrennt und das Lösungsmittel entfernt. Der Rückstand wird in Ethylacetat aufgenommen, es wird wie üblich aufgearbeitet und man erhält **13**, das für die weitere Umsetzung nicht mehr weiter aufgereinigt wird.
Ausbeute: 8.9 g (80% Reinheit), farbloser Sirup;
HPLC (Chromolith™ Performance RP-18e, Eluent Gradientensystem: Wasser + 0.1 % TFA / Acetonitril + 0.1 % TFA 99:1 → 1:99 in 10 min, 3 ml/min) Rₜ = 7.23 min.
Chiral HPLC: (Chiralpak™ AD, 250 mm, Heptan / Isopropanol 60:40, 0.8 ml/min) Rₜ = 19.25 min, Diastereomerenreinheit = 99%
DC: Si-60, Toluol / Aceton 9:1, Rf = 0.38
ESI-MS: M⁺¹ = 679.

### 3.3 (2S)-3-[(2S)-2-(2-Carboxy-ethyl)-3-oxo-3,4-dihydro-2H benzo[1,4]oxazin-6-yl]-2-(2,2-dimethyl-propoxycarbonylamino)-propionsäure-ethylester 14

Durch eine Lösung von 8,4 g **13** (Rohprodukt aus 3.3) in 80 ml Essigsäure leitet man bei 60° in Gegenwart von 2,5 g Palladium (10% auf Aktivkohle) 5 Stunden lang Wasserstoff. Nach Entfernen des Katalysators werden 15 g Zinkpulver zugegeben und eine 1 Stunde bei 60° nachgerührt. Man filtriert über Silicagel, entfernt das Lösungsmittel, nimmt in Ethylacetat auf, arbeitet wie üblich auf und erhält amorphes **14**.
Ausbeute: 3.8 g (85%)
HPLC (Chromolith™ Performance RP-18e, Eluent Gradientensystem: Wasser + 0.1 % TFA / Acetonitril +0.1% TFA 99:1→ 1:99 in 10 min, 3 ml/min) Rₜ = 4.83 min.
DC: Si-60, Dichloromethane / Methanol 9:1, Rf = 0.42
ESI-MS: M⁺¹ = 451

### 3.4 2-(S)-(2,2-Dimethyl-propoxycarbonylamino)-3-{3-oxo-2-(S)-[2-(1 H-imidazol-2-ylcarbamoyl)-ethyl]-3,4-dihydro-2H benzo[1,4]oxazin-6-yl}-propionsäure-ethylester 15

Analog der Herstellung von **5** werden 3,2 g **14** mit 1,88 g 2-Amino-imidazol, 2,96 g TBTU, 0,29 g HOBt und 2,95 g Kaliumcarbonat in 40 ml DMF umgesetzt. Nach üblicher Aufarbeitung erhält man **15**.
Ausbeute: 2.4 g (66%)
HPLC (Chromolith™ Performance RP-18e, Eluent Gradientensystem: Wasser + 0.1% TFA / Acetonitril + 0.1% TFA 99:1 → 1:99 in 10 min, 3 ml/min) Rₜ = 4.40 min.
Chiral HPLC: (Chiralpak™ AD, 250 mm, Heptan / Ethanol 40:60, 0.8 ml/min) Rₜ = 28.0 min, Diastereomerenreinheit = 99%
DC: Si-60, Dichloromethan / Methanol 9:1, Rf = 0.33
ESI-MS: M⁺¹ = 516.

### 3.5 (S)-2-(2,2-Dimethyl-propoxycarbonylamino)-3-{(S)-2-[2-(1 H-imidazol-2-ylcarbamoyl)-ethyl]-3-oxo-3,4-dihydro-2H benzo[1,4]oxazin-6-yl}-propionsäure 16

Die Esterspaltung von 2 g **15** erfolgt analog der Herstellung von **10.**
Ausbeute: 1.7 g (90%)
HPLC (Chromolith™ Performance RP-18e, Eluent Gradientensystem: Wasser + 0.1% TFA / Acetonitril + 0.1% TFA 99:1 → 1:99 in 10 min, 3 ml/min) Rₜ = 3.68 min.
Diastereomerentrennung: LiChroSorb™ RP-18, 250/4 mm, Eluent: 0.05 M (NH₄)H₂PO₄ buffer pH 4.0 / Acetonitril 82/18, 0.8 ml/min, Rₜ = 33.07 min
ESI-MS: M⁺¹ = 488.

### Beispiel 4 (Vergleichsversuch)

Die Herstellung von 2-(S)-(2,2-Dimethylpropoxycarbonylamino)-3-[2-(2-carboxy-ethyl)-3-oxo-3,4-dihydro-2H-benzo[1,4]oxazin-6-yl]-propionsäure-tert.butylester **20** analog WO 98/35949 (Beispiel 11):

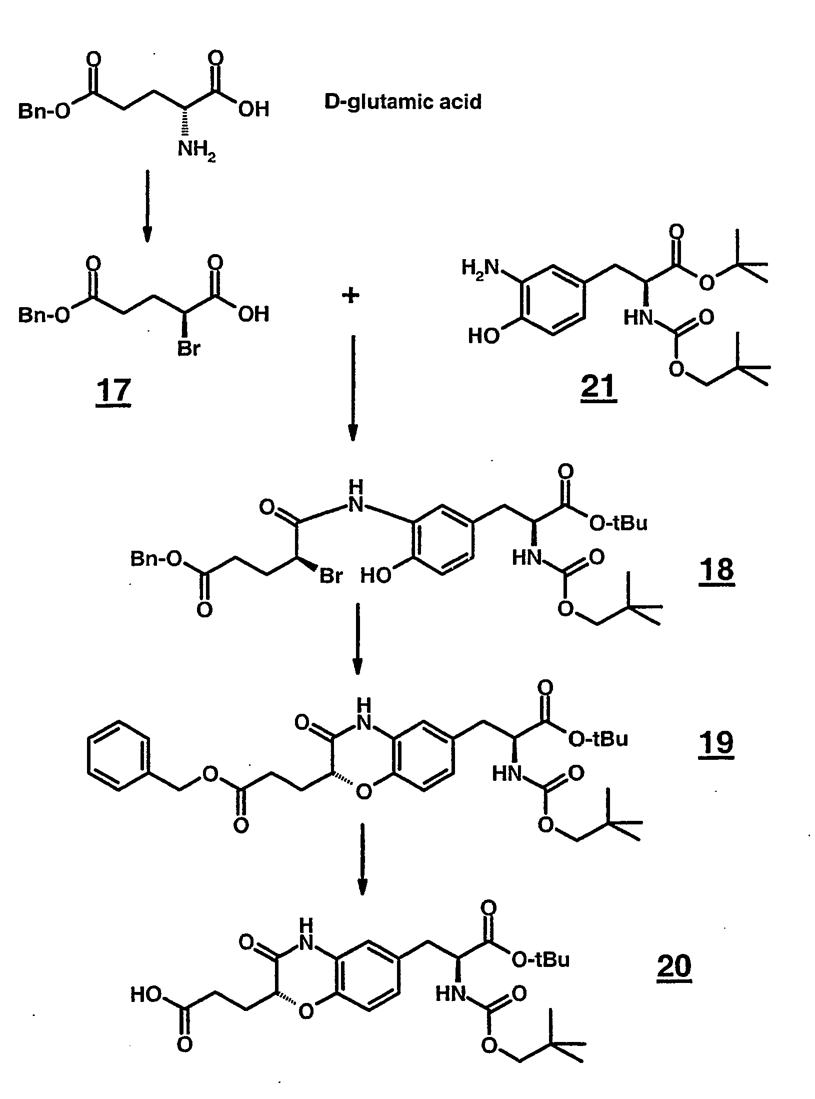

### 4.1 2-Brom-pentandicarbonsäure-5-benzyl ester 17

Zu einer Lösung von 115 g D-Glutaminsäure-5-benzylester und 190 g KBr in 1,4 L 2.5N Schwefelsäure gibt man bei 0-5° portionsweise 50 g Natriumnitrit und rührt 1 Stunde nach, später noch 1 Stunde bei Raumtemperatur. Nach üblicher Aufarbeitung erhält man **17**.
Ausbeute: 101.6 g (70%)
HPLC (Chromolith™ Performance RP-18e, Eluent Gradientensystem: Wasser + 0.1% TFA / Acetonitril + 0.1% TFA 99:1 → 1:99 in 10 min, 3 ml/min) Rₜ = 4.91 min.
ESI-MS: M⁺¹ = 302.

### 4.2 (S)-4-Brom-4-{5-[(S)-2-(2,2-dimethyl-propoxycarbonylamino)-2-tert.butyloxycarbonyl-ethyl]-2-hydroxy-phenylcarbamoyl}-buttersäure-benzylester 18

Zu einer Lösung von 106 g (S)-2-(2,2-Dimethyl-propoxycarbonylamino)-2-(3-amino-4-hydroxyphenyl)-propionsäure-tert.butylester (**12,** erhältlich aus dem entsprechenden 3-Nitroderivat durch reduktion mit H₂/Pd,C) und 101 g **17** in 1 L absolutem THF gibt man portionsweise 66,3 g EDCI Hydrochlorid und rührt 16 Stunden nach. Man entfernt das THF, nimmt in Ethylacetat auf und arbeitet wie üblich auf. Man erhält **18** als gelben Sirup, der ohne weitere Aufreinigung für die weitere Umsetzung verwendet wird.
Ausbeute: 216 g (85% Reinheit, 98%)
HPLC (Chromolith™ Performance RP-18e, Eluent Gradientensystem: Wasser+0.1%TFA/Acetonitri)+0.1%TFA 99:1 → 1:99 in 10 min, 3 ml/min) Rₜ = 7.07 min.
DC: Si-60, Toluol / Aceton 4:1, Rf = 0.41
ESI-MS: M⁺¹ = 650.

### 4.3 (S)-3-[2-(2-Benzyloxycarbonyl-ethyl)-3-oxo-3,4-dihydro-2H-benzo[1,4]oxazin-6-yl]-2-(2,2-dimethyl-propoxycarbonylamino)-propionsäure-tert.-butylester 19

Zu einer Lösung von 215 g **18** in 1250 ml Toluol gibt man 43,14 DBU und rührt eine Stunde bei Raumtemperatur nach. Das Lösungsmittel wird entfernt und der Rückstand durch Flashchromatographie über Silicagel gereinigt (Eluent Toluol/Aceton Gradient 20:1 → 4:1) gereinigt.
Chirale HPLC zeigt ein Diastereomerenverhältnis von 2:1 der R/S und S/S konfigurierten Benzoxazinon-Derivate.
Ausbeute: 69 g (43%)
HPLC (Chromolith™ Performance RP-18e, Eluent Gradientensystem: Wasser + 0.1% TFA / Acetonitril + 0.1% TFA 99:1 → 1:99 in 10 min, 3 ml/min) Rₜ = 7.01 min.
Chirale HPLC: (Chiralpak™ AD, 250 mm, Ethanol, 0.8 ml/min) Rₜ = 12.88 min, Diastereomerenreinheit = 61 % (de = 22%)
DC: Si-60, Toluol / Aceton 4:1, Rf = 0.27
ESI-MS: M⁺¹ = 569.

### 4.4 2-(S)-(2,2-Dimethylpropoxycarbonyl-amino)-3-[2-(2-carboxy-ethyl)-3-oxo-3,4-dihydro-2H-benzo[1,4]oxazin-6-yl]-propionsäure-tert-butylester 20

Durch eine Lösung von 26 g 13 in 250 ml THF leitet man bei Raumtemperatur in Gegenwart von 6,5 g Palladium (10% auf Aktivkohle) 6 Stunden lang Wasserstoff. Der Katalysator wird abgetrennt, das Lösungsmittel entfernt und der Rückstand im Vakuum getrocknet.
Ausbeute: 21.5 g (98%) amorpher Schaum
HPLC (Chromolith™. Performance RP-18e, Eluent Gradientensystem: Wasser + 0.1% TFA / Acetonitril + 0.1% TFA 99:1 → 1:99 in 10 min, 3 ml/min) Rₜ = 5.63 min.
DC: Si-60, Dichlormethan / Methanol 9:1, Rf = 0.44
ESI-MS: M⁺¹ = 479.

### Beispiel 5

Die Aminderivatisierung des Benzoxazinons erfolgt analog nachstehendem Schema:

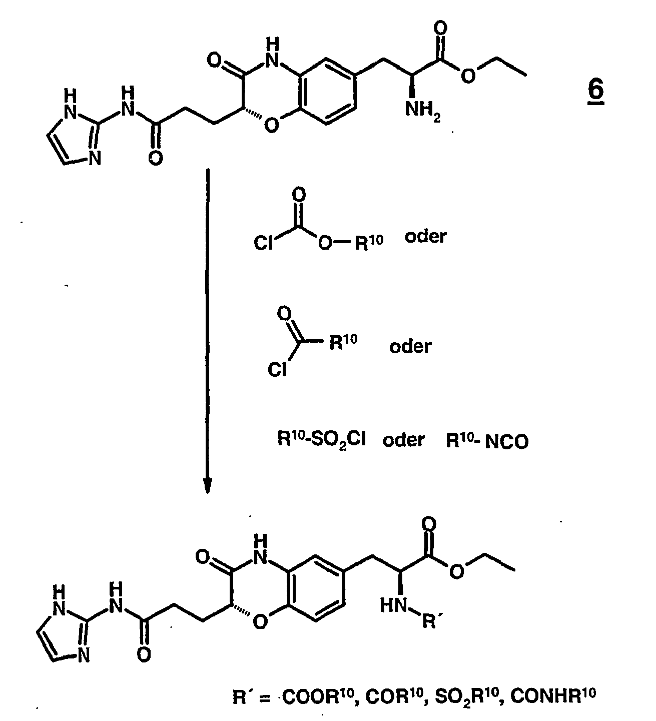

R¹⁰ hat die Bedeutungen wie in Anspruch 1 angegeben.

### 5.1 Sulfonylierung:

Eine Lösung des Amins **6** in absolutem DMF wird bei Raumtemperatur mit 1,1 Äquivalenten eines Alkyl- oder Arylsulfonylchlorids und 3 Äquivalenten Triethylamin umgesetzt. Nach der Reaktion wird wie üblich aufgearbeitet.

So erhält man durch Umsetzung von
2-(S)-Amino-3-{3-oxo-2-(R)-[2-(1*H*-imidazol-2-ylcarbamoyl)-ethyl]-3,4-dihydro-2*H*-benzo[1,4]oxazin-6-yl}-propionsäure-ethylester
   mit
Butylsulfonylchlorid,
4-Tolylsulfonylchlorid,
Benzylsulfonylchlorid,
(R oder S)-Campher-10-sulfonylchlorid
   die nachstehenden Verbindungen
2-(S)-Butylsulfonamido-3-{3-oxo-2-(R)-[2-(1*H*-imidazol-2-ylcarbamoyl)-ethyl]-3,4-dihydro-2*H*-benzo[1,4]oxazin-6-yl}-propionsäure-ethylester,
2-(S)-(4-Tolylsulfonamido)-3-{3-oxo-2-(R)-[2-(1*H*-imidazol-2-ylcarbamoyl)-ethyl]-3,4-dihydro-2*H*-benzo[1,4]oxazin-6-yl}-propionsäure-ethylester,
2-(S)-Benzylsulfonamido-3-{3-oxo-2-(R)-[2-(1*H*-imidazol-2-ylcarbamoyl)-ethyl]-3,4-dihydro-2*H*-benzo[1,4]oxazin-6-yl}-propionsäure-ethylester,
2-(S)-[(R)-Campher-10-yl-sulfonamido]-3-{3-oxo-2-(R)-[2-(1*H-*imidazol-2-ylcarbamoyl)-ethyl]-3,4-dihydro-2*H*-benzo[1,4]oxazin-6-yl}-propionsäure-ethylester,
2-(S)-[(S)-Campher-10-yl-sulfonamido]-3-{3-oxo-2-(R)-[2-(1*H-*imidazol-2-ylcarbamoyl)-ethyl]-3,4-dihydro-2*H*-benzo[1,4]oxazin-6-yl}-propionsäure-ethylester.

### 5.2 Carbamat-Bildung

Eine Lösung des Amins **5** in absolutem DMF wird bei Raumtemperatur mit 1,1 Äquivalenten eines Chlorameisensäure-alkyl- oder -arylesters oder mit einem Alkyl- oder Aryldicarbonat und 3 Äquivalenten Triethylamin umgesetzt. Nach der Reaktion wird wie üblich aufgearbeitet.

So erhält man durch Umsetzung von
2-(S)-Amino-3-{3-oxo-2-(R)-[2-(1*H*-imidazol-2-ylcarbamoyl)-ethyl]-3,4-dihydro-2*H*-benzo[1,4]oxazin-6-yl}-propionsäure-ethylester
   mit
Chlorameisensäureisopropylester,
Chlorameisensäurebenzylester
   die nachstehenden Verbindungen
2-(S)-Isobutoxycarboxamido-3-{3-oxo-2-(R)-[2-(1*H*-imidazol-2-ylcarbamoyl)-ethyl]-3,4-dihydro-2*H* benzo[1,4]oxazin-6-yl}-propionsäure-ethylester,
2-(S)-Benzyloxycarboxamido-3-{3-oxo-2-(R)-[2-(1*H*-imidazol-2-ylcarbamoyl)-ethyl]-3,4-dihydro-2*H*-benzo[1,4]oxazin-6-yl}-propionsäure-ethylester.

### 5.3 Acylierung

Eine Lösung des Amins **5** in absolutem DMF wird bei Raumtemperatur mit 1,1 Äquivalenten eines Alkyl- oder Arylcarbonylchlorids und 3 Äquivalenten Triethylamin umgesetzt. Nach der Reaktion wird wie üblich aufgearbeitet.

Alternativ sind die verschiedensten Varianten einsetzbar, wie z.B. mit EDCl, aktivierte Ester, HOBt/TBTU.

### 5.4 Harnstoffbildung

Eine Lösung des Amins **5** in absolutem DMF wird bei Raumtemperatur mit 1,1 Äquivalenten eines Alkyl- oder Arylisocyanats und 3 Äquivalenten Triethylamin umgesetzt. Nach der Reaktion wird wie üblich aufgearbeitet.

## Patentansprüche

1. Verfahren zur Herstellung von Verbindungen der Formel I 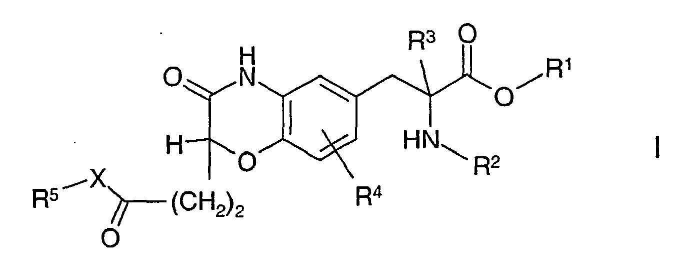 worin
R¹ H, Alkyl mit 1, 2, 3, 4, 5 oder 6 C-Atomen oder Benzyl,
R² R¹⁰, CO-R¹⁰, COOR⁶, COOR¹⁰, CONHR¹⁰, SO₂R⁶ oder SO₂R¹⁰,
R³ H oder Alkyl mit 1-6 C-Atomen,
R⁴ H, Hal, OA, NHR¹⁰, N(R¹⁰)₂, -NH-Acyl, -O-Acyl, CN, NO₂, OR¹⁰, SR¹⁰, R² oder CONHR¹⁰,
R⁵ NH₂, H₂N-C(=NH) oder H₂N-(C=NH)-NH, wobei die primären Aminogruppen auch mit konventionellen Aminoschutzgruppen versehen sein können, oder ein-, zwei- oder dreifach durch R¹⁰, CO-R¹⁰, COOR¹⁰ oder SO₂R¹⁰ substituiert sein können, oder R⁶,
X fehlt oder NH,
R⁶ einen ein- oder zweikernigen gesättigten, ungesättigten oder aromatischen Heterocyclus mit 1 bis 4 N-, O- und / oder S-Atomen, der unsubstituiert oder ein-, zwei- oder dreifach durch Hal, A, -CO-A, OA, CN, COOH, COOA, CONHA, NO₂, =NH oder =O substituiert sein kann,
R⁹ H, Hal, OA, NHA, NAA', NHAcyl, OAcyl, CN, NO₂, SA, SOA, SO₂A, SO₂Ph oder SO₃H,
R¹⁰ H, A, Ar oder Aralkylen mit 7-14 C-Atomen,
A, A' jeweils unabhängig voneinander H oder unsubstituiertes oder ein-, zwei- oder dreifach durch R⁹ substituiertes Alkyl oder Cycloalkyl mit 1-15 C-Atomen und worin eine, zwei- oder drei Methylengruppen durch N, O und/oder S ersetzt sein können,
Ar unsubstituiertes oder ein-, zwei- oder dreifach durch A und/oder R⁹ substituiertes ein- oder zweikerniges aromatisches Ringsystem mit 0, 1, 2, 3 oder 4 N-, O- und/oder S-Atomen,
Ph unsubstituiertes oder ein-, zwei- oder dreifach durch A substituiertes Phenyl,
Hal F, Cl, Br oder I und
bedeuten,
sowie deren Salze, Solvate und Stereoisomere,
wobei
a) 5-Oxo-tetrahydrofuran-2-carbonsäure mit einer Verbindung der Formel II
R⁷-L II
worin
R⁷ Benzyl oder Alkyl mit 1, 2, 3, 4, 5 oder 6 C-Atomen und
L Cl, Br, I oder eine freie oder reaktionsfähig funktionell abgewandelte OH-Gruppe
bedeuten,
zu einer Verbindung der Formel III 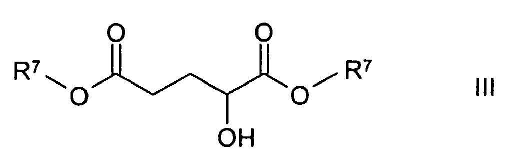 worin
R⁷ Benzyl oder Alkyl mit 1, 2, 3, 4, 5 oder 6 C-Atomen bedeutet,
umgesetzt wird,
b) dann eine Verbindung der Formel III mit einer Verbindung der Formel IV
R⁸-L IV
worin
R⁸ Alkylsulfonyl mit 1-6 C-Atomen oder Arylsulfonyl mit 6-10 C-Atomen und
L Cl, Br, I oder eine freie oder reaktionsfähig funktionell abgewandelte OH-Gruppe
bedeuten,
zu einer Verbindung der Formel V 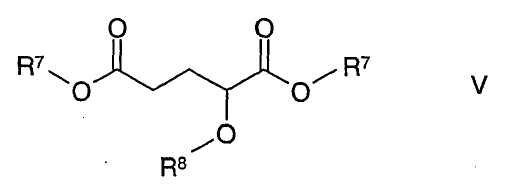 worin
R⁷ Benzyl oder Alkyl mit 1, 2, 3, 4, 5 oder 6 C-Atomen und
R⁸ Alkylsulfonyl mit 1-6 C-Atomen oder Arylsulfonyl mit 6-10 C-Atomen
bedeuten,
umgesetzt wird,
c) dann eine Verbindung der Formel III oder V mit einer Verbindung der Formel VI 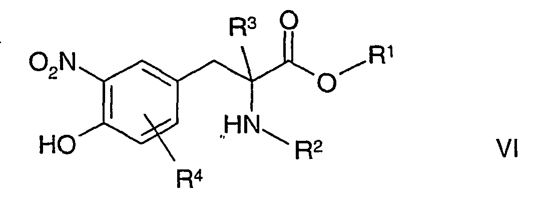 worin
R¹ H, Alkyl mit 1, 2, 3, 4, 5 oder 6 C-Atomen oder Benzyl,
R² R¹⁰, CO-R¹⁰, COOR⁶, COOR¹⁰, CONHR¹⁰, SO₂R⁶ oder SO₂R¹⁰,
R³ H oder Alkyl mit 1-6 C-Atomen,
R⁴ H, Hal, OA, NHR¹⁰, N(R¹⁰)₂, -NH-Acyl, -O-Acyl, CN, NO₂, OR¹⁰, SR¹⁰, R² oder CONHR¹⁰,
R⁶ einen ein- oder zweikernigen gesättigten, ungesättigten oder aromatischen Heterocyclus mit 1 bis 4 N-, 0- und / oder S-Atomen, der unsubstituiert oder ein-, zwei- oder dreifach durch Hal, A, -CO-A, OA, CN, COOH, COOA, CONHA, NO₂, =NH oder =O substituiert sein kann,
R⁹ H, Hal, OA, NHA, NAA', NHAcyl, OAcyl, CN, NO₂, SA, SOA, SO₂A, SO₂Ph oder SO₃H,
R¹⁰ H, A, Ar oder Aralkylen mit 7-14 C-Atomen,
A, A' jeweils unabhängig voneinander H oder unsubstituiertes oder ein-, zwei- oder dreifach durch R⁹ substituiertes Alkyl oder Cycloalkyl mit 1-15 C-Atomen und worin eine, zwei- oder drei Methylengruppen durch N, O und/oder S ersetzt sein können,
Ar unsubstituiertes oder ein-, zwei- oder dreifach durch A und/oder R⁹ substituiertes ein- oder zweikerniges aromatisches Ringsystem mit 0,1, 2, 3 oder 4 N-, O-und/oder S-Atomen,
Ph unsubstituiertes oder ein-, zwei- oder dreifach durch A substituiertes Phenyl,
Hal F, Cl, Br oder I
bedeuten,
zu einer Verbindung der Formel VII 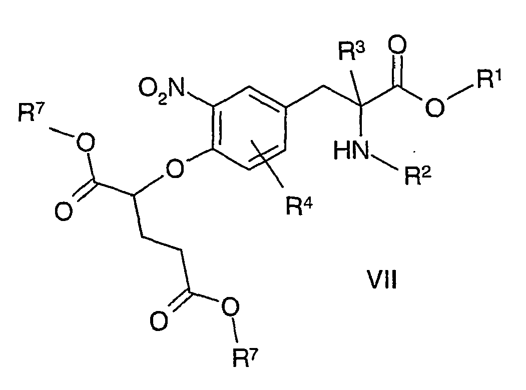 worin
R¹ H, Alkyl mit 1, 2, 3, 4, 5 oder 6 C-Atomen oder Benzyl,
R² R¹⁰, CO-R¹⁰, COOR⁶, COOR¹⁰, CONHR¹⁰, SO₂R⁶ oder SO₂R¹⁰,
R³ H oder Alkyl mit 1-6 C-Atomen,
R⁴ H, Hal, OA, NHR¹⁰, N(R¹⁰)₂, -NH-Acyl, -O-Acyl, CN, NO₂, OR¹⁰, SR¹⁰, R² oder CONHR¹⁰,
R⁶ einen ein- oder zweikernigen gesättigten, ungesättigten oder aromatischen Heterocyclus mit 1 bis 4 N-, O- und / oder S-Atomen, der unsubstituiert oder ein-, zwei- oder dreifach durch Hal, A, -CO-A, OA, CN, COOH, COOA, CONHA, NO₂, =NH oder =O substituiert sein kann,
R⁷ Benzyl oder Alkyl mit 1, 2, 3, 4, 5 oder 6 C-Atomen,
R⁹ H, Hal, OA, NHA, NAA', NHAcyl, OAcyl, CN, NO₂, SA, SOA, SO₂A, SO₂Ph oder SO₃H,
R¹⁰ H, A, Ar oder Aralkylen mit 7-14 C-Atomen,
A, A' jeweils unabhängig voneinander H oder unsubstituiertes oder ein-, zwei- oder dreifach durch R⁹ substituiertes Alkyl oder Cycloalkyl mit 1-15 C-Atomen und worin eine, zwei- oder drei Methylengruppen durch N, O und/oder S ersetzt sein können,
Ar unsubstituiertes oder ein-, zwei- oder dreifach durch A und/oder R⁹ substituiertes ein- oder zweikerniges aromatisches Ringsystem mit 0, 1, 2, 3 oder 4 N-, O-und/oder S-Atomen,
Ph unsubstituiertes oder ein-, zwei- oder dreifach durch A substituiertes Phenyl,
Hal F, Cl, Br oder I
bedeuten,
umgesetzt wird,
d) dann eine Verbindung der Formel VII zu einer Verbindung der Formel VIII 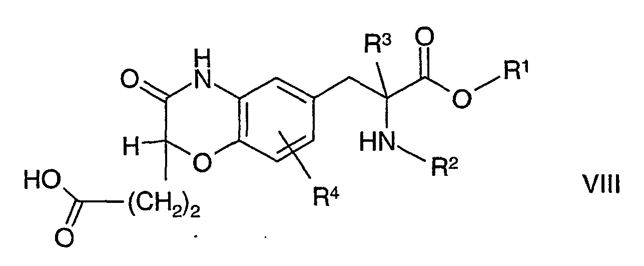 worin
R¹ H, Alkyl mit 1, 2, 3, 4, 5 oder 6 C-Atomen oder Benzyl,
R² R¹⁰, CO-R¹⁰, COOR⁶, COOR¹⁰, CONHR¹⁰, SO₂R⁶ oder SO₂R¹⁰,
R³ H oder Alkyl mit 1-6 C-Atomen,
R⁴ H, Hal, OA, NHR¹⁰, N(R¹⁰)₂, -NH-Acyl, -O-Acyl, CN, NO₂, OR¹⁰, SR¹⁰, R² oder CONHR¹⁰,
R⁶ einen ein- oder zweikernigen gesättigten, ungesättigten oder aromatischen Heterocyclus mit 1 bis 4 N-, O- und / oder S-Atomen, der unsubstituiert oder ein-, zwei- oder dreifach durch Hal, A, -CO-A, OA, CN, COOH, COOA, CONHA, NO₂, =NH oder =O substituiert sein kann,
R⁹ H, Hal, OA, NHA, NAA', NHAcyl, OAcyl, CN, NO₂, SA, SOA, SO₂A, SO₂Ph oder SO₃H,
R¹⁰ H, A, Ar oder Aralkylen mit 7-14 C-Atomen,
A, A' jeweils unabhängig voneinander H oder unsubstituiertes oder ein-, zwei- oder dreifach durch R⁹ substituiertes Alkyl oder Cycloalkyl mit 1-15 C-Atomen und worin eine, zwei- oder drei Methylengruppen durch N, O und/oder S ersetzt sein können,
Ar unsubstituiertes oder ein-, zwei- oder dreifach durch A und/oder R⁹ substituiertes ein- oder zweikerniges aromatisches Ringsystem mit 0,1, 2, 3 oder 4 N-, O-und/oder S-Atomen,
Ph unsubstituiertes oder ein-, zwei- oder dreifach durch A substituiertes Phenyl,
Hal F, Cl, Br oder I bedeuten,
umgesetzt wird,
e) anschließend gegebenenfalls eine Verbindung der Formel VIII in eine Verbindung der Formel VIIIa 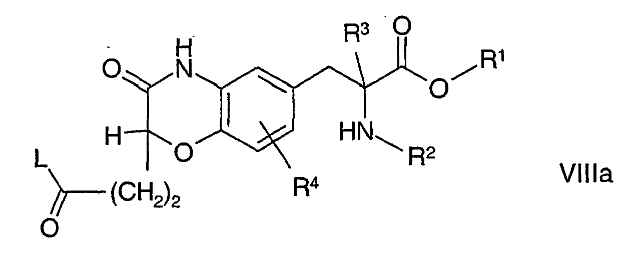 worin
R¹, R², R³ und R⁴ die unter d) angegebenen Bedeutungen haben und
L Cl, Br, I oder eine reaktionsfähig funktionell abgewandelte OH-Gruppe bedeutet,
umwandelt,
e) dann eine Verbindung der Formel VIII oder eine Verbindung der Formel Villa
e) i) mit einer Verbindung der Formel IX
R⁵-H IX
worin
R⁵ NH₂, H₂N-C(=NH) oder H₂N-(C=NH)-NH, wobei die primären Aminogruppen auch mit konventionellen Aminoschutzgruppen versehen sein können, oder ein-, zwei- oder dreifach durch R¹⁰, CO-R¹⁰, COOR¹⁰ oder SO₂R¹⁰ substituiert sein können,
worin R¹⁰ die bei Formel I angegebenen Bedeutungen hat,
bedeutet,
oder
e) ii) mit einer Verbindung der Formel X
R⁶-NH₂ X
worin
R⁶ einen ein- oder zweikernigen gesättigten, ungesättigten oder aromatischen Heterocyclus mit 1 bis 4 N-, O- und / oder S-Atomen, der unsubstituiert oder ein-, zwei- oder dreifach durch Hal, A, -CO-A, OA, CN, COOH, COOA, CONHA, NO₂, =NH oder =O substituiert sein kann,
bedeutet,
zu einer Verbindung der Formel I umsetzt,
gegebenenfalls anschließend einen Rest R² in einen anderen Rest R² umwandelt,
indem man eine Verbindung der Formel I, worin R² H bedeutet mit einer Verbindung ausgewählt aus der Gruppe
L-R¹⁰, L-CO-R¹⁰, L-COOR⁶, L-COOR¹⁰, R¹⁰'-N=C=O, R⁶-SO₂L, R¹⁰-SO₂L,
worin
R¹⁰ A, Ar oder Aralkylen mit 7-14 C-Atomen und
L Cl, Br, I oder eine freie oder reaktionsfähig funktionell abgewandelte OH-Gruppe bedeutet,
umsetzt,
und/ oder eine Base oder Säure der Formel I in eines ihrer Salze umwandelt.

2. Verfahren nach Anspruch 1, worin
R³ H,
R⁴ H bedeuten.

3. Verfahren nach Anspruch 1 oder 2, worin
R⁶ 1 *H*-Imidazol-2-yl, Thiazol-2-yl, 1 *H*-Benzimidazol-2-yl, 2*H-*Pyrazol-2-yl, 1 *H*-Tetrazol-5-yl, 2-Imino-imidazolidin-4-on-5-yl, 1-A-1,5-dihydro-imidazol-4-on-2-yl, Pyrimidin-2-yl oder 1,4,5,6-Tetrahydro-pyrimidin-2-yl bedeutet.

4. Verfahren nach einem oder mehreren der Ansprüche 1-3, worin
R⁹ H bedeutet.

5. Verfahren nach einem oder mehreren der Ansprüche 1-4, worin
R¹⁰ H, Alkyl mit 1, 2, 3, 4, 5 oder 6 C-Atomen, Campher-10-yl oder Benzyl
bedeutet.

6. Verfahren nach einem oder mehreren der Ansprüche 1-5, worin
A unsubstituiertes Alkyl oder Cycloalkyl mit 1-15 C-Atomen bedeutet.

7. Verfahren nach einem oder mehreren der Ansprüche 1-6, worin
A Alkyl mit 1, 2, 3, 4, 5 oder 6 C-Atomen,
R¹⁰ H, Alkyl mit 1, 2, 3, 4, 5 oder 6 C-Atomen, Campher-10-yl oder Benzyl
bedeuten.

8. Verfahren nach Anspruch 1, worin
R¹ H, Alkyl mit 1, 2, 3, 4, 5 oder 6 C-Atomen oder Benzyl,
R² R¹⁰, CO-R¹⁰, COOR¹⁰ oder SO₂R¹⁰,
R³ H,
R⁴ H,
R⁵ R⁶,
R⁶ einen ein- oder zweikernigen gesättigten, ungesättigten oder aromatischen Heterocyclus mit 1 bis 4 N-, O- und / oder S-Atomen, der unsubstituiert oder ein-, zwei- oder dreifach durch Hal, A, -CO-A, OA, CN, COOH, COOA, CONHA, NO₂, =NH oder =O substituiert sein kann,
X NH,
R¹⁰ H, Alkyl mit 1, 2, 3, 4, 5 oder 6 C-Atomen, Campher-10-yl oder Benzyl,
A Alkyl mit 1, 2, 3, 4, 5 oder 6 C-Atomen,
Hal F, Cl, Br oder I
bedeuten.

9. Verfahren nach Anspruch 8, worin
R⁶ 1*H*-Imidazol-2-yl, Thiazol-2-yl, 1*H*-Benzimidazol-2-yl, 2H-Pyrazol-2-yl, 1 *H*-Tetrazol-5-yl, 2-Imino-imidazolidin-4-on-5-yl, 1-A-1,5-dihydro-imidazol-4-on-2-yl, Pyrimidin-2-yl oder 1,4,5,6-Tetrahydro-pyrimidin-2-yl bedeutet.

10. Verfahren nach Anspruch 1 zur Herstellung von Verbindungen der Formel la 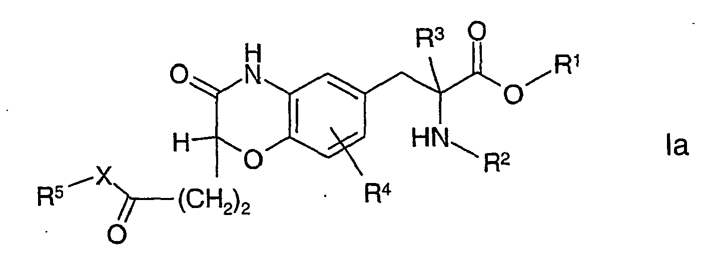 worin
R¹ H oder Alkyl mit 1, 2, 3 oder 4 C-Atomen,
R² R¹⁰, CO-R¹⁰, COOR¹⁰ oder SO₂R¹⁰,
R³ H,
R⁴ H,
R⁵ R⁶,
X NH,
R⁶ 1*H*-Imidazol-2-yl, Thiazol-2-yl, 1*H*-Benzimidazol-2-yl, 2H-Pyrazol-2-yl, 1*H*-Tetrazol-5-yl, 2-Imino-imidazolidin-4-on-5-yl, 1-A-1,5-dihydro-imidazol-4-on-2-yl, Pyrimidin-2-yl oder 1,4,5,6-Tetrahydro-pyrimidin-2-yl,
R¹⁰ H oder Alkyl mit 1,2,3,4,5 oder 6 C-Atomen und
A Alkyl mit 1, 2, 3, 4, 5 oder 6 C-Atomen,
bedeuten,
sowie deren Salze, Solvate und Stereoisomere,
wobei
a) 5-Oxo-tetrahydrofuran-2-carbonsäure mit einer Verbindung der Formel II
R⁷ -L II
worin
R⁷ Benzyl oder Alkyl mit 1, 2, 3, 4, 5 oder 6 C-Atomen und
L Cl, Br, I oder eine freie oder reaktionsfähig funktionell abgewandelte OH-Gruppe
bedeuten,
zu einer Verbindung der Formel III 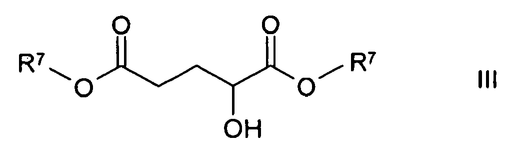 worin
R⁷ Benzyl oder Alkyl mit 1, 2, 3, 4, 5 oder 6 C-Atomen bedeutet,
umgesetzt wird,
b) dann eine Verbindung der Formel III mit einer Verbindung der Formel IV
R⁸-L IV
worin
R⁸ Alkylsulfonyl mit 1-6 C-Atomen oder Arylsulfonyl mit 6-10 C-Atomen und
L Cl, Br, I oder eine freie oder reaktionsfähig funktionell abgewandelte OH-Gruppe
bedeuten,
zu einer Verbindung der Formel V 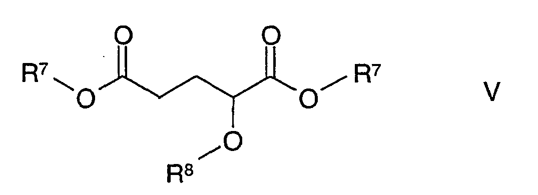 worin
R⁷ Benzyl oder Alkyl mit 1, 2, 3, 4, 5 oder 6 C-Atomen und
R⁸ Alkylsulfonyl mit 1-6 C-Atomen oder Arylsulfonyl mit 6-10 C-Atomen bedeuten,
umgesetzt wird,
c) dann eine Verbindung der Formel III oder V mit einer Verbindung der Formel VI 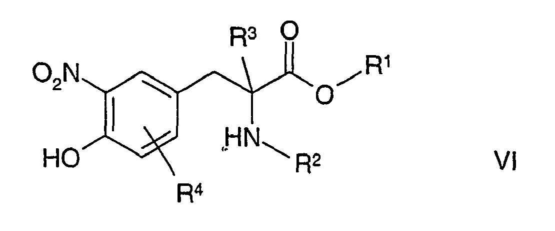 worin
R¹ H oder Alkyl mit 1, 2, 3 oder 4 C-Atomen,
R² R¹⁰, CO-R¹⁰, COOR¹⁰ oder SO₂R¹⁰,
R³ H,
R⁴ H,
R¹⁰ H oder Alkyl mit 1, 2, 3, 4, 5 oder 6 C-Atomen und
bedeuten,
zu einer Verbindung der Formel VII 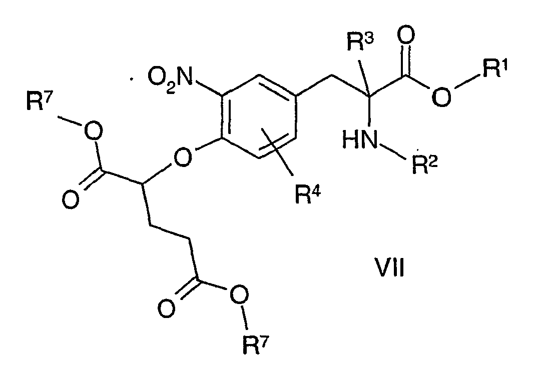 worin
R¹ H oder Alkyl mit 1, 2, 3 oder 4 C-Atomen,
R² R¹⁰, CO-R¹⁰, COOR¹⁰ oder SO₂R¹⁰,
R³ H,
R⁴ H,
R⁷ Benzyl oder Alkyl mit 1, 2, 3, 4, 5 oder 6 C-Atomen,
R¹⁰ H oder Alkyl mit 1, 2, 3, 4, 5 oder 6 C-Atomen
bedeuten,
umgesetzt wird,
d) dann eine Verbindung der Formel VII zu einer Verbindung der Formel VIII 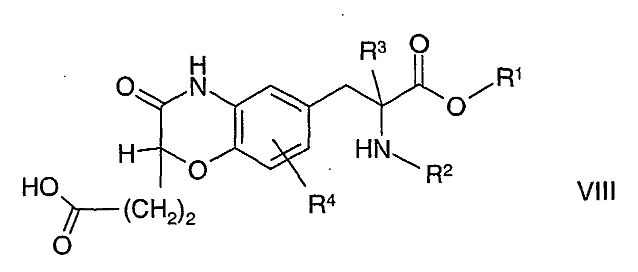 worin
R¹ H oder Alkyl mit 1, 2, 3 oder 4 C-Atomen,
R² R¹⁰, CO-R¹⁰, COOR¹⁰ oder SO₂R¹⁰,
R³ H,
R⁴ H,
R¹⁰ H oder Alkyl mit 1, 2, 3, 4, 5 oder 6 C-Atomen bedeuten,
umgesetzt wird,
e) dann eine Verbindung der Formel VIII
mit einer Verbindung der Formel X
R⁶-NH₂ X
worin
R⁶ 1 *H*-Imidazol-2-yl, Thiazol-2-yl, 1*H*-Benzimidazol-2-yl, 2*H-*Pyrazol-2-yl, 1*H*-Tetrazol-5-yl, 2-Imino-imidazolidin-4-on-5-yl, 1-A-1,5-dihydro-imidazol-4-on-2-yl, Pyrimidin-2-yl oder 1,4,5,6-Tetrahydro-pyrimidin-2-yl,
bedeutet,
zu einer Verbindung der Formel la umsetzt,
gegebenenfalls anschließend einen Rest R² in einen anderen Rest R² umwandelt,
indem man eine Verbindung der Formel Ia, worin R² H bedeutet mit einer Verbindung ausgewählt aus der Gruppe
L-R^{10'}, L-CO-R¹⁰', L-COOR^{10'}, R^{10'}-SO₂L,
worin
R^{10'} Alkyl mit 1, 2, 3, 4, 5 oder 6 C-Atomen und
L Cl, Br, I oder eine freie oder reaktionsfähig funktionell abgewandelte OH-Gruppe bedeutet,
umsetzt,
und / oder eine Base oder Säure der Formel la in eines ihrer Salze umwandelt.

11. Verfahren nach einem oder mehreren der Ansprüche 1-10, worin
R⁶ 1*H*-Imidazol-2-yl bedeutet.

12. Verfahren nach einem oder mehreren der Ansprüche 1-11, worin in Schritt b) in den Verbindungen der Formel IV und V
R⁸ Methylsulfonyl, Trifluormethylsulfonyl, Phenylsulfonyl oder p-Tolylsulfonyl
bedeutet.

13. Verfahren nach einem oder mehreren der Ansprüche 1-12, wobei die Umsetzung einer Verbindung der Formel VII zu einer Verbindung der Formel VIII
a) durch Reduktion der Nitrogruppe, anschließender Esterspaltung und Cyclisierung oder
b) durch Esterspaltung, anschließender Reduktion der Nitrogruppe und Cyclisierung
erfolgt.

14. Verfahren nach Anspruch 1 zur Herstellung von (S)-2-(2,2-Dimethylpropoxycarbonylamino)-3-{(S)-2-[2-(1*H*-imidazol-2-ylcarbamoyl)-ethyl]-3-oxo-3,4-dihydro-2*H*-benzo[1,4]oxazin-6-yl}-propionsäure,
wobei
a) (S)-3-Nitro-tyrosin-ethylester-tosylat mit Chlorameisensäure-2,2-dimethylpropylester zu (S)-2-(2,2-Dimethyl-propoxycarbonylamino)-3-(4-hydroxy-3-nitro-phenyl)-propionsäure-ethylester **12** umgesetzt wird,
b) **12** mit 2-Hydroxy-pentandicarbonsäure-dibenzylester zu (R)-2-{4-[(S)-2-(2,2-Dimethyl-propoxycarbonylamino)-2-ethoxycarbonyl-ethyl]-2-nitro-phenoxy}-pentanedicarbonsäuredibenzylester **13** umgesetzt wird,
c) **13** zu (2S)-3-[(2S)-2-(2-Carboxy-ethyl)-3-oxo-3,4-dihydro-2*H-*benzo[1,4]oxazin-6-yl]-2-(2,2-dimethyl-propoxycarbonylamino)-propionsäure-ethylester 14 cyclisiert wird,
d) **14** mit 2-Amino-imidazol zu 2-(S)-(2,2-Dimethylpropoxycarbonylamino)-3-{3-oxo-2 -(S)-[2-(1*H*-imidazol-2-ylcarbamoyl)-ethyl]-3,4-dihydro-2*H*-benzo[1,4]oxazin-6-yl}-propionsäure-ethylester **15** umgesetzt wird und
e) **15** durch Esterspaltung zu (S)-2-(2,2-Dimethylpropoxycarbonylamino)-3-{(S)-2-[2-(1*H*-imidazol-2-ylcarbamoyl)-ethyl]-3-oxo-3,4-dihydro-2*H*-benzo[1,4]oxazin-6-yl}-propionsäure **16** umgesetzt wird.

15. Zwischenverbindungen der Formel VII 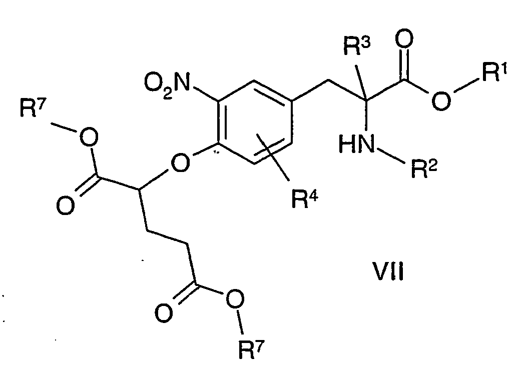 worin
R¹ H oder Alkyl mit 1, 2, 3 oder 4 C-Atomen,
R² R¹⁰, CO-R¹⁰, COOR¹⁰ oder SO₂R¹⁰,
R³ H,
R⁴ H,
R⁷ Benzyl oder Alkyl mit 1, 2, 3, 4, 5 oder 6 C-Atomen,
R⁹ H, Hal, OA, NHA, NAA', NHAcyl, OAcyl, CN, NO₂, SA, SOA, SO₂A, SO₂Ph oder SO₃H,
R¹⁰ H, A, Ar oder Aralkylen mit 7-14 C-Atomen,
A, A' jeweils unabhängig voneinander H oder unsubstituiertes oder ein-, zwei- oder dreifach durch R⁹ substituiertes Alkyl oder Cycloalkyl mit 1-15 C-Atomen und worin eine, zwei- oder drei Methylengruppen durch N, O und/oder S ersetzt sein können,
Ar unsubstituiertes oder ein-, zwei- oder dreifach durch A und/oder R⁹ substituiertes ein- oder zweikerniges aromatisches Ringsystem mit 0, 1, 2, 3 oder 4 N-, O- und/oder S-Atomen,
bedeuten,
sowie deren Salze, Solvate und Stereoisomere.

16. Verfahren zur Herstellung von Verbindungen der Formel VII, sowie deren Salze, Solvate und Stereoisomere,
wobei
a) 5-Oxo-tetrahydrofuran-2-carbonsäure mit einer Verbindung der Formel II
R⁷-L II
worin
R⁷ Benzyl oder Alkyl mit 1, 2, 3, 4, 5 oder 6 C-Atomen und
L Cl, Br, I oder eine freie oder reaktionsfähig funktionell abgewandelte OH-Gruppe
bedeuten,
zu einer Verbindung der Formel III 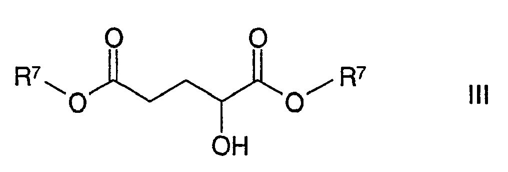 worin
R⁷ Benzyl oder Alkyl mit 1, 2, 3, 4, 5 oder 6 C-Atomen bedeutet,
umgesetzt wird,
b) dann eine Verbindung der Formel III mit einer Verbindung der Formel IV
R⁸-L IV
worin
R⁸ Alkylsulfonyl mit 1-6 C-Atomen oder Arylsulfonyl mit 6-10 C-Atomen und
L Cl, Br, I oder eine freie oder reaktionsfähig funktionell abgewandelte OH-Gruppe
bedeuten,
zu einer Verbindung der Formel V 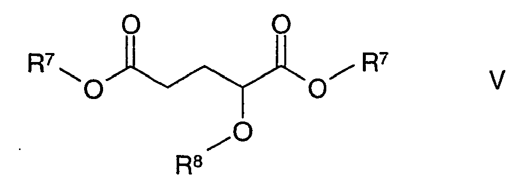 worin
R⁷ Benzyl oder Alkyl mit 1, 2, 3, 4, 5 oder 6 C-Atomen und
R⁸ Alkylsulfonyl mit 1-6 C-Atomen oder Arylsulfonyl mit 6-10 C-Atomen bedeuten,
umgesetzt wird,
c) dann eine Verbindung der Formel III oder V mit einer Verbindung der Formel VI 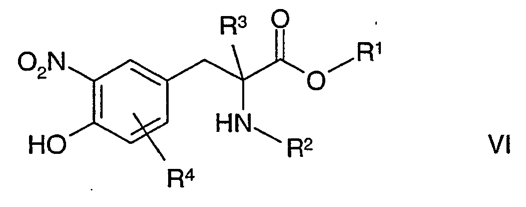
worin
R¹ H oder Alkyl mit 1, 2, 3 oder 4 C-Atomen,
R² R¹⁰, CO-R¹⁰, COOR¹⁰ oder SO₂R¹⁰,
R³ H,
R⁴ H,
R⁹ H, Hal, OA, NHA, NAA', NHAcyl, OAcyl, CN, NO₂, SA, SOA, SO₂A, SO₂Ph oder SO₃H,
R¹⁰ H, A, Ar oder Aralkylen mit 7-14 C-Atomen,
A, A' jeweils unabhängig voneinander H oder unsubstituiertes oder ein-, zwei- oder dreifach durch R⁹ substituiertes Alkyl oder Cycloalkyl mit 1-15 C-Atomen und worin eine, zwei- oder drei Methylengruppen durch N, O und/oder S ersetzt sein können,
Ar unsubstituiertes oder ein-, zwei- oder dreifach durch A und/oder R⁹ substituiertes ein- oder zweikerniges aromatisches Ringsystem mit 0, 1, 2, 3 oder 4 N-, O-und/oder S-Atomen,
bedeuten,
zu einer Verbindung der Formel VII umgesetzt wird,
und / oder eine Säure der Formel VII in eines ihrer Salze umgewandelt wird.

## Claims

1. Process for the preparation of compounds of the formula I 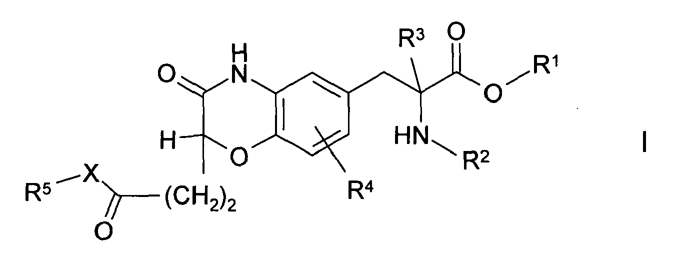 in which
R¹ denotes H, alkyl having 1, 2, 3, 4, 5 or 6 C atoms or benzyl,
R² denotes R¹⁰ , CO-R¹⁰ COOR⁶, COOR¹⁰, CONHR¹⁰, SO₂R⁶ or SO₂R¹⁰,
R³ denotes H or alkyl having 1-6 C atoms,
R⁴ denotes H, Hal, OA, NHR¹⁰, N(R¹⁰)₂, -NH-acyl, -O-acyl, CN, NO₂, OR¹⁰, SR¹⁰, R² or CONHR¹⁰,
R⁵ denotes NH₂, H₂N-C(=NH) or H₂N-(C=NH)-NH, where the primary amino groups may also be provided with conventional amino-protecting groups, or may be mono-, di- or trisubstituted by R¹⁰, CO-R¹⁰, COOR¹⁰ or SO₂R¹⁰, or R⁶,
X is absent or denotes NH,
R⁶ denotes a mono- or bicyclic saturated, unsaturated or aromatic heterocycle having 1 to 4 N, O and/or S atoms, which may be unsubstituted or mono-, di- or trisubstituted by Hal, A, -CO-A, OA, CN, COOH, COOA, CONHA, NO₂, =NH or =O,
R⁹ denotes H, Hal, OA, NHA, NAA', NHacyl, Oacyl, CN, NO₂, SA, SOA, SO₂A, SO₂Ph or SO₃H,
R¹⁰ denotes H, A, Ar or aralkylene having 7-14 C atoms,
A, A' each, independently of one another, denote H or alkyl or cycloalkyl having 1-15 C atoms, each of which is unsubstituted or mono-, di- or trisubstituted by R⁹ and in which one, two or three methylene groups may be replaced by N, O and/or S,
Ar denotes mono- or bicyclic aromatic ring system having 0, 1, 2, 3 or 4 N, O and/or S atoms which is unsubstituted or mono-, di- or trisubstituted by A and/or R⁹,
Ph denotes phenyl which is unsubstituted or mono-, di- or trisubstituted by A,
Hal denotes F, Cl, Br or I,
and salts, solvates and stereoisomers thereof,
where
a) 5-oxotetrahydrofuran-2-carboxylic acid is reacted with a compound of the formula II
R⁷-L II
in which
R⁷ denotes benzyl or alkyl having 1, 2, 3, 4, 5 or 6 C atoms and
L denotes Cl, Br, I or a free or reactively functionally modified OH group,
to give a compound of the formula III 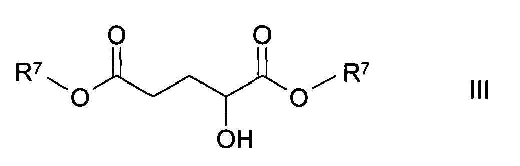 in which
R⁷ denotes benzyl or alkyl having 1, 2, 3, 4, 5 or 6 C atoms,
b) then a compound of the formula III is reacted with a compound of the formula IV
R⁸ -L IV
in which
R⁸ denotes alkylsulfonyl having 1-6 C atoms or arylsulfonyl having 6-10 C atoms and
L denotes Cl, Br, I or a free or reactively functionally modified OH group,
to give a compound of the formula V 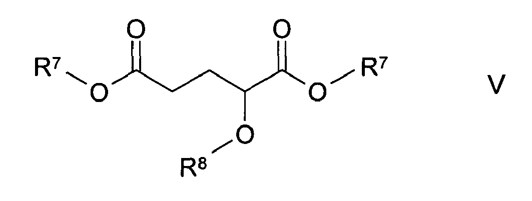 in which
R⁷ denotes benzyl or alkyl having 1, 2, 3, 4, 5 or 6 C atoms and
R⁸ denotes alkylsulfonyl having 1-6 C atoms or arylsulfonyl having 6-10 C atoms,
c) then a compound of the formula III or V is reacted with a compound of the formula VI 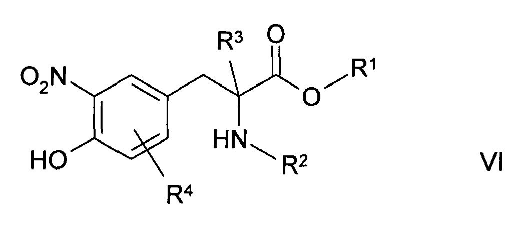 in which
R¹ denotes H, alkyl having 1, 2, 3, 4, 5 or 6 C atoms or benzyl,
R² denotes R¹⁰, CO-R¹⁰, COOR⁶ COOR¹⁰, CONHR¹⁰, SO₂R⁶ or SO₂R¹⁰,
R³ denotes H or alkyl having 1-6 C atoms,
R⁴ denotes H, Hal, OA, NHR¹⁰, N(R¹⁰)₂, -NH-acyl, -O-acyl, CN, NO₂, OR¹⁰, SR¹⁰, R² or CONHR¹⁰,
R⁶ denotes a mono- or bicyclic saturated, unsaturated or aromatic heterocycle having 1 to 4 N, O and/or S atoms, which may be unsubstituted or mono-, di- or trisubstituted by Hal, A, -CO-A, OA, CN, COOH, COOA, CONHA, NO₂, =NH or =O,
R⁹ denotes H, Hal, OA, NHA, NAA', NHacyl, Oacyl, CN, NO₂, SA, SOA, SO₂A, SO₂Ph or SO₃H,
R¹⁰ denotes H, A, Ar or aralkylene having 7-14 C atoms,
A, A' each, independently of one another, denote H or alkyl or cycloalkyl having 1-15 C atoms, each of which is unsubstituted or mono-, di- or trisubstituted by R⁹ and in which one, two or three methylene groups may be replaced by N, O and/or S,
Ar denotes mono- or bicyclic aromatic ring system having 0, 1, 2, 3 or 4 N, O and/or S atoms which is unsubstituted or mono-, di- or trisubstituted by A and/or R⁹,
Ph denotes phenyl which is unsubstituted or mono-, di- or trisubstituted by A,
Hal denotes F, Cl, Br or I,
to give a compound of the formula VII 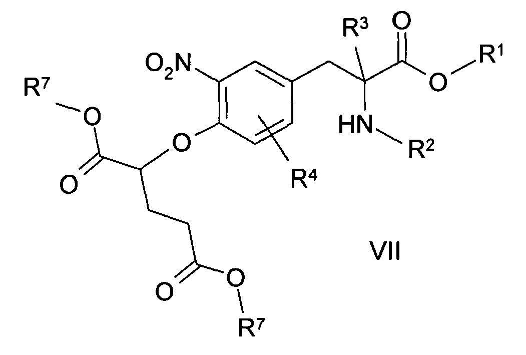 in which
R¹ denotes H, alkyl having 1, 2, 3, 4, 5 or 6 C atoms or benzyl,
R² denotes R¹⁰, CO-R¹⁰, COOR⁶, COOR¹⁰, CONHR¹⁰, SO₂R⁶ or SO₂R¹⁰,
R³ denotes H or alkyl having 1-6 C atoms,
R⁴ denotes H, Hal, OA, NHR¹⁰, N(R¹⁰)₂, -NH-acyl, -O-acyl, CN, NO₂, OR¹⁰, SR¹⁰, R² or CONHR¹⁰,
R⁶ denotes a mono- or bicyclic saturated, unsaturated or aromatic heterocycle having 1 to 4 N, O and/or S atoms, which may be unsubstituted or mono-, di- or trisubstituted by Hal, A, -CO-A, OA, CN, COOH, COOA, CONHA, NO₂, =NH or =O,
R⁷ denotes benzyl or alkyl having 1, 2, 3, 4, 5 or 6 C atoms,
R⁹ denotes H, Hal, OA, NHA, NAA', NHacyl, Oacyl, CN, NO₂, SA, SOA, SO₂A, SO₂Ph or SO₃H,
R¹⁰ denotes H, A, Ar or aralkylene having 7-14 C atoms,
A, A' each, independently of one another, denote H or alkyl or cycloalkyl having 1-15 C atoms, each of which is unsubstituted or mono-, di- or trisubstituted by R⁹ and in which one, two or three methylene groups may be replaced by N, O and/or S,
Ar denotes mono- or bicyclic aromatic ring system having 0, 1, 2, 3 or 4 N, O and/or S atoms which is unsubstituted or mono-, di- or trisubstituted by A and/or R⁹,
Ph denotes phenyl which is unsubstituted or mono-, di- or trisubstituted by A,
Hal denotes F, Cl, Br or I,
d) then a compound of the formula VII is converted into a compound of the formula VIII 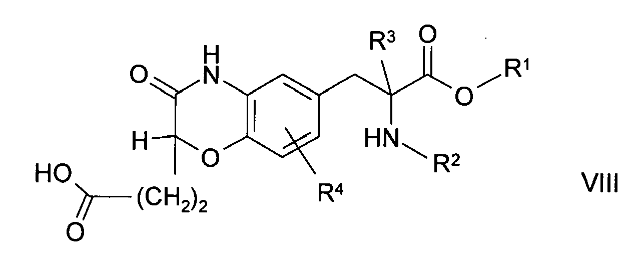 in which
R¹ denotes H, alkyl having 1, 2, 3, 4, 5 or 6 C atoms or benzyl,
R² denotes R¹⁰, CO-R¹⁰, COOR⁶, COOR¹⁰, CONHR¹⁰, SO₂R⁶ or SO₂R¹⁰
R³ denotes H or alkyl having 1-6 C atoms,
R⁴ denotes H, Hal, OA, NHR¹⁰, N(R¹⁰)₂, -NH-acyl, -O-acyl, CN, NO₂, OR¹⁰, SR¹⁰, R² or CONHR¹⁰,
R⁶ denotes a mono- or bicyclic saturated, unsaturated or aromatic heterocycle having 1 to 4 N, O and/or S atoms, which may be unsubstituted or mono-, di- or trisubstituted by Hal, A, -CO-A, OA, CN, COOH, COOA, CONHA, NO₂, =NH or =O,
R⁹ denotes H, Hal, OA, NHA, NAA', NHacyl, Oacyl, CN, NO₂, SA, SOA, SO₂A, SO₂Ph or SO₃H,
R¹⁰ denotes H, A, Ar or aralkylene having 7-14 C atoms,
A, A' each, independently of one another, denote H or alkyl or cycloalkyl having 1-15 C atoms, each of which is unsubstituted or mono-, di- or trisubstituted by R⁹ and in which one, two or three methylene groups may be replaced by N, O and/or S,
Ar denotes mono- or bicyclic aromatic ring system having 0, 1, 2, 3 or 4 N, O and/or S atoms which is unsubstituted or mono-, di- or trisubstituted by A and/or R⁹,
Ph denotes phenyl which is unsubstituted or mono-, di- or trisubstituted by A,
Hal denotes F, Cl, Br or I,
e) subsequently, if desired, a compound of the formula VIII is converted into a compound of the formula VIIIa 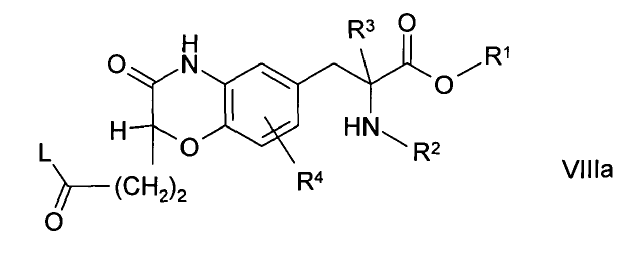 in which
R¹, R², R³ and R⁴ have the meanings indicated under d) and
L denotes Cl, Br, I or a reactively functionally modified OH group,
e) then a compound of the formula VIII or a compound of the formula VIIIa is reacted
e) i) with a compound of the formula IX
R⁵-H IX
in which
R⁵ denotes NH₂, H₂N-C(=NH) or H₂N-(C=NH)-NH, where the primary amino groups may also be provided with conventional amino-protecting groups, or may be mono-, di- or trisubstituted by R¹⁰, CO-R¹⁰, COOR¹⁰ or SO₂R¹⁰,
in which R¹⁰ has the meanings indicated for formula I,
or
e) ii) with a compound of the formula X
R⁶-NH₂ X
in which
R⁶ denotes a mono- or bicyclic saturated, unsaturated or aromatic heterocycle having 1 to 4 N, O and/or S atoms, which may be unsubstituted or mono-, di- or trisubstituted by Hal, A, -CO-A, OA, CN, COOH, COOA, CONHA, NO₂, =NH or =O,
to give a compound of the formula I,
subsequently, if desired, a radical R² is converted into another radical R²
by reacting a compound of the formula I in which R² denotes H with a compound selected from the group
L-R¹⁰, L-CO-R¹⁰, L-COOR⁶, L-COOR¹⁰, R^{10'}-N=C=O, R⁶-SO₂L, R¹⁰-SO₂L,
in which
R¹⁰ denotes A, Ar or aralkylene having 7-14 C atoms and
L denotes Cl, Br, I or a free or reactively functionally modified OH group,
and/or a base or acid of the formula I is converted into one of its salts.

2. Process according to Claim 1, in which
R³ denotes H,
R⁴ denotes H.

3. Process according to Claim 1 or 2, in which
R⁶ denotes 1*H*-imidazol-2-yl, thiazol-2-yl, 1*H*-benzimidazol-2-yl, 2H-pyrazol-2-yl, 1*H*-tetrazol-5-yl, 2-iminoimidazolidin-4-on-5-yl, 1-A-1,5-dihydroimidazol-4-on-2-yl, pyrimidin-2-yl or 1,4,5,6-tetrahydropyrimidin-2-yl.

4. Process according to one or more of Claims 1-3, in which
R⁹ denotes H.

5. Process according to one or more of Claims 1-4, in which
R¹⁰ denotes H, alkyl having 1, 2, 3, 4, 5 or 6 C atoms, camphor-10-yl or benzyl.

6. Process according to one or more of Claims 1-5, in which
A denotes unsubstituted alkyl or cycloalkyl having 1-15 C atoms.

7. Process according to one or more of Claims 1-6, in which
A denotes alkyl having 1, 2, 3, 4, 5 or 6 C atoms,
R¹⁰ denotes H, alkyl having 1, 2, 3, 4, 5 or 6 C atoms, camphor-10-yl or benzyl.

8. Process according to Claim 1, in which
R¹ denotes H, alkyl having 1, 2, 3, 4, 5 or 6 C atoms or benzyl,
R² denotes R¹⁰, CO-R¹⁰, COOR¹⁰ or SO₂R¹⁰,
R³ denotes H,
R⁴ denotes H,
R⁵ denotes R⁶,
R⁶ denotes a mono- or bicyclic saturated, unsaturated or aromatic heterocycle having 1 to 4 N, O and/or S atoms, which may be unsubstituted or mono-, di- or trisubstituted by Hal, A, -CO-A, OA, CN, COOH, COOA, CONHA, NO₂, =NH or =O,
X denotes NH,
R¹⁰ denotes H, alkyl having 1, 2, 3, 4, 5 or 6 C atoms, camphor-10-yl or benzyl,
A denotes alkyl having 1, 2, 3, 4, 5 or 6 C atoms,
Hal denotes F, Cl, Br or I.

9. Process according to Claim 8, in which
R⁶ denotes 1*H*-imidazol-2-yl, thiazol-2-yl, 1*H*-benzimidazol-2-yl, 2H-pyrazol-2-yl, 1*H*-tetrazol-5-yl, 2-iminoimidazolidin-4-on-5-yl, 1-A-1,5-dihydroimidazol-4-on-2-yl, pyrimidin-2-yl or 1,4,5,6-tetrahydropyrimidin-2-yl.

10. Process according to Claim 1 for the preparation of compounds of the formula la 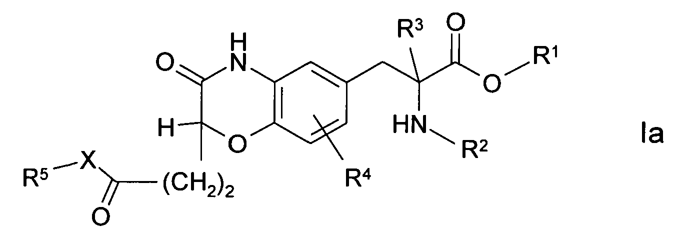 in which
R¹ denotes H or alkyl having 1, 2, 3 or 4 C atoms,
R² denotes R¹⁰, CO-R¹⁰, COOR¹⁰ or SO₂R¹⁰,
R³ denotes H,
R⁴ denotes H,
R⁵ denotes R⁶
X denotes NH,
R⁶ denotes *1H-imidazol-2-yl,* thiazol-2-yl, *1H-benzimidazol-2-yl,* 2H-pyrazol-2-yl, *1H-tetrazol-5-yl,* 2-iminoimidazolidin-4-on-5-yl, 1-A-1,5-dihydroimidazol-4-on-2-yl, pyrimidin-2-yl or 1,4,5,6-tetrahydropyrimidin-2-yl,
R¹⁰ denotes H or alkyl having 1, 2, 3, 4, 5 or 6 C atoms and
A denotes alkyl having 1, 2, 3, 4, 5 or 6 C atoms,
and salts, solvates and stereoisomers thereof,
where
a) 5-oxotetrahydrofuran-2-carboxylic acid is reacted with a compound of the formula II
R⁷-L II
in which
R⁷ denotes benzyl or alkyl having 1, 2, 3, 4, 5 or 6 C atoms and
L denotes Cl, Br, I or a free or reactively functionally modified OH group,
to give a compound of the formula III 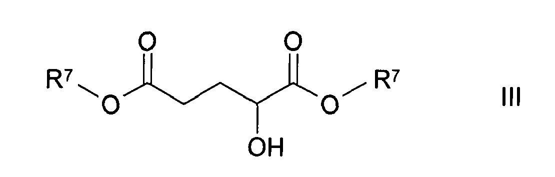 in which
R⁷ denotes benzyl or alkyl having 1, 2, 3, 4, 5 or 6 C atoms,
b) then a compound of the formula III is reacted with a compound of the formula IV
R⁸ -L IV
in which
R⁸ denotes alkylsulfonyl having 1-6 C atoms or arylsulfonyl having 6-10 C atoms and
L denotes Cl, Br, I or a free or reactively functionally modified OH group,
to give a compound of the formula V 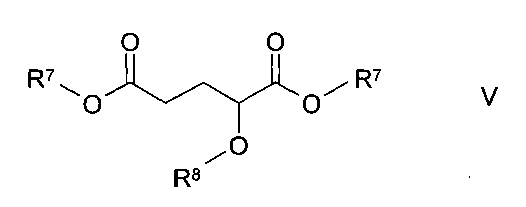 in which
R⁷ denotes benzyl or alkyl having 1, 2, 3, 4, 5 or 6 C atoms and
R⁸ denotes alkylsulfonyl having 1-6 C atoms or arylsulfonyl having 6-10 C atoms,
c) then a compound of the formula III or V is reacted with a compound of the formula VI 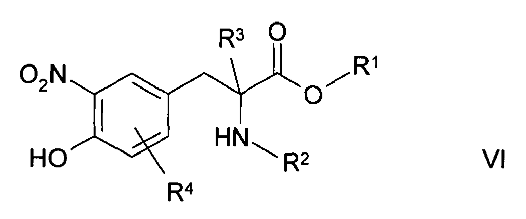 in which
R¹ denotes H or alkyl having 1, 2, 3 or 4 C atoms,
R² denotes R¹⁰, CO-R¹⁰, COOR¹⁰ or SO₂R¹⁰,
R³ denotes H,
R⁴ denotes H,
R¹⁰ denotes H or alkyl having 1, 2, 3, 4, 5 or 6 C atoms and,
to give a compound of the formula VII 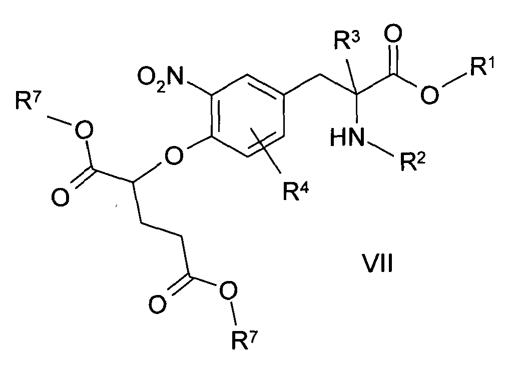 in which
R¹ denotes H or alkyl having 1, 2, 3 or 4 C atoms,
R² denotes R¹⁰ CO-R¹⁰ COOR¹⁰ or SO₂R¹⁰,
R³ denotes H,
R⁴ denotes H,
R⁷ denotes benzyl or alkyl having 1, 2, 3, 4, 5 or 6 C atoms,
R¹⁰ denotes H or alkyl having 1, 2, 3, 4, 5 or 6 C atoms,
d) then a compound of the formula VII is converted into a compound of the formula VIII 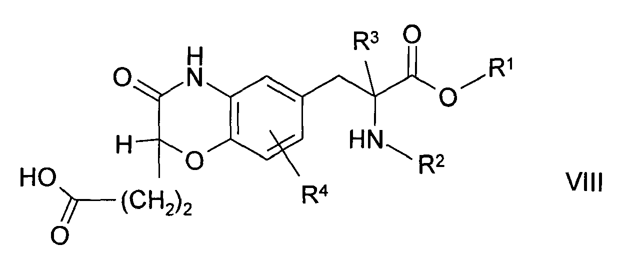 in which
R¹ denotes H or alkyl having 1, 2, 3 or 4 C atoms,
R² denotes R¹⁰, CO-R¹⁰, COOR¹⁰ or SO₂R¹⁰,
R³ denotes H,
R⁴ denotes H,
R¹⁰ denotes H or alkyl having 1, 2, 3, 4, 5 or 6 C atoms,
e) then a compound of the formula VIII
is reacted with a compound of the formula X
R⁶-NH₂ X
in which
R⁶ denotes 1*H*-imidazol-2-yl, thiazol-2-yl, 1*H*-benzimidazol-2-yl, 2*H*-pyrazol-2-yl, 1*H*-tetrazol-5-yl, 2-iminoimidazolidin-4-on-5-yl, 1-A-1,5-dihydroimidazol-4-on-2-yl, pyrimidin-2-yl or 1,4,5,6-tetrahydropyrimidin-2-yl,
to give a compound of the formula la,
subsequently, if desired, a radical R² is converted into another radical R²
by reacting a compound of the formula la in which R² denotes H with a compound selected from the group
L-R^{10'}, L-CO-R^{10'}, L-COOR^{10'}, R^{10'}-SO₂L,
in which
R¹⁰' denotes alkyl having 1, 2, 3, 4, 5 or 6 C atoms and
L denotes Cl, Br, I or a free or reactively functionally modified OH group,
and/or a base or acid of the formula la is converted into one of its salts.

11. Process according to one or more of Claims 1-10, in which
R⁶ denotes 1*H*-imidazol-2-yl.

12. Process according to one or more of Claims 1-11, in which in step b), in the compounds of the formula IV and V,
R⁸ denotes methylsulfonyl, trifluoromethylsulfonyl, phenylsulfonyl or p-tolylsulfonyl.

13. Process according to one or more of Claims 1-12, where the conversion of a compound of the formula VII into a compound of the formula VIII is carried out
a) by reduction of the nitro group, subsequent ester cleavage and cyclisation or
b) by ester cleavage, subsequent reduction of the nitro group and cyclisation.

14. Process according to Claim 1 for the preparation of (S)-2-(2,2-dimethylpropoxycarbonylamino)-3-{(S)-2-[2-(1H-imidazol-2-yl-carbamoyl)ethyl]-3-oxo-3,4-dihydro-2*H*-benzo[1,4]oxazin-6-yl}propionic acid,
where
a) (S)-3-nitrotyrosine ethyl ester tosylate is reacted with 2,2-dimethylpropyl chloroformate to give ethyl (S)-2-(2,2-dimethylpropoxycarbonylamino)-3-(4-hydroxy-3-nitrophenyl)propionate **12,**
b) **12** is reacted with dibenzyl 2-hydroxypentanedicarboxylate to give dibenzyl (R)-2-{4-[(S)-2-(2,2-dimethylpropoxycarbonylamino)-2-ethoxycarbonylethyl]-2-nitrophenoxy}pentanedicarboxylate **13**,
c) **13** is cyclised to give ethyl (2S)-3-[(2S)-2-(2-carboxyethyl)-3-oxo-3,4-dihydro-2*H*-benzo[1,4]oxazin-6-yl]-2-(2,2-dimethylpropoxycarbonylamino)propionate **14,**
d) **14** is reacted with 2-aminoimidazole to give ethyl 2-(S)-(2,2-dimethylpropoxycarbonylamino)-3-{3-oxo-2-(S)-[2-(1*H*-imidazol-2-yl-carbamoyl)ethyl]-3,4-dihydro-2H-benzo[1,4]oxazin-6-yl}propionate **15** and
e) **15** is converted by ester cleavage into (S)-2-(2,2-dimethylpropoxycarbonylamino)-3-{(S)-2-[2-(1*H*-imidazol-2-ylcarbamoyl)-ethyl]-3-oxo-3,4-dihydro-2*H*-benzo[1,4]oxazin-6-yl}propionic acid **16.**

15. Intermediate compounds of the formula VII 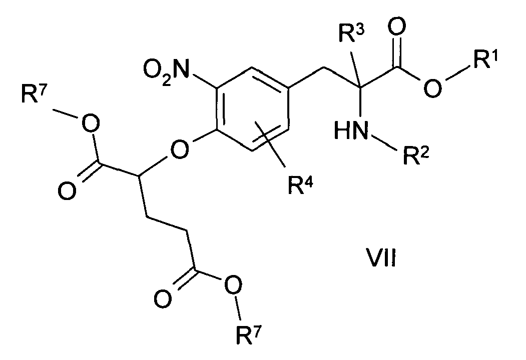 in which
R¹ denotes H or alkyl having 1, 2, 3 or 4 C atoms,
R² denotes R¹⁰ CO-R¹⁰, COOR¹⁰ or SO₂R¹⁰,
R³ denotes H,
R⁴ denotes H,
R⁷ denotes benzyl or alkyl having 1, 2, 3, 4, 5 or 6 C atoms,
R⁹ denotes H, Hal, OA, NHA, NAA', NHacyl, Oacyl, CN, NO₂, SA, SOA, SO₂A, SO₂Ph or SO₃H,
R¹⁰ denotes H, A, Ar or aralkylene having 7-14 C atoms,
A, A' each, independently of one another, denote H or alkyl or cycloalkyl having 1-15 C atoms, each of which is unsubstituted or mono-, di- or trisubstituted by R⁹ and in which one, two or three methylene groups may be replaced by N, O and/or S,
Ar denotes mono- or bicyclic aromatic ring system having 0, 1, 2, 3 or 4 N, O and/or S atoms, which is unsubstituted or mono-, di- or trisubstituted by A and/or R⁹,
and salts, solvates and stereoisomers thereof.

16. Process for the preparation of compounds of the formula VII, and salts, solvates and stereoisomers thereof,
where
a) 5-oxotetrahydrofuran-2-carboxylic acid is reacted with a compound of the formula II
R⁷-L II
in which
R⁷ denotes benzyl or alkyl having 1, 2, 3, 4, 5 or 6 C atoms and
L denotes Cl, Br, I or a free or reactively functionally modified OH group,
to give a compound of the formula III 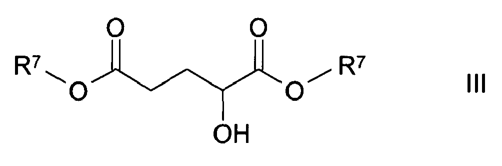 in which
R⁷ denotes benzyl or alkyl having 1, 2, 3, 4, 5 or 6 C atoms,
b) then a compound of the formula III is reacted with a compound of the formula IV
R⁸-L IV
in which
R⁸ denotes alkylsulfonyl having 1-6 C atoms or arylsulfonyl having 6-10 C atoms and
L denotes Cl, Br, I or a free or reactively functionally modified OH group,
to give a compound of the formula V 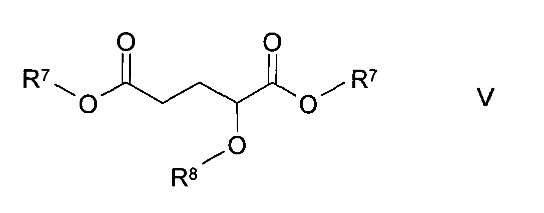 in which
R⁷ denotes benzyl or alkyl having 1, 2, 3, 4, 5 or 6 C atoms and
R⁸ denotes alkylsulfonyl having 1-6 C atoms or arylsulfonyl having 6-10 C atoms,
c) then a compound of the formula III or V is reacted with a compound of the formula VI 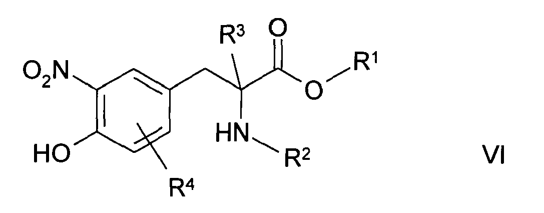
in which
R¹ denotes H or alkyl having 1, 2, 3 or 4 C atoms,
R² denotes R¹⁰ CO-R¹⁰ COOR¹⁰ or SO₂R¹⁰ ,
R³ denotes H,
R⁴ denotes H,
R⁹ denotes H, Hal, OA, NHA, NAA', NHacyl, Oacyl, CN, NO₂, SA, SOA, SO₂A, SO₂Ph or SO₃H,
R¹⁰ denotes H, A, Ar or aralkylene having 7-14 C atoms,
A, A' each, independently of one another, denote H or alkyl or cycloalkyl having 1-15 C atoms, each of which is unsubstituted or mono-, di- or trisubstituted by R⁹ and in which one, two or three methylene groups may be replaced by N, O and/or S,
Ar denotes mono- or bicyclic aromatic ring system having 0, 1, 2, 3 or 4 N, O and/or S atoms which is unsubstituted or mono-, di- or trisubstituted by A and/or R⁹,
to give a compound of the formula VII,
and / or an acid of the formula VII is converted into one of its salts.

## Revendications

1. Procédé de préparation de composés de formule I 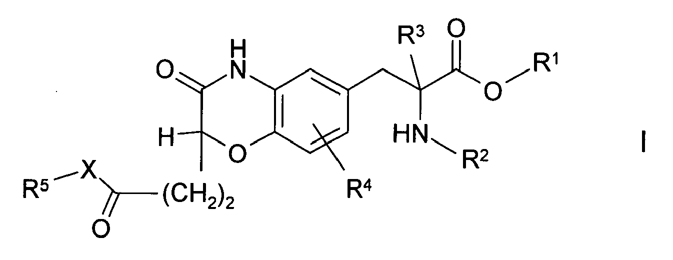 dans laquelle
R¹ désigne H, alkyle ayant 1, 2,3, 4, 5 ou 6 atomes de C ou benzyle,
R² désigne R¹⁰, CO-R¹⁰, COOR⁶, COOR¹⁰, CONHR¹⁰, SO₂R⁶ ou SO₂R¹⁰,
R³ désigne H ou alkyle ayant 1-6 atomes de C,
R⁴ désigne H, Hal, OA, NHR¹⁰, N(R¹⁰)₂, -NH-acyle, -O-acyle, CN, NO₂, OR¹⁰ SR¹⁰, R² ou CONHR¹⁰,
R⁵ désigne NH₂, H₂N-C(=NH) ou H₂N-(C=NH)-NH, où les groupements amino primaires peuvent également être fournis avec des groupements protecteurs d'amino classiques, ou peuvent être mono-, di- ou trisubstitués par R¹⁰, CO-R¹⁰, COOR¹⁰ ou SO₂R¹⁰,
ou R⁶,
X est absent ou désigne NH,
R⁶ désigne un hétérocycle mono- ou bicyclique saturé, insaturé ou aromatique ayant 1 à 4 atomes de N, O et/ou S, pouvant être non substitué ou mono-, di- ou trisubstitué par Hal, A, -CO-A, OA, CN, COOH, COOA, CONHA, NO₂, =NH ou =O,
R⁹ désigne H, Hal, OA, NHA, NAA', NHacyle, Oacyle, CN, NO₂, SA, SOA, SO₂A, SO₂Ph ou SO₃H,
R¹⁰ désigne H, A, Ar ou aralkylène ayant 7-14 atomes de C,
A, A' désignent chacun, indépendamment l'un de l'autre, H ou alkyle ou cycloalkyle ayant 1-15 atomes de C, chacun d'entre eux étant non substitué ou mono-, di- ou trisubstitué par R⁹ et où un, deux ou trois groupements méthylène peuvent être remplacés par N, O et/ou S,
Ar désigne un noyau aromatique mono- ou bicyclique ayant 0, 1, 2, 3 ou 4 atomes de N, O et/ou S qui est non substitué ou mono-, di- ou trisubstitué par A et/ou R⁹,
Ph désigne phényle qui est non substitué ou mono-, di- ou trisubstitué par A,
Hal désigne F, CI, Br ou I,
et les sels, solvats et stéréoisomères de ceux-ci,
où
a) de l'acide 5-oxotétrahydrofuran-2-carboxylique est réagi avec un composé de formule II
R⁷-L II
dans laquelle
R⁷ désigne benzyle ou alkyle ayant 1, 2, 3, 4, 5 ou 6 atomes de
C et L désigne CI, Br, I ou un groupement OH libre ou modifié de manière réactive et fonctionnelle,
pour donner un composé de formule III 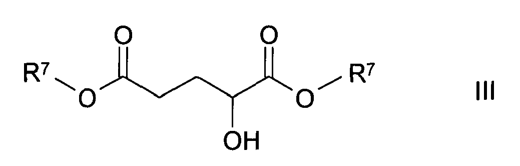 dans laquelle
R⁷ désigne benzyle ou alkyle ayant 1, 2, 3, 4, 5 ou 6 atomes de C,
b) puis un composé de formule III est réagi avec un composé de formule IV
R⁸ -L IV
dans laquelle
R⁸ désigne alkylsulfonyle ayant 1-6 atomes de C ou arylsulfonyle ayant 6-10 atomes de C et
L désigne CI, Br, I ou un groupement OH libre ou modifié de manière réactive et fonctionnelle,
pour donner un composé de formule V 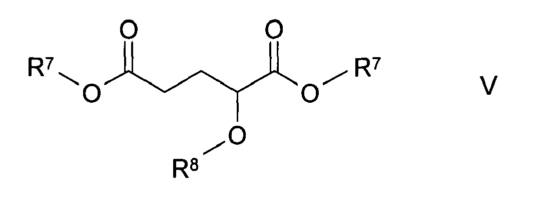 dans laquelle
R⁷ désigne benzyle ou alkyle ayant 1, 2, 3, 4, 5 ou 6 atomes de C et
R⁸ désigne alkylsulfonyle ayant 1-6 atomes de C ou arylsulfonyle ayant 6-10 atomes de C,
c) puis un composé de formule III ou V est réagi avec un composé de formule VI 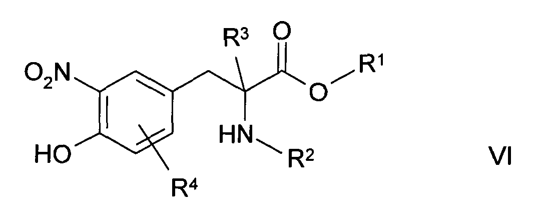 dans laquelle
R¹ désigne H, alkyle ayant 1, 2, 3, 4, 5 ou 6 atomes de C ou benzyle,
R² désigne R¹⁰, CO-R¹⁰, COOR⁶, COOR¹⁰, CONHR¹⁰, SO₂R⁶ ou SO₂R¹⁰,
R³ désigne H ou alkyle ayant 1-6 atomes de C,
R⁴ désigne H, Hal, OA, NHR¹⁰, N(R¹⁰)₂, -NH-acyle, -O-acyle, CN, NO₂, OR¹⁰, SR¹⁰, R² ou CONHR¹⁰,
R⁶ désigne un hétérocycle mono- ou bicyclique saturé, insaturé ou aromatique ayant 1 à 4 atomes de N, O et/ou S, pouvant être non substitué ou mono-, di- ou trisubstitué par Hal, A, -CO-A, OA, CN, COOH, COOA, CONHA, NO₂, =NH ou =O,
R⁹ désigne H, Hal, OA, NHA, NAA', NHacyle, Oacyle, CN, NO₂, SA, SOA, SO₂A, SO₂Ph ou SO₃H,
R¹⁰ désigne H, A, Ar ou aralkylène ayant 7-14 atomes de C,
A, A' désignent chacun, indépendamment l'un de l'autre, H ou alkyle ou cycloalkyle ayant 1-15 atomes de C, chacun d'entre eux étant non substitué ou mono-, di- ou trisubstitué par R⁹ et où un, deux ou trois groupements méthylène peuvent être remplacés par N, O et/ou S,
Ar désigne un noyau aromatique mono- ou bicyclique ayant 0, 1, 2, 3 ou 4 atomes de N, O et/ou S qui est non substitué ou mono-, di- ou trisubstitué par A et/ou R⁹,
Ph désigne phényle qui est non substitué ou mono-, di- ou trisubstitué par A,
Hal désigne F, CI, Br ou l,
pour donner un composé de formule VII 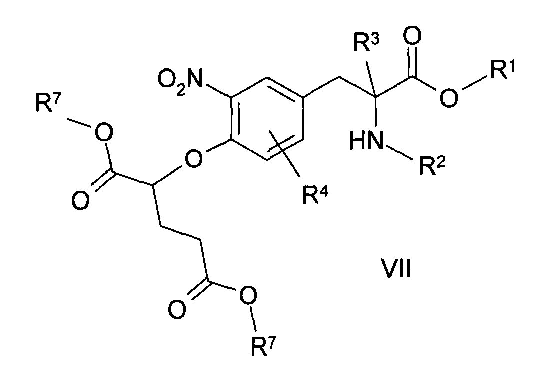 dans laquelle
R¹ désigne H, alkyle ayant 1, 2, 3, 4, 5 ou 6 atomes de C ou benzyle,
R² désigne R¹⁰, CO-R¹⁰, COOR⁶, COOR¹⁰, CONHR¹⁰, SO₂R⁶ ou SO₂R¹⁰
R³ désigne H ou alkyle ayant 1-6 atomes de C,
R⁴ désigne H, Hal, OA, NHR¹⁰, N(R¹⁰)₂, -NH-acyle, -O-acyle, CN, NO₂, OR¹⁰ SR¹⁰ R² ou CONHR¹⁰,
R⁶ désigne un hétérocycle mono- ou bicyclique saturé, insaturé ou aromatique ayant 1 à 4 atomes de N, O et/ou S, pouvant être non substitué ou mono-, di- ou trisubstitué par Hal, A, -CO-A, OA, CN, COOH, COOA, CONHA, NO₂, =NH ou =O,
R⁷ désigne benzyle ou alkyle ayant 1, 2, 3, 4, 5 ou 6 atomes de C,
R⁹ désigne H, Hal, OA, NHA, NAA', NHacyle, Oacyle, CN, NO₂, SA, SOA, SO₂A, SO₂Ph ou SO₃H,
R¹⁰ désigne H, A, Ar ou aralkylène ayant 7-14 atomes de C,
A, A' désignent chacun, indépendamment l'un de l'autre, H ou alkyle ou cycloalkyle ayant 1-15 atomes de C, chacun d'entre eux étant non substitué ou mono-, di- ou trisubstitué par R⁹ et où un, deux ou trois groupements méthylène peuvent être remplacés par N, O et/ou S,
Ar désigne un noyau aromatique mono- ou bicyclique ayant 0, 1, 2, 3 ou 4 atomes de N, O et/ou S qui est non substitué ou mono-, di- ou trisubstitué par A et/ou R⁹,
Ph désigne phényle qui est non substitué ou mono-, di- ou trisubstitué par A,
Hal désigne F, CI, Br ou I,
d) puis un composé de formule VII est converti en un composé de formule VIII 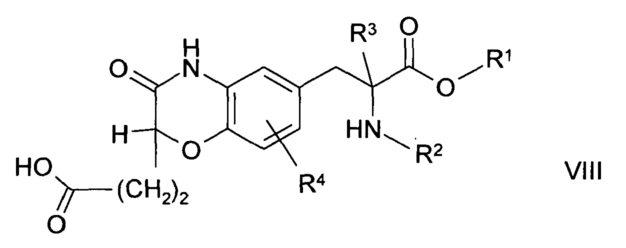 dans laquelle
R¹ désigne H, alkyle ayant 1, 2, 3, 4, 5 ou 6 atomes de C ou benzyle,
R² désigne R¹⁰, CO-R¹⁰, COOR⁶, COOR¹⁰, CONHR¹⁰, SO₂R⁶ ou SO₂R¹⁰,
R³ désigne H ou alkyle ayant 1-6 atomes de C,
R⁴ désigne H, Hal, OA, NHR¹⁰, N(R¹⁰)₂, -NH-acyle, -O-acyle, CN, NO₂, OR¹⁰, SR¹⁰, R² ou CONHR¹⁰,
R⁶ désigne un hétérocycle mono- ou bicyclique saturé, insaturé ou aromatique ayant 1 à 4 atomes de N, O et/ou S, pouvant être non substitué ou mono-, di- ou trisubstitué par Hal, A, -CO-A, OA, CN, COOH, COOA, CONHA, NO₂, =NH ou =O,
R⁹ désigne H, Hal, OA, NHA, NAA', NHacyle, Oacyle, CN, NO₂, SA, SOA, SO₂A, SO₂Ph ou SO₃H,
R¹⁰ désigne H, A, Ar ou aralkylène ayant 7-14 atomes de C,
A, A' désignent chacun, indépendamment l'un de l'autre, H ou alkyle ou cycloalkyle ayant 1-15 atomes de C, chacun d'entre eux étant non substitué ou mono-, di- ou trisubstitué par R⁹ et où un, deux ou trois groupements méthylène peuvent être remplacés par N, O et/ou S,
Ar désigne un noyau aromatique mono- ou bicyclique ayant 0, 1, 2, 3 ou 4 atomes de N, O et/ou S qui est non substitué ou mono-, di- ou trisubstitué par A et/ou R⁹,
Ph désigne phényle qui est non substitué ou mono-, di- ou trisubstitué par A,
Hal désigne F, CI, Br ou I,
e) puis, si on le souhaite, un composé de formule VIII est converti en un composé de formule VIIIa 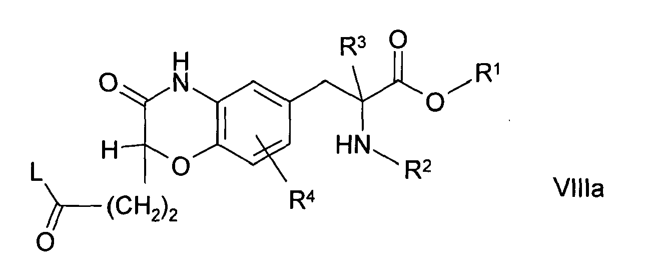 dans laquelle
R¹, R², R³ et R⁴ ont les significations indiquées en d) et
L désigne CI, Br, I ou un groupement OH modifié de manière réactive et fonctionnelle,
e) puis un composé de formule VIII ou un composé de formule VIIIa est réagi
e) i) avec un composé de formule IX
R⁵-H IX
dans laquelle
R⁵ désigne NH₂, H₂N-C(=NH) ou H₂N-(C=NH)-NH, où les groupements amino primaires peuvent également être fournis avec des groupements protecteurs d'amino classiques, ou peuvent être mono-, di- ou trisubstitués par R¹⁰, CO-R¹⁰, COOR¹⁰ ou SO₂R¹⁰,
où R¹⁰ prend les significations indiquées pour la formule I,
ou
e) ii) avec un composé de formule X
R⁶-NH₂ X
dans laquelle
R⁶ désigne un hétérocycle mono- ou bicyclique saturé, insaturé ou aromatique ayant 1 à 4 atomes de N, O et/ou S, pouvant être non substitué ou mono-, di- ou trisubstitué par Hal, A, -CO-A, OA, CN, COOH, COOA, CONHA, NO₂, =NH ou =O,
pour donner un composé de formule I,
puis, si on le souhaite, un radical R² est converti en un autre radical R²
en faisant réagir un composé de formule I dans laquelle R² désigne H avec un composé choisi parmi le groupe
L-R¹⁰, L-CO-R¹⁰, L-COOR⁶, L-COOR¹⁰, R¹⁰'-N=C=O, R⁶-SO₂L, R¹⁰-SO₂L,
où
R¹⁰ désigne A, Ar ou aralkylène ayant 7-14 atomes de C et
L désigne CI, Br, I ou un groupement OH libre ou modifié de manière réactive et fonctionnelle,
et/ou une base ou un acide de formule I est converti(e) en l'un de ses sels.

2. Procédé selon la revendication 1, dans lequel
R³ désigne H,
R⁴ désigne H.

3. Procédé selon la revendication 1 ou 2, dans lequel
R⁶ désigne 1*H*-imidazol-2-yle, thiazol-2-yle, 1*H*-benzimidazol-2-yle, 2*H*-pyrazol-2-yle, 1*H*-tétrazol-5-yle, 2-iminoimidazolidin-4-on-5-yle; 1-A-1,5-dihydroimidazol-4-on-2-yle, pyrimidin-2-yle
ou 1,4,5,6-tétrahydropyrimidin-2-yle.

4. Procédé selon l'une ou plusieurs parmi les revendications 1-3, dans lequel
R⁹ désigne H.

5. Procédé selon l'une ou plusieurs parmi les revendications 1-4, dans lequel
R¹⁰ désigne H, alkyle ayant 1, 2, 3, 4, 5 ou 6 atomes de C, camphr-10-yle ou benzyle.

6. Procédé selon l'une ou plusieurs parmi les revendications 1-5, dans lequel
A désigne alkyle ou cycloalkyle non substitué ayant 1-15 atomes de C.

7. Procédé selon l'une ou plusieurs parmi les revendications 1-6, dans lequel
A désigne alkyle ayant 1, 2, 3, 4, 5 ou 6 atomes de C,
R¹⁰ désigne H, alkyle ayant 1, 2, 3, 4, 5 ou 6 atomes de C, camphr-10-yle ou benzyle.

8. Procédé selon la revendication 1, dans lequel
R¹ désigne H, alkyle ayant 1, 2, 3, 4, 5 ou 6 atomes de C ou benzyle,
R² désigne R¹⁰, CO-R¹⁰, COOR¹⁰ ou SO₂R¹⁰,
R³ désigne H,
R⁴ désigne H,
R⁵ désigne R⁶
R⁶ désigne un hétérocycle mono- ou bicyclique saturé, insaturé ou aromatique ayant 1 à 4 atomes de N, O et/ou S, pouvant être non substitué ou mono-, di- ou trisubstitué par Hal, A, -CO-A, OA, CN, COOH, COOA, CONHA, NO₂, =NH ou =O,
X désigne NH,
R¹⁰ désigne H, alkyle ayant 1, 2, 3, 4, 5 ou 6 atomes de C, camphr-10-yle ou benzyle,
A désigne alkyle ayant 1, 2, 3, 4, 5 ou 6 atomes de C,
Hal désigne F, CI, Br ou I.

9. Procédé selon la revendication 8, dans lequel
R⁶ désigne 1 H-imidazol-2-yle, thiazol-2-yle, 1 H-benzimidazol-2-yle, 2*H*-pyrazol-2-yle, 1*H*-tétrazol-5-yle, 2-iminoimidazolidin-4-on-5-yle, 1-A-1,5-dihydroimidazol-4-on-2-yle, pyrimidin-2-yle ou 1,4,5,6-tétrahydropyrimidin-2-yle.

10. Procédé selon la revendication 1, pour la préparation de composés de formule la 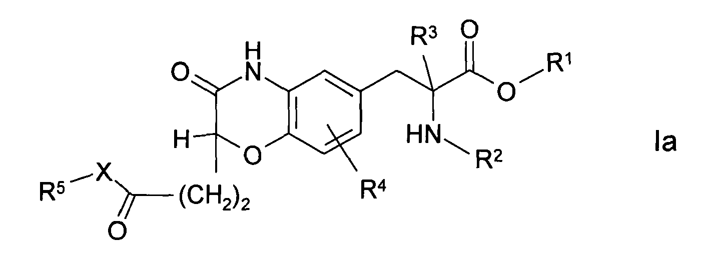 dans laquelle
R¹ désigne H ou alkyle ayant 1, 2, 3 ou 4 atomes de C,
R² désigne R¹⁰, CO-R¹⁰, COOR¹⁰ ou SO₂R¹⁰,
R³ désigne H,
R⁴ désigne H,
R⁵ désigne R⁶,
X désigne NH,
R⁶ désigne 1*H*-imidazol-2-yle, thiazol-2-yle, 1*H*-benzimidazol-2-yle, 2H-pyrazol-2-yle, 1*H*-tétrazol-5-yle, 2-iminoimidazolidin-4-on-5-yle, 1-A-1,5-dihydroimidazol-4-on-2-yle, pyrimidin-2-yle ou 1,4,5,6-tétrahydropyrimidin-2-yle,
R¹⁰ désigne H ou alkyle ayant 1, 2, 3, 4, 5 ou 6 atomes de C et
A désigne alkyle ayant 1, 2, 3, 4, 5 ou 6 atomes de C,
et les sels, solvats et stéréoisomères de ceux-ci,
où
a) de l'acide 5-oxotétrahydrofuran-2-carboxylique est réagi avec un composé de formule II
R⁷ -L II
dans laquelle
R⁷ désigne benzyle ou alkyle ayant 1, 2, 3, 4, 5 ou 6 atomes de C et
L désigne CI, Br, I ou un groupement OH libre ou modifié de manière réactive et fonctionnelle,
pour donner un composé de formule III 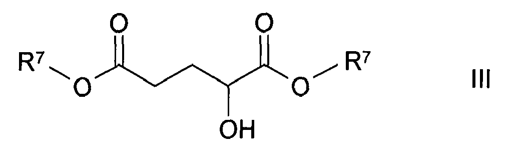 dans laquelle
R⁷ désigne benzyle ou alkyle ayant 1, 2, 3, 4, 5 ou 6 atomes de C,
b) puis un composé de formule III est réagi avec un composé de formule IV
R⁸ -L IV
dans laquelle
R⁸ désigne alkylsulfonyle ayant 1-6 atomes de C ou arylsulfonyle ayant 6-10 atomes de C et
L désigne Cl, Br, I ou un groupement OH libre ou modifié de manière réactive et fonctionnelle,
pour donner un composé de formule V 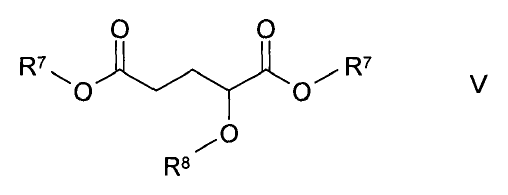 dans laquelle
R⁷ désigne benzyle ou alkyle ayant 1, 2, 3, 4, 5 ou 6 atomes de C et
R⁸ désigne alkylsulfonyle ayant 1-6 atomes de C ou arylsulfonyle ayant 6-10 atomes de C,
c) puis un composé de formule III ou V est réagi avec un composé de formule VI 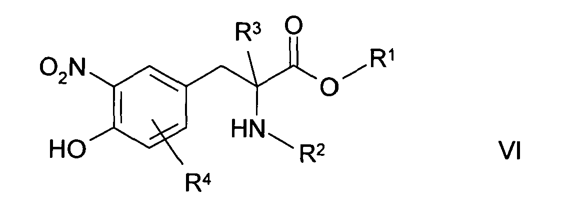 dans laquelle
R¹ désigne H ou alkyle ayant 1, 2, 3 ou 4 atomes de C,
R² désigne R¹⁰ CO-R¹⁰, COOR¹⁰ ou SO₂R¹⁰,
R³ désigne H,
R⁴ désigne H,
R¹⁰ désigne H ou alkyle ayant 1, 2, 3, 4, 5 ou 6 atomes de C,
pour donner un composé de formule VII 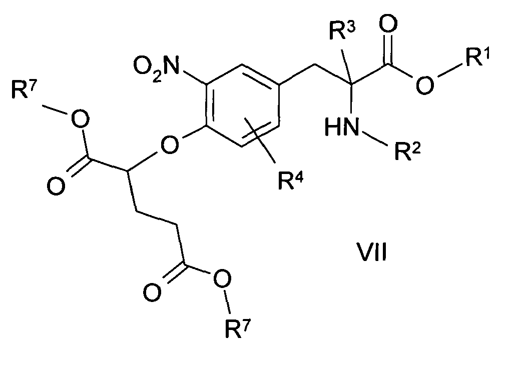 dans laquelle
R¹ désigne H ou alkyle ayant 1, 2, 3 ou 4 atomes de C,
R² désigne R¹⁰ CO-R¹⁰, COOR¹⁰ ou SO₂R¹⁰
R³ désigne H,
R⁴ désigne H,
R⁷ désigne benzyle ou alkyle ayant 1, 2, 3, 4, 5 ou 6 atomes de C,
R¹⁰ désigne H ou alkyle ayant 1, 2, 3, 4, 5 ou 6 atomes de C,
d) puis un composé de formule VII est converti en un composé de formule VIII 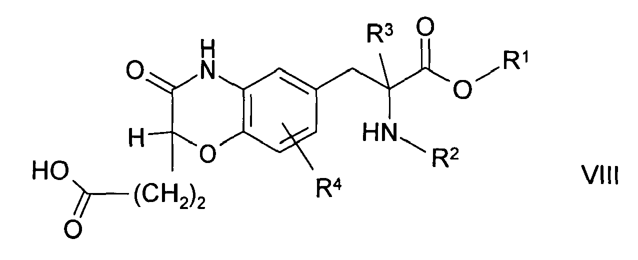 dans laquelle
R¹ désigne H ou alkyle ayant 1, 2, 3 ou 4 atomes de C,
R² désigne R¹⁰, CO-R¹⁰, COOR¹⁰ ou SO₂R¹⁰,
R³ désigne H,
R⁴ désigne H,
R¹⁰ désigne H ou alkyle ayant 1, 2, 3, 4, 5 ou 6 atomes de C,
e) puis un composé de formule VIII
est réagi avec un composé de formule X
R⁶-NH₂ X
dans laquelle
R⁶ désigne 1*H*-imidazol-2-yle, thiazol-2-yle, 1*H*-benzimidazol-2-yle, 2*H*-pyrazol-2-yle, 1*H*-tétrazol-5-yle, 2-iminoimidazolidin-4-on-5-yle, 1-A-1,5-dihydroimidazol-4-on-2-yle, pyrimidin-2-yle ou 1,4,5,6-tétrahydropyrimidin-2-yle,
pour donner un composé de formule la,
puis, si on le souhaite, un radical R² est converti en un autre radical R²
en faisant réagir un composé de formule la dans laquelle R² désigne H avec un composé choisi parmi le groupe
L-R^{10'}, L-CO-R^{10'}, L-COOR^{10'}, R^{10'}-SO₂L,
où
R¹⁰' désigne alkyle ayant 1, 2, 3, 4, 5 ou 6 atomes de C et
L L désigne Cl, Br, I ou un groupement OH libre ou modifié de manière réactive et fonctionnelle,
et/ou une base ou un acide de formule la est converti(e) en l'un de ses sels.

11. Procédé selon l'une ou plusieurs parmi les revendications 1-10, dans lequel
R⁶ désigne 1*H*-imidazol-2-yle.

12. Procédé selon l'une ou plusieurs parmi les revendications 1-11, dans lequel
dans l'étape b), dans les composés de formule IV et V,
R⁸ désigne méthylsulfonyle, trifluorométhylsulfonyle, phénylsulfonyle ou p-tolylsulfonyle.

13. Procédé selon l'une ou plusieurs parmi les revendications 1-12, où la conversion d'un composé de formule VII en un composé de formule VIII est réalisée
a) par réduction du groupement nitro, puis clivage d'ester et cyclisation ou
b) par clivage d'ester, puis réduction du groupement nitro et cyclisation.

14. Procédé selon la revendication 1, pour la préparation de l'acide (S)-2-(2,2-diméthylpropoxycarbonylamino)-3-{(S)-2-[2-(1*H*-imidazol-2-yl-carbamoyl)éthyl]-3-oxo-3,4-dihydro-2*H*-benzo[1,4]oxazin-6-yl}propionique,
où
a) du tosylate de (S)-3-nitrotyrosine éthylester est réagi avec du chloroformiate de 2,2-diméthylpropyle pour donner le (S)-2-(2,2-diméthylpropoxycarbonylamino)-3-(4-hydroxy-3-nitrophényl)propionate d'éthyle **12**,
b) **12** est réagi avec du 2-hydroxypentanedicarboxylate de dibenzyle pour donner le (R)-2-{4-[(S)-2-(2,2-diméthylpropoxycarbonylamino)-2-éthoxycarbonyléthyl]-2-nitrophénoxy}pentanedicarboxylate de dibenzyle **13**,
c) **13** est cyclisé pour donner le (2S)-3-[(2S)-2-(2-carboxyéthyl)-3-oxo-3,4-dihydro-2*H*-benzo[1,4]oxazin-6-yl]-2-(2,2-diméthylpropoxycarbonylamino)propionate d'éthyle **14**,
d) **14** est réagi avec du 2-aminoimidazole pour donner le 2-(S)-(2,2-diméthylpropoxycarbonylamino)-3-{3-oxo-2-(S)-[2-(1*H-*imidazol-2-ylcarbamoyl)éthyl]-3,4-dihydro-2*H*-benzo[1,4]oxazin-6-yl}propionate d'éthyle 15 et
e) **15** est converti par clivage d'ester en acide (S)-2-(2,2-diméthylpropoxycarbonylamino)-3-{(S)-2-[2-(1*H*-imidazol-2-ylcarbamoyl)-éthyl]-3-oxo-3,4-dihydro-2*H*-benzo[1,4]oxazin-6-yl}propionique **16.**

15. Composés intermédiaires de formule VII 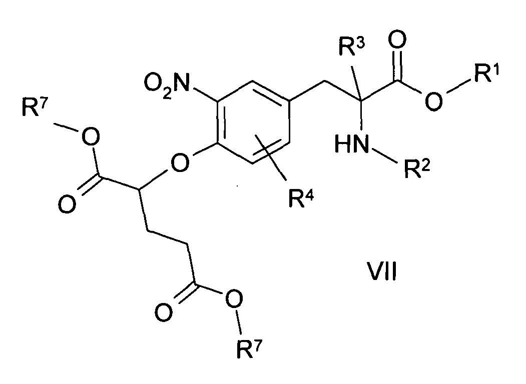 dans laquelle
R¹ désigne H ou alkyle ayant 1, 2, 3 ou 4 atomes de C,
R² désigne R¹⁰, CO-R¹⁰, COOR¹⁰ ou SO₂R¹⁰,
R³ désigne H,
R⁴ désigne H,
R⁷ désigne benzyle ou alkyle ayant 1, 2, 3, 4, 5 ou 6 atomes de C,
R⁹ désigne H, Hal, OA, NHA, NAA', NHacyle, Oacyle, CN, NO₂, SA, SOA, SO₂A, SO₂Ph ou SO₃H,
R¹⁰ désigne H, A, Ar ou aralkylène ayant 7-14 atomes de C,
A, A' désignent chacun, indépendamment l'un de l'autre, H ou alkyle ou cycloalkyle ayant 1-15 atomes de C, chacun d'entre eux étant non substitué ou mono-, di- ou trisubstitué par R⁹ et où un, deux ou trois groupements méthylène peuvent être remplacés par N, O et/ou S,
Ar désigne un noyau aromatique mono- ou bicyclique ayant 0, 1, 2, 3 ou 4 atomes de N, O et/ou S, qui est non substitué ou mono-, di- ou trisubstitué par A et/ou R⁹,
et les sels, solvats et stéréoisomères de ceux-ci.

16. Procédé de préparation de composés de formule VII, et des sels, solvats et stéréoisomères de ceux-ci,
où
a) de l'acide 5-oxotétrahydrofuran-2-carboxylique est réagi avec un composé de formule II
R⁷-L II
dans laquelle
R⁷ désigne benzyle ou alkyle ayant 1, 2, 3, 4, 5 ou 6 atomes de C et
L désigne CI, Br, I ou un groupement OH libre ou modifié de manière réactive et fonctionnelle,
pour donner un composé de formule III 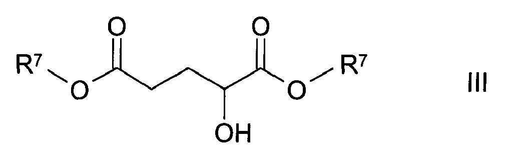 dans laquelle
R⁷ désigne benzyle ou alkyle ayant 1, 2, 3, 4, 5 ou 6 atomes de C,
b) puis un composé de formule III est réagi avec un composé de formule IV
R⁸ -L IV
dans laquelle
R⁸ désigne alkylsulfonyle ayant 1-6 atomes de C ou arylsulfonyle ayant 6-10 atomes de C et
L désigne CI, Br, I ou un groupement OH libre ou modifié de manière réactive et fonctionnelle,
pour donner un composé de formule V 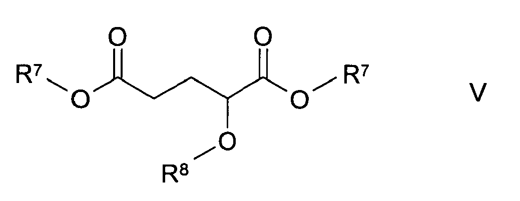 dans laquelle
R⁷ désigne benzyle ou alkyle ayant 1, 2, 3, 4, 5 ou 6 atomes de C et
R⁸ désigne alkylsulfonyle ayant 1-6 atomes de C ou arylsulfonyle ayant 6-10 atomes de C,
c) puis un composé de formule III ou V est réagi avec un composé de formule VI 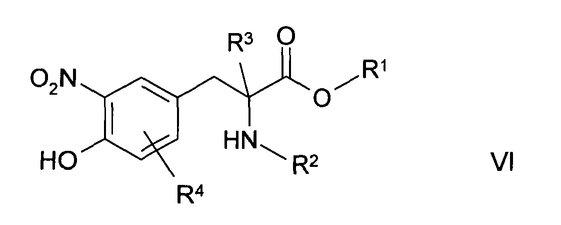
dans laquelle
R¹ désigne H ou alkyle ayant 1, 2, 3 ou 4 atomes de C,
R² désigne R¹⁰, CO-R¹⁰, COOR¹⁰ ou SO₂R¹⁰,
R³ désigne H,
R⁴ désigne H,
R⁹ désigne H, Hal, OA, NHA, NAA', NHacyle, Oacyle, CN, NO₂, SA, SOA, SO₂A, SO₂Ph ou SO₃H,
R¹⁰ désigne H, A, Ar ou aralkylène ayant 7-14 atomes de C,
A, A' désignent chacun, indépendamment l'un de l'autre, H ou alkyle ou cycloalkyle ayant 1-15 atomes de C, chacun d'entre eux étant non substitué ou mono-, di- ou trisubstitué par R⁹ et où un, deux ou trois groupements méthylène peuvent être remplacés par N, O et/ou S,
Ar désigne un noyau aromatique mono- ou bicyclique ayant 0, 1, 2, 3 ou 4 atomes de N, O et/ou S qui est non substitué ou mono-, di- ou trisubstitué par A et/ou R⁹,
pour donner un composé de formule VII,
et/ou un acide de formule VII est converti en l'un de ses sels.
